# EUROPEAN PATENT APPLICATION

(11) **EP 1 576 966 A1**
(43) Date of publication of application: **21.09.2005**
(21) Application number: 03815518.0
(22) Date of filing: 26.12.2003
(51) Int. Cl.: A61K 39/00, A61K 39/12, C12N 15/00, C12N 15/13, A61P 35/00

(54) **METHOD OF PREPARING A VACCINE AND ANTI-TUMOR VACCINES**

(30) Priority: 27.12.2002 CN 02159602
(71) Applicant: Shenzhen Tsinghua Yuanxing Bio-Pharm Science & Technology Co., Ltd., 518057 (CN)
(72) Inventor: Tian, Ling, Room 08-48, Chengdu, Sichuan 610015 (CN); Wei, Yuquan, Chengdu Sichuan 610015 (CN)
(74) Representative: Grünecker, Kinkeldey, Stockmair & Schwanhäusser Anwaltssozietät
(86) International application number: PCT/CN2003/001127
(87) International publication number: WO 2004/067029

(57) **Abstract**

The present invention provides a method of preparing a vaccine. The method includes: (1) analyzing specific antigen of particular pathogen; (2) obtaining a polynucleotide encoding the specific antigen; (3) obtaining a polynucleotide sequence having a sufficient difference from the polynucleotide; (4) preparing a vaccine using the polynucleotide sequence. The present invention also provides antitumor vaccines, particularly antitumor EGFR molecular vaccines. The antitumor EGFR molecular vaccines are a variety of new biotechnological vaccines constructed by using EGFR molecule as antigens, including protein vaccines, gene vaccines, virus vaccines, gene-modified vaccines and stably transformed symbiotic bacterium. One of the biological function of the EGFR molecular vaccines has an antitumor effect on a variety of solid tumor cancer which over-express the EGFR molecule, e.g., lung cancer, breast cancer, ovary cancer, colon cancer, prostate cancer, stomach cancer, etc., including a protective antitumor immune effect, therapeutic antitumor effect and inhibiting cancer metastasis. The antitumor action mechanism of EGFR molecular vaccine is that the EGFR molecule may destroy an individual's immune tolerance to self-EGFR molecule as cross-reactive antigens, and therefore induce an immuno-cross-response to the self-EGFR molecule in the individual, including active immune reaction (cellular immune reaction and humoral immune reaction) and passive immune reaction (adoptive immune reaction). The antitumor EGFR molecular vaccines are either traditional preparation (aqueous, lyophilized powder) or nano preparation prepared by nano biotechnology. The EGFR molecular vaccine exhibits an enhanced antitumor effect through the synergistic action of other immunopotentiator.

SEQUENCE LISTING

<110>  Shenzhen Tsinghua Yuanxing Bio-pharm Science & Technology Co. Ltd.

<120>  Method of Preparing a Vaccine and Anti-tumor Vaccines

<130>  CPS40580

<150>  CN02159602.6
<151>  2002-12-27

<160>  27

<170>  PatentIn version 3.1

<210>  1

<211>  3633

<212>  DNA

<213>  ÈË(homo sapiens)

<220>

<221>  CDS

<222>  (1)..(3630)

<223>

<400>  1
atg cga ccc tcc ggg acg gcc ggg gca gcg ctc ctg gcg ctg ctg gct        48
gcg ctc tgc ccg gcg agt cgg gct ctg gag gaa aag aaa gtt tgc caa        96
ggc acg agt aac aag ctc acg cag ttg ggc act ttt gaa gat cat ttt        144
ctc agc ctc cag agg atg ttc aat aac tgt gag gtg gtc ctt ggg aat        192
ttg gaa att acc tat gtg cag agg aat tat gat ctt tcc ttc tta aag        240
acc atc cag gag gtg gct ggt tat gtc ctc att gcc ctc aac aca gtg        288
gag cga att cct ttg gaa aac ctg cag atc atc aga gga aat atg tac 336
tac gaa aat tcc tat gcc tta gca gtc tta tct aac tat gat gca aat 384
aaa acc gga ctg aag gag ctg ccc atg aga aat tta cag gaa atc ctg 432
cat ggc gcc gtg cgg ttc agc aac aac cct gcc ctg tgc aac gtg gag 480
agc atc cag tgg cgg gac ata gtc agc agt gac ttt ctc agc aac atg 528
tcg atg gac ttc cag aac cac ctg ggc agc tgc caa aag tgt gat cca 576
agc tgt ccc aat ggg agc tgc tgg ggt gca gga gag gag aac tgc cag 624
aaa ctg acc aaa atc atc tgt gcc cag cag tgc tcc ggg cgc tgc cgt 672
ggc aag tcc ccc agt gac tgc tgc cac aac cag tgt gct gca ggc tgc 720
aca ggc ccc cgg gag agc gac tgc ctg gtc tgc cgc aaa ttc cga gac 768
gaa gcc acg tgc aag gac acc tgc ccc cca ctc atg ctc tac aac ccc 816
acc acg tac cag atg gat gtg aac ccc gag ggc aaa tac agc ttt ggt 864
gcc acc tgc gtg aag aag tgt ccc cgt aat tat gtg gtg aca gat cac 912
ggc tcg tgc gtc cga gcc tgt ggg gcc gac agc tat gag atg gag gaa 960
gac ggc gtc cgc aag tgt aag aag tgc gaa ggg cct tgc cgc aaa gtg 1008
tgt aac gga ata ggt att ggt gaa ttt aaa gac tca ctc tcc ata aat 1056
gct acg aat att aaa cac ttc aaa aac tgc acc tcc atc agt ggc gat 1104
ctc cac atc ctg ccg gtg gca ttt agg ggt gac tcc ttc aca cat act 1152
cct cct ctg gat cca cag gaa ctg gat att ctg aaa acc gta aag gaa 1200
atc aca ggg ttt ttg ctg att cag gct tgg cct gaa aac agg acg gac 1248
ctc cat gcc ttt gag aac cta gaa atc ata cgc ggc agg acc aag caa 1296
cat ggt cag ttt tct ctt gca gtc gtc agc ctg aac ata aca tcc ttg 1344
gga tta cgc tcc ctc aag gag ata agt gat gga gat gtg ata att tca 1392
gga aac aaa aat ttg tgc tat gca aat aca ata aac tgg aaa aaa ctg 1440
ttt ggg acc tcc ggt cag aaa acc aaa att ata agc aac aga ggt gaa 1488
aac agc tgc aag gcc aca ggc cag gtc tgc cat gcc ttg tgc tcc ccc 1536
gag ggc tgc tgg ggc ccg gag ccc agg gac tgc gtc tct tgc cgg aat 1584
gtc agc cga ggc agg gaa tgc gtg gac aag tgc aac ctt ctg gag ggt 1632
gag cca agg gag ttt gtg gag aac tct gag tgc ata cag tgc cac cca 1680
gag tgc ctg cct cag gcc atg aac atc acc tgc aca gga cgg gga cca 1728
gac aac tgt atc cag tgt gcc cac tac att gac ggc ccc cac tgc gtc 1776
aag acc tgc ccg gca gga gtc atg gga gaa aac aac acc ctg gtc tgg 1824
aag tac gca gac gcc ggc cat gtg tgc cac ctg tgc cat cca aac tgc 1872
acc tac gga tgc act ggg cca ggt ctt gaa ggc tgt cca acg aat ggg 1920
cct aag atc ccg tcc atc gcc act ggg atg gtg ggg gcc ctc ctc ttg 1968
ctg ctg gtg gtg gcc ctg ggg atc ggc ctc ttc atg cga agg cgc cac 2016
atc gtt cgg aag cgc acg ctg cgg agg ctg ctg cag gag agg gag ctt 2064
gtg gag cct ctt aca ccc agt gga gaa gct ccc aac caa gct ctc ttg 2112
agg atc ttg aag gaa act gaa ttc aaa aag atc aaa gtg ctg ggc tcc 2160
ggt gcg ttc ggc acg gtg tat aag gga ctc tgg atc cca gaa ggt gag 2208
aaa gtt aaa att ccc gtc gct atc aag gaa tta aga gaa gca aca tct 2256
ccg aaa gcc aac aag gaa atc ctc gat gaa gcc tac gtg atg gcc agc 2304
gtg gac aac ccc cac gtg tgc cgc ctg ctg ggc atc tgc ctc acc tcc 2352
acc gtg cag ctc atc acg cag ctc atg ccc ttc ggc tgc ctc ctg gac 2400
tat gtc cgg gaa cac aaa gac aat att ggc tcc cag tac ctg ctc aac 2448
tgg tgt gtg cag atc gca aag ggc atg aac tac ttg gag gac cgt cgc 2496
ttg gtg cac cgc gac ctg gca gcc agg aac gta ctg gtg aaa aca ccg 2544
cag cat gtc aag atc aca gat ttt ggg ctg gcc aaa ctg ctg ggt gcg 2592
gaa gag aaa gaa tac cat gca gaa gga ggc aaa gtg cct atc aag tgg 2640
atg gca ttg gaa tca att tta cac aga atc tat acc cac cag agt gat 2688
gtc tgg agc tac ggg gtg acc gtt tgg gag ttg atg acc ttt gga tcc 2736
aag cca tat gac gga atc cct gcc agc gag atc tcc tcc atc ctg gag     2784
aaa gga gaa cgc ctc cct cag cca ccc ata tgt acc atc gat gtc tac     2832
atg atc atg gtc aag tgc tgg atg ata gac gca gat agt cgc cca aag     2880
ttc cgt gag ttg atc atc gaa ttc tcc aaa atg gcc cga gac ccc cag     2928
cgc tac ctt gtc att cag ggg gat gaa aga atg cat ttg cca agt cct     2976
aca gac tcc aac ttc tac cgt gcc  ctg atg gat gaa gaa  gac atg gac     3024
gac gtg  gtg gat gcc gac gag  tac ctc atc cca cag  cag ggc ttc     3069
ttc agc  agc ccc tcc acg tca  cgg act ccc ctc ctg  agc tct ctg     3114
agt gca  acc agc aac aat tcc  acc gtg gct tgc att  gat aga aat     3159
ggg ctg  caa agc tgt ccc atc  aag gaa gac agc ttc  ttg cag cga     3204
tac agc  tca gac ccc aca ggc  gcc ttg act gag gac  agc ata gac     3249
gac acc  ttc ctc cca gtg cct  gaa tac ata aac cag  tcc gtt ccc     3294
aaa agg  ccc gct ggc tct gtg  cag aat cct gtc tat  cac aat cag     3339
cct ctg  aac ccc gcg ccc agc  aga gac cca cac tac  cag gac ccc     3384
cac agc  act gca gtg ggc aac  ccc gag tat ctc aac  act gtc cag     3429
ccc acc  tgt gtc aac agc aca  ttc gac agc cct gcc  cac tgg gcc     3474
cag aaa  ggc agc cac caa att  agc ctg gac aac cct  gac tac cag     3519
cag gac  ttc ttt ccc aag gaa  gcc aag cca aat ggc  atc ttt aag     3564
ggc tcc aca gct gaa aat gca gaa tac cta agg gtc gcg cca caa        3609
agc agt gaa ttt att gga gca tga                                    3633

<210> 2

<211> 1210

<212> PRT

<213> ÈË(homo sapiens)

<400> 2

<210>  3

<211>  1974

<212>  DNA

<213>  ÈË(homo sapiens)


<220>

<221>  CDS

<222>  (1)..(1971)

<223>

<400>  3
atg cga ccc tcc ggg acg gcc ggg gca gcg ctc ctg gcg ctg ctg gct      48
Met Arg Pro Ser Gly Thr Ala Gly Ala Ala Leu Leu Ala Leu Leu Ala
1               5                   10                  15

gcg ctc tgc ccg gcg agt cgg gct ctg gag gaa aag aaa gtt tgc caa      96
Ala Leu Cys Pro Ala Ser Arg Ala Leu Glu Glu Lys Lys Val Cys Gln
            20                  25                  30

ggc acg agt aac aag ctc acg cag ttg ggc act ttt gaa gat cat ttt      144
Gly Thr Ser Asn Lys Leu Thr Gln Leu Gly Thr Phe Glu Asp His Phe
        35                  40                  45

ctc agc ctc cag agg atg ttc aat aac tgt gag gtg gtc ctt ggg aat      192
Leu Ser Leu Gln Arg Met Phe Asn Asn Cys Glu Val Val Leu Gly Asn
    50                  55                  60

ttg gaa att acc tat gtg cag agg aat tat gat ctt tcc ttc tta aag      240
Leu Glu Ile Thr Tyr Val Gln Arg Asn Tyr Asp Leu Ser Phe Leu Lys
65                  70                  75                  80

acc atc cag gag gtg gct ggt tat gtc ctc att gcc ctc aac aca gtg      288
Thr Ile Gln Glu Val Ala Gly Tyr Val Leu Ile Ala Leu Asn Thr Val
                85                  90                  95

gag cga att cct ttg gaa aac ctg cag atc atc aga gga aat atg tac      336
Glu Arg Ile Pro Leu Glu Asn Leu Gln Ile Ile Arg Gly Asn Met Tyr
            100                 105                 110

tac gaa aat tcc tat gcc tta gca gtc tta tct aac tat gat gca aat      384
Tyr Glu Asn Ser Tyr Ala Leu Ala Val Leu Ser Asn Tyr Asp Ala Asn
        115                 120                 125

aaa acc gga ctg aag gag ctg ccc atg aga aat tta cag gaa atc ctg      432
Lys Thr Gly Leu Lys Glu Leu Pro Met Arg Asn Leu Gln Glu Ile Leu
    130                 135                 140

cat ggc gcc gtg cgg ttc agc aac aac cct gcc ctg tgc aac gtg gag      480
His Gly Ala Val Arg Phe Ser Asn Asn Pro Ala Leu Cys Asn Val Glu
145                 150                 155                 160

agc atc cag tgg cgg gac ata gtc agc agt gac ttt ctc agc aac atg      528
Ser Ile Gln Trp Arg Asp Ile Val Ser Ser Asp Phe Leu Ser Asn Met
                165                 170                 175

tcg atg gac ttc cag aac cac ctg ggc agc tgc caa aag tgt gat cca      576
Ser Met Asp Phe Gln Asn His Leu Gly Ser Cys Gln Lys Cys Asp Pro
            180                 185                 190

agc tgt ccc aat ggg agc tgc tgg ggt gca gga gag gag aac tgc cag      624
Ser Cys Pro Asn Gly Ser Cys Trp Gly Ala Gly Glu Glu Asn Cys Gln
        195                 200                 205

aaa ctg acc aaa atc atc tgt gcc cag cag tgc tcc ggg cgc tgc cgt      672
Lys Leu Thr Lys Ile Ile Cys Ala Gln Gln Cys Ser Gly Arg Cys Arg
    210                 215                 220

ggc aag tcc ccc agt gac tgc tgc cac aac cag tgt gct gca ggc tgc      720
Gly Lys Ser Pro Ser Asp Cys Cys His Asn Gln Cys Ala Ala Gly Cys
225                 230                 235                 240

aca ggc ccc cgg gag agc gac tgc ctg gtc tgc cgc aaa ttc cga gac      768

Thr Gly Pro Arg Glu Ser Asp Cys Leu Val Cys Arg Lys Phe Arg Asp
                245                 250                 255

gaa gcc acg tgc aag gac acc tgc ccc cca ctc atg ctc tac aac ccc      816
Glu Ala Thr Cys Lys Asp Thr Cys Pro Pro Leu Met Leu Tyr Asn Pro
            260                 265                 270

acc acg tac cag atg gat gtg aac ccc gag ggc aaa tac agc ttt ggt      864
Thr Thr Tyr Gln Met Asp Val Asn Pro Glu Gly Lys Tyr Ser Phe Gly
        275                 280                 285

gcc acc tgc gtg aag aag tgt ccc cgt aat tat gtg gtg aca gat cac      912
Ala Thr Cys Val Lys Lys Cys Pro Arg Asn Tyr Val Val Thr Asp His
    290                 295                 300

ggc tcg tgc gtc cga gcc tgt ggg gcc gac agc tat gag atg gag gaa      960
Gly Ser Cys Val Arg Ala Cys Gly Ala Asp Ser Tyr Glu Met Glu Glu
305                 310                 315                 320

gac ggc gtc cgc aag tgt aag aag tgc gaa ggg cct tgc cgc aaa gtg     1008
Asp Gly Val Arg Lys Cys Lys Lys Cys Glu Gly Pro Cys Arg Lys Val
                325                 330                 335

tgt aac gga ata ggt att ggt gaa ttt aaa gac tca ctc tcc ata aat     1056
Cys Asn Gly Ile Gly Ile Gly Glu Phe Lys Asp Ser Leu Ser Ile Asn
            340                 345                 350

gct acg aat att aaa cac ttc aaa aac tgc acc tcc atc agt ggc gat     1104
Ala Thr Asn Ile Lys His Phe Lys Asn Cys Thr Ser Ile Ser Gly Asp
        355                 360                 365

ctc cac atc ctg ccg gtg gca ttt agg ggt gac tcc ttc aca cat act     1152
Leu His Ile Leu Pro Val Ala Phe Arg Gly Asp Ser Phe Thr His Thr
    370                 375                 380

cct cct ctg gat cca cag gaa ctg gat att ctg aaa acc gta aag gaa     1200
Pro Pro Leu Asp Pro Gln Glu Leu Asp Ile Leu Lys Thr Val Lys Glu
385                 390                 395                 400

atc aca ggg ttt ttg ctg att cag gct tgg cct gaa aac agg acg gac     1248
Ile Thr Gly Phe Leu Leu Ile Gln Ala Trp Pro Glu Asn Arg Thr Asp
                405                 410                 415

ctc cat gcc ttt gag aac cta gaa atc ata cgc ggc agg acc aag caa     1296
Leu His Ala Phe Glu Asn Leu Glu Ile Ile Arg Gly Arg Thr Lys Gln
            420                 425                 430

cat ggt cag ttt tct ctt gca gtc gtc agc ctg aac ata aca tcc ttg     1344
His Gly Gln Phe Ser Leu Ala Val Val Ser Leu Asn Ile Thr Ser Leu
        435                 440                 445

gga tta cgc tcc ctc aag gag ata agt gat gga gat gtg ata att tca     1392
Gly Leu Arg Ser Leu Lys Glu Ile Ser Asp Gly Asp Val Ile Ile Ser
    450                 455                 460

gga aac aaa aat ttg tgc tat gca aat aca ata aac tgg aaa aaa ctg     1440
Gly Asn Lys Asn Leu Cys Tyr Ala Asn Thr Ile Asn Trp Lys Lys Leu
465                 470                 475                 480

ttt ggg acc tcc ggt cag aaa acc aaa att ata agc aac aga ggt gaa     1488
Phe Gly Thr Ser Gly Gln Lys Thr Lys Ile Ile Ser Asn Arg Gly Glu
                485                 490                 495

aac agc tgc aag gcc aca ggc cag gtc tgc cat gcc ttg tgc tcc ccc     1536
Asn Ser Cys Lys Ala Thr Gly Gln Val Cys His Ala Leu Cys Ser Pro
            500                 505                 510

gag ggc tgc tgg ggc ccg gag ccc agg gac tgc gtc tct tgc cgg aat     1584
Glu Gly Cys Trp Gly Pro Glu Pro Arg Asp Cys Val Ser Cys Arg Asn

515                    520                    525

gtc agc cga ggc agg gaa tgc gtg gac aag tgc aac ctt ctg gag ggt        1632
Val Ser Arg Gly Arg Glu Cys Val Asp Lys Cys Asn Leu Leu Glu Gly
    530                 535                 540

gag cca agg gag ttt gtg gag aac tct gag tgc ata cag tgc cac cca        1680
Glu Pro Arg Glu Phe Val Glu Asn Ser Glu Cys Ile Gln Cys His Pro
545                 550                 555                 560

gag tgc ctg cct cag gcc atg aac atc acc tgc aca gga cgg gga cca        1728
Glu Cys Leu Pro Gln Ala Met Asn Ile Thr Cys Thr Gly Arg Gly Pro
                565                 570                 575

gac aac tgt atc cag tgt gcc cac tac att gac ggc ccc cac tgc gtc        1776
Asp Asn Cys Ile Gln Cys Ala His Tyr Ile Asp Gly Pro His Cys Val
            580                 585                 590

aag acc tgc ccg gca gga gtc atg gga gaa aac aac acc ctg gtc tgg        1824
Lys Thr Cys Pro Ala Gly Val Met Gly Glu Asn Asn Thr Leu Val Trp
        595                 600                 605

aag tac gca gac gcc ggc cat gtg tgc cac ctg tgc cat cca aac tgc        1872
Lys Tyr Ala Asp Ala Gly His Val Cys His Leu Cys His Pro Asn Cys
    610                 615                 620

acc tac gga tgc act ggg cca ggt ctt gaa ggc tgt cca acg aat gga        1920
Thr Tyr Gly Cys Thr Gly Pro Gly Leu Glu Gly Cys Pro Thr Asn Gly
625                 630                 635                 640

agc tac ata gtg tct cac ttt cca aga tca ttc tac aag atg tca gtg        1968
Ser Tyr Ile Val Ser His Phe Pro Arg Ser Phe Tyr Lys Met Ser Val
                645                 650                 655

cac tga                                                                1974
His

<210>  4

<211>  657

<212>  PRT

<213>  ÈË(homo sapiens)

<400>  4

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
                165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
        195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Ala Asp Ser Tyr Glu Met Glu Glu
305                 310                 315                 320

Asp Gly Val Arg Lys Cys Lys Lys Cys Glu Gly Pro Cys Arg Lys Val
                325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
        515                 520                 525

Val Ser Arg Gly Arg Glu Cys Val Asp Lys Cys Asn Leu Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Val Met Gly Glu Asn Asn Thr Leu Val Trp
        595                 600                 605

Lys Tyr Ala Asp Ala Gly His Val Cys His Leu Cys His Pro Asn Cys
    610                 615                 620

Thr Tyr Gly Cys Thr Gly Pro Gly Leu Glu Gly Cys Pro Thr Asn Gly
625                 630                 635                 640

Ser Tyr Ile Val Ser His Phe Pro Arg Ser Phe Tyr Lys Met Ser Val
                645                 650                 655

His

<210>  5

<211>  2118

<212>  DNA

<213>  ÈË(homo sapiens)

<220>

<221>  CDS

<222>  (1)..(2115)

<223>

<400>  5
atg cga ccc tcc ggg acg gcc ggg gca gcg ctc ctg gcg ctg ctg gct      48
Met Arg Pro Ser Gly Thr Ala Gly Ala Ala Leu Leu Ala Leu Leu Ala
1               5                   10                  15

gcg ctc tgc ccg gcg agt cgg gct ctg gag gaa aag aaa gtt tgc caa      96
Ala Leu Cys Pro Ala Ser Arg Ala Leu Glu Glu Lys Lys Val Cys Gln
            20                  25                  30

ggc acg agt aac aag ctc acg cag ttg ggc act ttt gaa gat cat ttt      144
Gly Thr Ser Asn Lys Leu Thr Gln Leu Gly Thr Phe Glu Asp His Phe
        35                  40                  45

ctc agc ctc cag agg atg ttc aat aac tgt gag gtg gtc ctt ggg aat      192
Leu Ser Leu Gln Arg Met Phe Asn Asn Cys Glu Val Val Leu Gly Asn
    50                  55                  60

ttg gaa att acc tat gtg cag agg aat tat gat ctt tcc ttc tta aag      240
Leu Glu Ile Thr Tyr Val Gln Arg Asn Tyr Asp Leu Ser Phe Leu Lys
65                  70                  75                  80

acc atc cag gag gtg gct ggt tat gtc ctc att gcc ctc aac aca gtg      288
Thr Ile Gln Glu Val Ala Gly Tyr Val Leu Ile Ala Leu Asn Thr Val
                85                  90                  95

gag cga att cct ttg gaa aac ctg cag atc atc aga gga aat atg tac      336
Glu Arg Ile Pro Leu Glu Asn Leu Gln Ile Ile Arg Gly Asn Met Tyr
            100                 105                 110

tac gaa aat tcc tat gcc tta gca gtc tta tct aac tat gat gca aat      384
Tyr Glu Asn Ser Tyr Ala Leu Ala Val Leu Ser Asn Tyr Asp Ala Asn
        115                 120                 125

aaa acc gga ctg aag gag ctg ccc atg aga aat tta cag gaa atc ctg      432
Lys Thr Gly Leu Lys Glu Leu Pro Met Arg Asn Leu Gln Glu Ile Leu
    130                 135                 140

cat ggc gcc gtg cgg ttc agc aac aac cct gcc ctg tgc aac gtg gag 480
His Gly Ala Val Arg Phe Ser Asn Asn Pro Ala Leu Cys Asn Val Glu
145                 150                 155                 160

agc atc cag tgg cgg gac ata gtc agc agt gac ttt ctc agc aac atg 528
Ser Ile Gln Trp Arg Asp Ile Val Ser Ser Asp Phe Leu Ser Asn Met
                165                 170                 175

tcg atg gac ttc cag aac cac ctg ggc agc tgc caa aag tgt gat cca 576
Ser Met Asp Phe Gln Asn His Leu Gly Ser Cys Gln Lys Cys Asp Pro
            180                 185                 190

agc tgt ccc aat ggg agc tgc tgg ggt gca gga gag gag aac tgc cag 624
Ser Cys Pro Asn Gly Ser Cys Trp Gly Ala Gly Glu Glu Asn Cys Gln
        195                 200                 205

aaa ctg acc aaa atc atc tgt gcc cag cag tgc tcc ggg cgc tgc cgt 672
Lys Leu Thr Lys Ile Ile Cys Ala Gln Gln Cys Ser Gly Arg Cys Arg
    210                 215                 220

ggc aag tcc ccc agt gac tgc tgc cac aac cag tgt gct gca ggc tgc 720
Gly Lys Ser Pro Ser Asp Cys Cys His Asn Gln Cys Ala Ala Gly Cys
225                 230                 235                 240

aca ggc ccc cgg gag agc gac tgc ctg gtc tgc cgc aaa ttc cga gac 768
Thr Gly Pro Arg Glu Ser Asp Cys Leu Val Cys Arg Lys Phe Arg Asp
                245                 250                 255

gaa gcc acg tgc aag gac acc tgc ccc cca ctc atg ctc tac aac ccc 816
Glu Ala Thr Cys Lys Asp Thr Cys Pro Pro Leu Met Leu Tyr Asn Pro
            260                 265                 270

acc acg tac cag atg gat gtg aac ccc gag ggc aaa tac agc ttt ggt 864
Thr Thr Tyr Gln Met Asp Val Asn Pro Glu Gly Lys Tyr Ser Phe Gly
        275                 280                 285

gcc acc tgc gtg aag aag tgt ccc cgt aat tat gtg gtg aca gat cac 912
Ala Thr Cys Val Lys Lys Cys Pro Arg Asn Tyr Val Val Thr Asp His
    290                 295                 300

ggc tcg tgc gtc cga gcc tgt ggg gcc gac agc tat gag atg gag gaa 960
Gly Ser Cys Val Arg Ala Cys Gly Ala Asp Ser Tyr Glu Met Glu Glu
305                 310                 315                 320

gac ggc gtc cgc aag tgt aag aag tgc gaa ggg cct tgc cgc aaa gtg 1008
Asp Gly Val Arg Lys Cys Lys Lys Cys Glu Gly Pro Cys Arg Lys Val
                325                 330                 335

tgt aac gga ata ggt att ggt gaa ttt aaa gac tca ctc tcc ata aat 1056
Cys Asn Gly Ile Gly Ile Gly Glu Phe Lys Asp Ser Leu Ser Ile Asn
            340                 345                 350

gct acg aat att aaa cac ttc aaa aac tgc acc tcc atc agt ggc gat 1104
Ala Thr Asn Ile Lys His Phe Lys Asn Cys Thr Ser Ile Ser Gly Asp
        355                 360                 365

ctc cac atc ctg ccg gtg gca ttt agg ggt gac tcc ttc aca cat act 1152
Leu His Ile Leu Pro Val Ala Phe Arg Gly Asp Ser Phe Thr His Thr
    370                 375                 380

cct cct ctg gat cca cag gaa ctg gat att ctg aaa acc gta aag gaa 1200
Pro Pro Leu Asp Pro Gln Glu Leu Asp Ile Leu Lys Thr Val Lys Glu
385                 390                 395                 400

atc aca ggg ttt ttg ctg att cag gct tgg cct gaa aac agg acg gac 1248
Ile Thr Gly Phe Leu Leu Ile Gln Ala Trp Pro Glu Asn Arg Thr Asp
                405                 410                 415

ctc cat gcc ttt gag aac cta gaa atc ata cgc ggc agg acc aag caa 1296

Leu His Ala Phe Glu Asn Leu Glu Ile Ile Arg Gly Arg Thr Lys Gln
            420                 425                 430

cat ggt cag ttt tct ctt gca gtc gtc agc ctg aac ata aca tcc ttg 1344
His Gly Gln Phe Ser Leu Ala Val Val Ser Leu Asn Ile Thr Ser Leu
        435                 440                 445

gga tta cgc tcc ctc aag gag ata agt gat gga gat gtg ata att tca 1392
Gly Leu Arg Ser Leu Lys Glu Ile Ser Asp Gly Asp Val Ile Ile Ser
    450                 455                 460

gga aac aaa aat ttg tgc tat gca aat aca ata aac tgg aaa aaa ctg 1440
Gly Asn Lys Asn Leu Cys Tyr Ala Asn Thr Ile Asn Trp Lys Lys Leu
465                 470                 475                 480

ttt ggg acc tcc ggt cag aaa acc aaa att ata agc aac aga ggt gaa 1488
Phe Gly Thr Ser Gly Gln Lys Thr Lys Ile Ile Ser Asn Arg Gly Glu
                485                 490                 495

aac agc tgc aag gcc aca ggc cag gtc tgc cat gcc ttg tgc tcc ccc 1536
Asn Ser Cys Lys Ala Thr Gly Gln Val Cys His Ala Leu Cys Ser Pro
            500                 505                 510

gag ggc tgc tgg ggc ccg gag ccc agg gac tgc gtc tct tgc cgg aat 1584
Glu Gly Cys Trp Gly Pro Glu Pro Arg Asp Cys Val Ser Cys Arg Asn
        515                 520                 525

gtc agc cga ggc agg gaa tgc gtg gac aag tgc aac ctt ctg gag ggt 1632
Val Ser Arg Gly Arg Glu Cys Val Asp Lys Cys Asn Leu Leu Glu Gly
    530                 535                 540

gag cca agg gag ttt gtg gag aac tct gag tgc ata cag tgc cac cca 1680
Glu Pro Arg Glu Phe Val Glu Asn Ser Glu Cys Ile Gln Cys His Pro
545                 550                 555                 560

gag tgc ctg cct cag gcc atg aac atc acc tgc aca gga cgg gga cca 1728
Glu Cys Leu Pro Gln Ala Met Asn Ile Thr Cys Thr Gly Arg Gly Pro
                565                 570                 575

gac aac tgt atc cag tgt gcc cac tac att gac ggc ccc cac tgc gtc 1776
Asp Asn Cys Ile Gln Cys Ala His Tyr Ile Asp Gly Pro His Cys Val
            580                 585                 590

aag acc tgc ccg gca gga gtc atg gga gaa aac aac acc ctg gtc tgg 1824
Lys Thr Cys Pro Ala Gly Val Met Gly Glu Asn Asn Thr Leu Val Trp
        595                 600                 605

aag tac gca gac gcc ggc cat gtg tgc cac ctg tgc cat cca aac tgc 1872
Lys Tyr Ala Asp Ala Gly His Val Cys His Leu Cys His Pro Asn Cys
    610                 615                 620

acc tac ggg cca gga aat gag agt ctc aaa gcc atg tta ttc tgc ctt 1920
Thr Tyr Gly Pro Gly Asn Glu Ser Leu Lys Ala Met Leu Phe Cys Leu
625                 630                 635                 640

ttt aaa cta tca tcc tgt aat caa agt aat gat ggc agc gtg tcc cac 1968
Phe Lys Leu Ser Ser Cys Asn Gln Ser Asn Asp Gly Ser Val Ser His
                645                 650                 655

cag agc ggg agc cca gct gct cag gag tca tgc tta gga tgg atc cct 2016
Gln Ser Gly Ser Pro Ala Ala Gln Glu Ser Cys Leu Gly Trp Ile Pro
            660                 665                 670

tct ctt ctg ccg tca gag ttt cag ctg ggt tgg ggt gga tgc agc cac 2064
Ser Leu Leu Pro Ser Glu Phe Gln Leu Gly Trp Gly Gly Cys Ser His
        675                 680                 685

ctc cat gcc tgg cct tct gca tct gtg atc atc acg gcc tcc tcc tgc 2112
Leu His Ala Trp Pro Ser Ala Ser Val Ile Ile Thr Ala Ser Ser Cys

690                    695                    700

cac tga                                                                    2118
His
705

<210>  6

<211>  705

<212>  PRT

<213>  ÈË(homo sapiens)

<400>  6

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
                165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
        195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Ala Asp Ser Tyr Glu Met Glu Glu
305                 310                 315                 320

Asp Gly Val Arg Lys Cys Lys Lys Cys Glu Gly Pro Cys Arg Lys Val
                325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
        515                 520                 525

Val Ser Arg Gly Arg Glu Cys Val Asp Lys Cys Asn Leu Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Val Met Gly Glu Asn Asn Thr Leu Val Trp
        595                 600                 605

Lys Tyr Ala Asp Ala Gly His Val Cys His Leu Cys His Pro Asn Cys
    610                 615                 620

Thr Tyr Gly Pro Gly Asn Glu Ser Leu Lys Ala Met Leu Phe Cys Leu
625                 630                 635                 640

Phe Lys Leu Ser Ser Cys Asn Gln Ser Asn Asp Gly Ser Val Ser His
                645                 650                 655

Gln Ser Gly Ser Pro Ala Ala Gln Glu Ser Cys Leu Gly Trp Ile Pro
            660                 665                 670

Ser Leu Leu Pro Ser Glu Phe Gln Leu Gly Trp Gly Gly Cys Ser His
        675                 680                 685

Leu His Ala Trp Pro Ser Ala Ser Val Ile Ile Thr Ala Ser Ser Cys
    690                 695                 700

His
705

<210>  7

<211>  1887

<212>  DNA

<213>  ÈË(homo sapiens)

<220>

<221>  CDS

<222>  (1)..(1884)

<223>

<400>  7
atg cga ccc tcc ggg acg gcc ggg gca gcg ctc ctg gcg ctg ctg gct      48
Met Arg Pro Ser Gly Thr Ala Gly Ala Ala Leu Leu Ala Leu Leu Ala
1               5                   10                  15

gcg ctc tgc ccg gcg agt cgg gct ctg gag gaa aag aaa gtt tgc caa      96
Ala Leu Cys Pro Ala Ser Arg Ala Leu Glu Glu Lys Lys Val Cys Gln
            20                  25                  30

ggc acg agt aac aag ctc acg cag ttg ggc act ttt gaa gat cat ttt      144
Gly Thr Ser Asn Lys Leu Thr Gln Leu Gly Thr Phe Glu Asp His Phe
        35                  40                  45

ctc agc ctc cag agg atg ttc aat aac tgt gag gtg gtc ctt ggg aat      192
Leu Ser Leu Gln Arg Met Phe Asn Asn Cys Glu Val Val Leu Gly Asn
    50                  55                  60

ttg gaa att acc tat gtg cag agg aat tat gat ctt tcc ttc tta aag      240
Leu Glu Ile Thr Tyr Val Gln Arg Asn Tyr Asp Leu Ser Phe Leu Lys
65                  70                  75                  80

acc atc cag gag gtg gct ggt tat gtc ctc att gcc ctc aac aca gtg      288
Thr Ile Gln Glu Val Ala Gly Tyr Val Leu Ile Ala Leu Asn Thr Val
                85                  90                  95

gag cga att cct ttg gaa aac ctg cag atc atc aga gga aat atg tac      336
Glu Arg Ile Pro Leu Glu Asn Leu Gln Ile Ile Arg Gly Asn Met Tyr
            100                 105                 110

tac gaa aat tcc tat gcc tta gca gtc tta tct aac tat gat gca aat      384
Tyr Glu Asn Ser Tyr Ala Leu Ala Val Leu Ser Asn Tyr Asp Ala Asn
        115                 120                 125

aaa acc gga ctg aag gag ctg ccc atg aga aat tta cag gaa atc ctg      432
Lys Thr Gly Leu Lys Glu Leu Pro Met Arg Asn Leu Gln Glu Ile Leu
    130                 135                 140

cat ggc gcc gtg cgg ttc agc aac aac cct gcc ctg tgc aac gtg gag      480
His Gly Ala Val Arg Phe Ser Asn Asn Pro Ala Leu Cys Asn Val Glu
145                 150                 155                 160

agc atc cag tgg cgg gac ata gtc agc agt gac ttt ctc agc aac atg      528
Ser Ile Gln Trp Arg Asp Ile Val Ser Ser Asp Phe Leu Ser Asn Met
                165                 170                 175

tcg atg gac ttc cag aac cac ctg ggc agc tgc caa aag tgt gat cca      576
Ser Met Asp Phe Gln Asn His Leu Gly Ser Cys Gln Lys Cys Asp Pro
            180                 185                 190

agc tgt ccc aat ggg agc tgc tgg ggt gca gga gag gag aac tgc cag      624
Ser Cys Pro Asn Gly Ser Cys Trp Gly Ala Gly Glu Glu Asn Cys Gln
        195                 200                 205

aaa ctg acc aaa atc atc tgt gcc cag cag tgc tcc ggg cgc tgc cgt      672
Lys Leu Thr Lys Ile Ile Cys Ala Gln Gln Cys Ser Gly Arg Cys Arg
    210                 215                 220

ggc aag tcc ccc agt gac tgc tgc cac aac cag tgt gct gca ggc tgc 720
Gly Lys Ser Pro Ser Asp Cys Cys His Asn Gln Cys Ala Ala Gly Cys
225                 230                 235                 240

aca ggc ccc cgg gag agc gac tgc ctg gtc tgc cgc aaa ttc cga gac 768
Thr Gly Pro Arg Glu Ser Asp Cys Leu Val Cys Arg Lys Phe Arg Asp
                245                 250                 255

gaa gcc acg tgc aag gac acc tgc ccc cca ctc atg ctc tac aac ccc 816
Glu Ala Thr Cys Lys Asp Thr Cys Pro Pro Leu Met Leu Tyr Asn Pro
            260                 265                 270

acc acg tac cag atg gat gtg aac ccc gag ggc aaa tac agc ttt ggt 864
Thr Thr Tyr Gln Met Asp Val Asn Pro Glu Gly Lys Tyr Ser Phe Gly
        275                 280                 285

gcc acc tgc gtg aag aag tgt ccc cgt aat tat gtg gtg aca gat cac 912
Ala Thr Cys Val Lys Lys Cys Pro Arg Asn Tyr Val Val Thr Asp His
    290                 295                 300

ggc tcg tgc gtc cga gcc tgt ggg gcc gac agc tat gag atg gag gaa 960
Gly Ser Cys Val Arg Ala Cys Gly Ala Asp Ser Tyr Glu Met Glu Glu
305                 310                 315                 320

gac ggc gtc cgc aag tgt aag aag tgc gaa ggg cct tgc cgc aaa gtg 1008
Asp Gly Val Arg Lys Cys Lys Lys Cys Glu Gly Pro Cys Arg Lys Val
                325                 330                 335

tgt aac gga ata ggt att ggt gaa ttt aaa gac tca ctc tcc ata aat 1056
Cys Asn Gly Ile Gly Ile Gly Glu Phe Lys Asp Ser Leu Ser Ile Asn
            340                 345                 350

gct acg aat att aaa cac ttc aaa aac tgc acc tcc atc agt ggc gat 1104
Ala Thr Asn Ile Lys His Phe Lys Asn Cys Thr Ser Ile Ser Gly Asp
        355                 360                 365

ctc cac atc ctg ccg gtg gca ttt agg ggt gac tcc ttc aca cat act 1152
Leu His Ile Leu Pro Val Ala Phe Arg Gly Asp Ser Phe Thr His Thr
    370                 375                 380

cct cct ctg gat cca cag gaa ctg gat att ctg aaa acc gta aag gaa 1200
Pro Pro Leu Asp Pro Gln Glu Leu Asp Ile Leu Lys Thr Val Lys Glu
385                 390                 395                 400

atc aca ggg ttt ttg ctg att cag gct tgg cct gaa aac agg acg gac 1248
Ile Thr Gly Phe Leu Leu Ile Gln Ala Trp Pro Glu Asn Arg Thr Asp
                405                 410                 415

ctc cat gcc ttt gag aac cta gaa atc ata cgc ggc agg acc aag caa 1296
Leu His Ala Phe Glu Asn Leu Glu Ile Ile Arg Gly Arg Thr Lys Gln
            420                 425                 430

cat ggt cag ttt tct ctt gca gtc gtc agc ctg aac ata aca tcc ttg 1344
His Gly Gln Phe Ser Leu Ala Val Val Ser Leu Asn Ile Thr Ser Leu
        435                 440                 445

gga tta cgc tcc ctc aag gag ata agt gat gga gat gtg ata att tca 1392
Gly Leu Arg Ser Leu Lys Glu Ile Ser Asp Gly Asp Val Ile Ile Ser
    450                 455                 460

gga aac aaa aat ttg tgc tat gca aat aca ata aac tgg aaa aaa ctg 1440
Gly Asn Lys Asn Leu Cys Tyr Ala Asn Thr Ile Asn Trp Lys Lys Leu
465                 470                 475                 480

ttt ggg acc tcc ggt cag aaa acc aaa att ata agc aac aga ggt gaa 1488
Phe Gly Thr Ser Gly Gln Lys Thr Lys Ile Ile Ser Asn Arg Gly Glu
                485                 490                 495

aac agc tgc aag gcc aca ggc cag gtc tgc cat gcc ttg tgc tcc ccc 1536

Asn Ser Cys Lys Ala Thr Gly Gln Val Cys His Ala Leu Cys Ser Pro
            500                 505                 510

gag ggc tgc tgg ggc ccg gag ccc agg gac tgc gtc tct tgc cgg aat 1584
Glu Gly Cys Trp Gly Pro Glu Pro Arg Asp Cys Val Ser Cys Arg Asn
        515                 520                 525

gtc agc cga ggc agg gaa tgc gtg gac aag tgc aac ctt ctg gag ggt 1632
Val Ser Arg Gly Arg Glu Cys Val Asp Lys Cys Asn Leu Leu Glu Gly
    530                 535                 540

gag cca agg gag ttt gtg gag aac tct gag tgc ata cag tgc cac cca 1680
Glu Pro Arg Glu Phe Val Glu Asn Ser Glu Cys Ile Gln Cys His Pro
545                 550                 555                 560

gag tgc ctg cct cag gcc atg aac atc acc tgc aca gga cgg gga cca 1728
Glu Cys Leu Pro Gln Ala Met Asn Ile Thr Cys Thr Gly Arg Gly Pro
                565                 570                 575

gac aac tgt atc cag tgt gcc cac tac att gac ggc ccc cac tgc gtc 1776
Asp Asn Cys Ile Gln Cys Ala His Tyr Ile Asp Gly Pro His Cys Val
            580                 585                 590

aag acc tgc ccg gca gga gtc atg gga gaa aac aac acc ctg gtc tgg 1824
Lys Thr Cys Pro Ala Gly Val Met Gly Glu Asn Asn Thr Leu Val Trp
        595                 600                 605

aag tac gca gac gcc ggc cat gtg tgc cac ctg tgc cat cca aac tgc 1872
Lys Tyr Ala Asp Ala Gly His Val Cys His Leu Cys His Pro Asn Cys
    610                 615                 620

acc tac ggg tcc taa 1887
Thr Tyr Gly Ser
625

<210> 8

<211> 628

<212> PRT

<213> ÈË(homo sapiens)

<400> 8

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val

                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
                165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
        195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Ala Asp Ser Tyr Glu Met Glu Glu
305                 310                 315                 320

Asp Gly Val Arg Lys Cys Lys Lys Cys Glu Gly Pro Cys Arg Lys Val
                325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
        515                 520                 525

Val Ser Arg Gly Arg Glu Cys Val Asp Lys Cys Asn Leu Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Val Met Gly Glu Asn Asn Thr Leu Val Trp
        595                 600                 605

Lys Tyr Ala Asp Ala Gly His Val Cys His Leu Cys His Pro Asn Cys
    610                 615                 620

Thr Tyr Gly Ser
625

<210>  9

<211>  1218

<212>  DNA

<213>  ÈË(homo sapiens)

<220>

<221>  CDS

<222>  (1)..(1215)

<223>

<400>  9
atg cga ccc tcc ggg acg gcc ggg gca gcg ctc ctg gcg ctg ctg gct      48
Met Arg Pro Ser Gly Thr Ala Gly Ala Ala Leu Leu Ala Leu Leu Ala
1               5                   10                  15

gcg ctc tgc ccg gcg agt cgg gct ctg gag gaa aag aaa gtt tgc caa      96
Ala Leu Cys Pro Ala Ser Arg Ala Leu Glu Glu Lys Lys Val Cys Gln
            20                  25                  30

ggc acg agt aac aag ctc acg cag ttg ggc act ttt gaa gat cat ttt      144
Gly Thr Ser Asn Lys Leu Thr Gln Leu Gly Thr Phe Glu Asp His Phe
        35                  40                  45

ctc agc ctc cag agg atg ttc aat aac tgt gag gtg gtc ctt ggg aat      192
Leu Ser Leu Gln Arg Met Phe Asn Asn Cys Glu Val Val Leu Gly Asn
    50                  55                  60

ttg gaa att acc tat gtg cag agg aat tat gat ctt tcc ttc tta aag      240
Leu Glu Ile Thr Tyr Val Gln Arg Asn Tyr Asp Leu Ser Phe Leu Lys
65                  70                  75                  80

acc atc cag gag gtg gct ggt tat gtc ctc att gcc ctc aac aca gtg      288
Thr Ile Gln Glu Val Ala Gly Tyr Val Leu Ile Ala Leu Asn Thr Val
                85                  90                  95

gag cga att cct ttg gaa aac ctg cag atc atc aga gga aat atg tac      336
Glu Arg Ile Pro Leu Glu Asn Leu Gln Ile Ile Arg Gly Asn Met Tyr
            100                 105                 110

tac gaa aat tcc tat gcc tta gca gtc tta tct aac tat gat gca aat      384
Tyr Glu Asn Ser Tyr Ala Leu Ala Val Leu Ser Asn Tyr Asp Ala Asn
        115                 120                 125

aaa acc gga ctg aag gag ctg ccc atg aga aat tta cag gaa atc ctg      432
Lys Thr Gly Leu Lys Glu Leu Pro Met Arg Asn Leu Gln Glu Ile Leu
    130                 135                 140

cat ggc gcc gtg cgg ttc agc aac aac cct gcc ctg tgc aac gtg gag      480
His Gly Ala Val Arg Phe Ser Asn Asn Pro Ala Leu Cys Asn Val Glu
145                 150                 155                 160

agc atc cag tgg cgg gac ata gtc agc agt gac ttt ctc agc aac atg      528
Ser Ile Gln Trp Arg Asp Ile Val Ser Ser Asp Phe Leu Ser Asn Met
                165                 170                 175

tcg atg gac ttc cag aac cac ctg ggc agc tgc caa aag tgt gat cca      576
Ser Met Asp Phe Gln Asn His Leu Gly Ser Cys Gln Lys Cys Asp Pro
            180                 185                 190

agc tgt ccc aat ggg agc tgc tgg ggt gca gga gag gag aac tgc cag     624
Ser Cys Pro Asn Gly Ser Cys Trp Gly Ala Gly Glu Glu Asn Cys Gln
        195                 200                 205

aaa ctg acc aaa atc atc tgt gcc cag cag tgc tcc ggg cgc tgc cgt     672
Lys Leu Thr Lys Ile Ile Cys Ala Gln Gln Cys Ser Gly Arg Cys Arg
    210                 215                 220

ggc aag tcc ccc agt gac tgc tgc cac aac cag tgt gct gca ggc tgc     720
Gly Lys Ser Pro Ser Asp Cys Cys His Asn Gln Cys Ala Ala Gly Cys
225                 230                 235                 240

aca ggc ccc cgg gag agc gac tgc ctg gtc tgc cgc aaa ttc cga gac     768
Thr Gly Pro Arg Glu Ser Asp Cys Leu Val Cys Arg Lys Phe Arg Asp
                245                 250                 255

gaa gcc acg tgc aag gac acc tgc ccc cca ctc atg ctc tac aac ccc     816
Glu Ala Thr Cys Lys Asp Thr Cys Pro Pro Leu Met Leu Tyr Asn Pro
            260                 265                 270

acc acg tac cag atg gat gtg aac ccc gag ggc aaa tac agc ttt ggt     864
Thr Thr Tyr Gln Met Asp Val Asn Pro Glu Gly Lys Tyr Ser Phe Gly
        275                 280                 285

gcc acc tgc gtg aag aag tgt ccc cgt aat tat gtg gtg aca gat cac     912
Ala Thr Cys Val Lys Lys Cys Pro Arg Asn Tyr Val Val Thr Asp His
    290                 295                 300

ggc tcg tgc gtc cga gcc tgt ggg gcc gac agc tat gag atg gag gaa     960
Gly Ser Cys Val Arg Ala Cys Gly Ala Asp Ser Tyr Glu Met Glu Glu
305                 310                 315                 320

gac ggc gtc cgc aag tgt aag aag tgc gaa ggg cct tgc cgc aaa gtg     1008
Asp Gly Val Arg Lys Cys Lys Lys Cys Glu Gly Pro Cys Arg Lys Val
                325                 330                 335

tgt aac gga ata ggt att ggt gaa ttt aaa gac tca ctc tcc ata aat     1056
Cys Asn Gly Ile Gly Ile Gly Glu Phe Lys Asp Ser Leu Ser Ile Asn
            340                 345                 350

gct acg aat att aaa cac ttc aaa aac tgc acc tcc atc agt ggc gat     1104
Ala Thr Asn Ile Lys His Phe Lys Asn Cys Thr Ser Ile Ser Gly Asp
        355                 360                 365

ctc cac atc ctg ccg gtg gca ttt agg ggt gac tcc ttc aca cat act     1152
Leu His Ile Leu Pro Val Ala Phe Arg Gly Asp Ser Phe Thr His Thr
    370                 375                 380

cct cct ctg gat cca cag gaa ctg gat att ctg aaa acc gta aag gaa     1200
Pro Pro Leu Asp Pro Gln Glu Leu Asp Ile Leu Lys Thr Val Lys Glu
385                 390                 395                 400

atc aca ggt ttg agc tga                                             1218
Ile Thr Gly Leu Ser
                405

<210>  10

<211>  405

<212>  PRT

<213>  ÈË(homo sapiens)


<400>  10

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Met Tyr
            100                 105                 110

Tyr Glu Asn Ser Tyr Ala Leu Ala Val Leu Ser Asn Tyr Asp Ala Asn
        115                 120                 125

Lys Thr Gly Leu Lys Glu Leu Pro Met Arg Asn Leu Gln Glu Ile Leu
    130                 135                 140

His Gly Ala Val Arg Phe Ser Asn Asn Pro Ala Leu Cys Asn Val Glu
145                 150                 155                 160

Ser Ile Gln Trp Arg Asp Ile Val Ser Ser Asp Phe Leu Ser Asn Met
                165                 170                 175

Ser Met Asp Phe Gln Asn His Leu Gly Ser Cys Gln Lys Cys Asp Pro
            180                 185                 190

Ser Cys Pro Asn Gly Ser Cys Trp Gly Ala Gly Glu Glu Asn Cys Gln
        195                 200                 205

Lys Leu Thr Lys Ile Ile Cys Ala Gln Gln Cys Ser Gly Arg Cys Arg
    210                 215                 220

Gly Lys Ser Pro Ser Asp Cys Cys His Asn Gln Cys Ala Ala Gly Cys
225                 230                 235                 240

Thr Gly Pro Arg Glu Ser Asp Cys Leu Val Cys Arg Lys Phe Arg Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Ala Asp Ser Tyr Glu Met Glu Glu
305                 310                 315                 320

Asp Gly Val Arg Lys Cys Lys Lys Cys Glu Gly Pro Cys Arg Lys Val
                325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
        355                 360                 365

Leu His Ile Leu Pro Val Ala Phe Arg Gly Asp Ser Phe Thr His Thr
    370                 375                 380

Pro Pro Leu Asp Pro Gln Glu Leu Asp Ile Leu Lys Thr Val Lys Glu
385                 390                 395                 400

Ile Thr Gly Leu Ser
                405

<210>  11

<211>  2829

<212>  DNA

<213>  ÈË(homo sapiens)


<220>

<221>  CDS

<222>  (1)..(2826)

<223>


<400>  11
atg cga ccc tcc ggg acg gcc ggg gca gcg ctc ctg gcg ctg ctg gct      48
Met Arg Pro Ser Gly Thr Ala Gly Ala Ala Leu Leu Ala Leu Leu Ala
1               5                   10                  15

gcg ctc tgc ccg gcg agt cgg gct ctg gag gaa aag cgt aat tat gtg      96
Ala Leu Cys Pro Ala Ser Arg Ala Leu Glu Glu Lys Arg Asn Tyr Val
            20                  25                  30

gtg aca gat cac ggc tcg tgc gtc cga gcc tgt ggg gcc gac agc tat      144
Val Thr Asp His Gly Ser Cys Val Arg Ala Cys Gly Ala Asp Ser Tyr
        35                  40                  45

gag atg gag gaa gac ggc gtc cgc aag tgt aag aag tgc gaa ggg cct 192
Glu Met Glu Glu Asp Gly Val Arg Lys Cys Lys Lys Cys Glu Gly Pro
    50                  55                  60

tgc cgc aaa gtg tgt aac gga ata ggt att ggt gaa ttt aaa gac tca 240
Cys Arg Lys Val Cys Asn Gly Ile Gly Ile Gly Glu Phe Lys Asp Ser
65                  70                  75                  80

ctc tcc ata aat gct acg aat att aaa cac ttc aaa aac tgc acc tcc 288
Leu Ser Ile Asn Ala Thr Asn Ile Lys His Phe Lys Asn Cys Thr Ser
                85                  90                  95

atc agt ggc gat ctc cac atc ctg ccg gtg gca ttt agg ggt gac tcc 336
Ile Ser Gly Asp Leu His Ile Leu Pro Val Ala Phe Arg Gly Asp Ser
            100                 105                 110

ttc aca cat act cct cct ctg gat cca cag gaa ctg gat att ctg aaa 384
Phe Thr His Thr Pro Pro Leu Asp Pro Gln Glu Leu Asp Ile Leu Lys
        115                 120                 125

acc gta aag gaa atc aca ggg ttt ttg ctg att cag gct tgg cct gaa 432
Thr Val Lys Glu Ile Thr Gly Phe Leu Leu Ile Gln Ala Trp Pro Glu
    130                 135                 140

aac agg acg gac ctc cat gcc ttt gag aac cta gaa atc ata cgc ggc 480
Asn Arg Thr Asp Leu His Ala Phe Glu Asn Leu Glu Ile Ile Arg Gly
145                 150                 155                 160

agg acc aag caa cat ggt cag ttt tct ctt gca gtc gtc agc ctg aac 528
Arg Thr Lys Gln His Gly Gln Phe Ser Leu Ala Val Val Ser Leu Asn
                165                 170                 175

ata aca tcc ttg gga tta cgc tcc ctc aag gag ata agt gat gga gat 576
Ile Thr Ser Leu Gly Leu Arg Ser Leu Lys Glu Ile Ser Asp Gly Asp
            180                 185                 190

gtg ata att tca gga aac aaa aat ttg tgc tat gca aat aca ata aac 624
Val Ile Ile Ser Gly Asn Lys Asn Leu Cys Tyr Ala Asn Thr Ile Asn
        195                 200                 205

tgg aaa aaa ctg ttt ggg acc tcc ggt cag aaa acc aaa att ata agc 672
Trp Lys Lys Leu Phe Gly Thr Ser Gly Gln Lys Thr Lys Ile Ile Ser
    210                 215                 220

aac aga ggt gaa aac agc tgc aag gcc aca ggc cag gtc tgc cat gcc 720
Asn Arg Gly Glu Asn Ser Cys Lys Ala Thr Gly Gln Val Cys His Ala
225                 230                 235                 240

ttg tgc tcc ccc gag ggc tgc tgg ggc ccg gag ccc agg gac tgc gtc 768
Leu Cys Ser Pro Glu Gly Cys Trp Gly Pro Glu Pro Arg Asp Cys Val
                245                 250                 255

tct tgc cgg aat gtc agc cga ggc agg gaa tgc gtg gac aag tgc aac 816
Ser Cys Arg Asn Val Ser Arg Gly Arg Glu Cys Val Asp Lys Cys Asn
            260                 265                 270

ctt ctg gag ggt gag cca agg gag ttt gtg gag aac tct gag tgc ata 864
Leu Leu Glu Gly Glu Pro Arg Glu Phe Val Glu Asn Ser Glu Cys Ile
        275                 280                 285

cag tgc cac cca gag tgc ctg cct cag gcc atg aac atc acc tgc aca 912
Gln Cys His Pro Glu Cys Leu Pro Gln Ala Met Asn Ile Thr Cys Thr
    290                 295                 300

gga cgg gga cca gac aac tgt atc cag tgt gcc cac tac att gac ggc 960
Gly Arg Gly Pro Asp Asn Cys Ile Gln Cys Ala His Tyr Ile Asp Gly
305                 310                 315                 320

ccc cac tgc gtc aag acc tgc ccg gca gga gtc atg gga gaa aac aac 1008

Pro His Cys Val Lys Thr Cys Pro Ala Gly Val Met Gly Glu Asn Asn
                325                 330                 335

acc ctg gtc tgg aag tac gca gac gcc ggc cat gtg tgc cac ctg tgc 1056
Thr Leu Val Trp Lys Tyr Ala Asp Ala Gly His Val Cys His Leu Cys
            340                 345                 350

cat cca aac tgc acc tac gga tgc act ggg cca ggt ctt gaa ggc tgt 1104
His Pro Asn Cys Thr Tyr Gly Cys Thr Gly Pro Gly Leu Glu Gly Cys
        355                 360                 365

cca acg aat ggg cct aag atc ccg tcc atc gcc act ggg atg gtg ggg 1152
Pro Thr Asn Gly Pro Lys Ile Pro Ser Ile Ala Thr Gly Met Val Gly
    370                 375                 380

gcc ctc ctc ttg ctg ctg gtg gtg gcc ctg ggg atc ggc ctc ttc atg 1200
Ala Leu Leu Leu Leu Leu Val Val Ala Leu Gly Ile Gly Leu Phe Met
385                 390                 395                 400

cga agg cgc cac atc gtt cgg aag cgc acg ctg cgg agg ctg ctg cag 1248
Arg Arg Arg His Ile Val Arg Lys Arg Thr Leu Arg Arg Leu Leu Gln
                405                 410                 415

gag agg gag ctt gtg gag cct ctt aca ccc agt gga gaa gct ccc aac 1296
Glu Arg Glu Leu Val Glu Pro Leu Thr Pro Ser Gly Glu Ala Pro Asn
            420                 425                 430

caa gct ctc ttg agg atc ttg aag gaa act gaa ttc aaa aag atc aaa 1344
Gln Ala Leu Leu Arg Ile Leu Lys Glu Thr Glu Phe Lys Lys Ile Lys
        435                 440                 445

gtg ctg ggc tcc ggt gcg ttc ggc acg gtg tat aag gga ctc tgg atc 1392
Val Leu Gly Ser Gly Ala Phe Gly Thr Val Tyr Lys Gly Leu Trp Ile
    450                 455                 460

cca gaa ggt gag aaa gtt aaa att ccc gtc gct atc aag gaa tta aga 1440
Pro Glu Gly Glu Lys Val Lys Ile Pro Val Ala Ile Lys Glu Leu Arg
465                 470                 475                 480

gaa gca aca tct ccg aaa gcc aac aag gaa atc ctc gat gaa gcc tac 1488
Glu Ala Thr Ser Pro Lys Ala Asn Lys Glu Ile Leu Asp Glu Ala Tyr
                485                 490                 495

gtg atg gcc agc gtg gac aac ccc cac gtg tgc cgc ctg ctg ggc atc 1536
Val Met Ala Ser Val Asp Asn Pro His Val Cys Arg Leu Leu Gly Ile
            500                 505                 510

tgc ctc acc tcc acc gtg cag ctc atc acg cag ctc atg ccc ttc ggc 1584
Cys Leu Thr Ser Thr Val Gln Leu Ile Thr Gln Leu Met Pro Phe Gly
        515                 520                 525

tgc ctc ctg gac tat gtc cgg gaa cac aaa gac aat att ggc tcc cag 1632
Cys Leu Leu Asp Tyr Val Arg Glu His Lys Asp Asn Ile Gly Ser Gln
    530                 535                 540

tac ctg ctc aac tgg tgt gtg cag atc gca aag ggc atg aac tac ttg 1680
Tyr Leu Leu Asn Trp Cys Val Gln Ile Ala Lys Gly Met Asn Tyr Leu
545                 550                 555                 560

gag gac cgt cgc ttg gtg cac cgc gac ctg gca gcc agg aac gta ctg 1728
Glu Asp Arg Arg Leu Val His Arg Asp Leu Ala Ala Arg Asn Val Leu
                565                 570                 575

gtg aaa aca ccg cag cat gtc aag atc aca gat ttt ggg ctg gcc aaa 1776
Val Lys Thr Pro Gln His Val Lys Ile Thr Asp Phe Gly Leu Ala Lys
            580                 585                 590

ctg ctg ggt gcg gaa gag aaa gaa tac cat gca gaa gga ggc aaa gtg 1824
Leu Leu Gly Ala Glu Glu Lys Glu Tyr His Ala Glu Gly Gly Lys Val

        595                 600                 605

cct atc aag tgg atg gca ttg gaa tca att tta cac aga atc tat acc 1872
Pro Ile Lys Trp Met Ala Leu Glu Ser Ile Leu His Arg Ile Tyr Thr
    610                 615                 620

cac cag agt gat gtc tgg agc tac ggg gtg acc gtt tgg gag ttg atg 1920
His Gln Ser Asp Val Trp Ser Tyr Gly Val Thr Val Trp Glu Leu Met
625                 630                 635                 640

acc ttt gga tcc aag cca tat gac gga atc cct gcc agc gag atc tcc 1968
Thr Phe Gly Ser Lys Pro Tyr Asp Gly Ile Pro Ala Ser Glu Ile Ser
                645                 650                 655

tcc atc ctg gag aaa gga gaa cgc ctc cct cag cca ccc ata tgt acc 2016
Ser Ile Leu Glu Lys Gly Glu Arg Leu Pro Gln Pro Pro Ile Cys Thr
            660                 665                 670

atc gat gtc tac atg atc atg gtc aag tgc tgg atg ata gac gca gat 2064
Ile Asp Val Tyr Met Ile Met Val Lys Cys Trp Met Ile Asp Ala Asp
        675                 680                 685

agt cgc cca aag ttc cgt gag ttg atc atc gaa ttc tcc aaa atg gcc 2112
Ser Arg Pro Lys Phe Arg Glu Leu Ile Ile Glu Phe Ser Lys Met Ala
    690                 695                 700

cga gac ccc cag cgc tac ctt gtc att cag ggg gat gaa aga atg cat 2160
Arg Asp Pro Gln Arg Tyr Leu Val Ile Gln Gly Asp Glu Arg Met His
705                 710                 715                 720

ttg cca agt cct aca gac tcc aac ttc tac cgt gcc ctg atg gat gaa 2208
Leu Pro Ser Pro Thr Asp Ser Asn Phe Tyr Arg Ala Leu Met Asp Glu
                725                 730                 735

gaa gac atg gac gac gtg gtg gat gcc gac gag tac ctc atc cca cag 2256
Glu Asp Met Asp Asp Val Val Asp Ala Asp Glu Tyr Leu Ile Pro Gln
            740                 745                 750

cag ggc ttc ttc agc agc ccc tcc acg tca cgg act ccc ctc ctg agc 2304
Gln Gly Phe Phe Ser Ser Pro Ser Thr Ser Arg Thr Pro Leu Leu Ser
        755                 760                 765

tct ctg agt gca acc agc aac aat tcc acc gtg gct tgc att gat aga 2352
Ser Leu Ser Ala Thr Ser Asn Asn Ser Thr Val Ala Cys Ile Asp Arg
    770                 775                 780

aat ggg ctg caa agc tgt ccc atc aag gaa gac agc ttc ttg cag cga 2400
Asn Gly Leu Gln Ser Cys Pro Ile Lys Glu Asp Ser Phe Leu Gln Arg
785                 790                 795                 800

tac agc tca gac ccc aca ggc gcc ttg act gag gac agc ata gac gac 2448
Tyr Ser Ser Asp Pro Thr Gly Ala Leu Thr Glu Asp Ser Ile Asp Asp
                805                 810                 815

acc ttc ctc cca gtg cct gaa tac ata aac cag tcc gtt ccc aaa agg 2496
Thr Phe Leu Pro Val Pro Glu Tyr Ile Asn Gln Ser Val Pro Lys Arg
            820                 825                 830

ccc gct ggc tct gtg cag aat cct gtc tat cac aat cag cct ctg aac 2544
Pro Ala Gly Ser Val Gln Asn Pro Val Tyr His Asn Gln Pro Leu Asn
        835                 840                 845

ccc gcg ccc agc aga gac cca cac tac cag gac ccc cac agc act gca 2592
Pro Ala Pro Ser Arg Asp Pro His Tyr Gln Asp Pro His Ser Thr Ala
    850                 855                 860

gtg ggc aac ccc gag tat ctc aac act gtc cag ccc acc tgt gtc aac 2640
Val Gly Asn Pro Glu Tyr Leu Asn Thr Val Gln Pro Thr Cys Val Asn
865                 870                 875                 880

agc aca ttc gac agc cct gcc cac tgg gcc cag aaa ggc agc cac caa     2688
Ser Thr Phe Asp Ser Pro Ala His Trp Ala Gln Lys Gly Ser His Gln
                885                 890                 895

att agc ctg gac aac cct gac tac cag cag gac ttc ttt ccc aag gaa     2736
Ile Ser Leu Asp Asn Pro Asp Tyr Gln Gln Asp Phe Phe Pro Lys Glu
            900                 905                 910

gcc aag cca aat ggc atc ttt aag ggc tcc aca gct gaa aat gca gaa     2784
Ala Lys Pro Asn Gly Ile Phe Lys Gly Ser Thr Ala Glu Asn Ala Glu
        915                 920                 925

tac cta agg gtc gcg cca caa agc agt gaa ttt att gga gca tga         2829
Tyr Leu Arg Val Ala Pro Gln Ser Ser Glu Phe Ile Gly Ala
    930                 935                 940

<210>  12

<211>  942

<212>  PRT

<213>  ÈË(homo sapiens)

<400>  12

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Arg Asn Tyr Val
            20                  25                  30

Val Thr Asp His Gly Ser Cys Val Arg Ala Cys Gly Ala Asp Ser Tyr
        35                  40                  45

Glu Met Glu Glu Asp Gly Val Arg Lys Cys Lys Lys Cys Glu Gly Pro
    50                  55                  60

Cys Arg Lys Val Cys Asn Gly Ile Gly Ile Gly Glu Phe Lys Asp Ser
65                  70                  75                  80

Leu Ser Ile Asn Ala Thr Asn Ile Lys His Phe Lys Asn Cys Thr Ser
                85                  90                  95

Ile Ser Gly Asp Leu His Ile Leu Pro Val Ala Phe Arg Gly Asp Ser
            100                 105                 110

Phe Thr His Thr Pro Pro Leu Asp Pro Gln Glu Leu Asp Ile Leu Lys
        115                 120                 125

Thr Val Lys Glu Ile Thr Gly Phe Leu Leu Ile Gln Ala Trp Pro Glu
    130                 135                 140

Asn Arg Thr Asp Leu His Ala Phe Glu Asn Leu Glu Ile Ile Arg Gly
145                 150                 155                 160

Arg Thr Lys Gln His Gly Gln Phe Ser Leu Ala Val Val Ser Leu Asn
                165                 170                 175

Ile Thr Ser Leu Gly Leu Arg Ser Leu Lys Glu Ile Ser Asp Gly Asp
            180                 185                 190

Val Ile Ile Ser Gly Asn Lys Asn Leu Cys Tyr Ala Asn Thr Ile Asn
        195                 200                 205

Trp Lys Lys Leu Phe Gly Thr Ser Gly Gln Lys Thr Lys Ile Ile Ser
    210                 215                 220

Asn Arg Gly Glu Asn Ser Cys Lys Ala Thr Gly Gln Val Cys His Ala
225                 230                 235                 240

Leu Cys Ser Pro Glu Gly Cys Trp Gly Pro Glu Pro Arg Asp Cys Val
                245                 250                 255

Ser Cys Arg Asn Val Ser Arg Gly Arg Glu Cys Val Asp Lys Cys Asn
            260                 265                 270

Leu Leu Glu Gly Glu Pro Arg Glu Phe Val Glu Asn Ser Glu Cys Ile
        275                 280                 285

Gln Cys His Pro Glu Cys Leu Pro Gln Ala Met Asn Ile Thr Cys Thr
    290                 295                 300

Gly Arg Gly Pro Asp Asn Cys Ile Gln Cys Ala His Tyr Ile Asp Gly
305                 310                 315                 320

Pro His Cys Val Lys Thr Cys Pro Ala Gly Val Met Gly Glu Asn Asn
                325                 330                 335

Thr Leu Val Trp Lys Tyr Ala Asp Ala Gly His Val Cys His Leu Cys
            340                 345                 350

His Pro Asn Cys Thr Tyr Gly Cys Thr Gly Pro Gly Leu Glu Gly Cys
        355                 360                 365

Pro Thr Asn Gly Pro Lys Ile Pro Ser Ile Ala Thr Gly Met Val Gly
    370                 375                 380

Ala Leu Leu Leu Leu Leu Val Val Ala Leu Gly Ile Gly Leu Phe Met
385                 390                 395                 400

Arg Arg Arg His Ile Val Arg Lys Arg Thr Leu Arg Arg Leu Leu Gln
                405                 410                 415

Glu Arg Glu Leu Val Glu Pro Leu Thr Pro Ser Gly Glu Ala Pro Asn
            420                 425                 430

Gln Ala Leu Leu Arg Ile Leu Lys Glu Thr Glu Phe Lys Lys Ile Lys
        435                 440                 445

Val Leu Gly Ser Gly Ala Phe Gly Thr Val Tyr Lys Gly Leu Trp Ile
    450                 455                 460

Pro Glu Gly Glu Lys Val Lys Ile Pro Val Ala Ile Lys Glu Leu Arg
465                 470                 475                 480

Glu Ala Thr Ser Pro Lys Ala Asn Lys Glu Ile Leu Asp Glu Ala Tyr
                485                 490                 495

Val Met Ala Ser Val Asp Asn Pro His Val Cys Arg Leu Leu Gly Ile
            500                 505                 510

Cys Leu Thr Ser Thr Val Gln Leu Ile Thr Gln Leu Met Pro Phe Gly
        515                 520                 525

Cys Leu Leu Asp Tyr Val Arg Glu His Lys Asp Asn Ile Gly Ser Gln
    530                 535                 540

Tyr Leu Leu Asn Trp Cys Val Gln Ile Ala Lys Gly Met Asn Tyr Leu
545                 550                 555                 560

Glu Asp Arg Arg Leu Val His Arg Asp Leu Ala Ala Arg Asn Val Leu
                565                 570                 575

Val Lys Thr Pro Gln His Val Lys Ile Thr Asp Phe Gly Leu Ala Lys
            580                 585                 590

Leu Leu Gly Ala Glu Glu Lys Glu Tyr His Ala Glu Gly Gly Lys Val
        595                 600                 605

Pro Ile Lys Trp Met Ala Leu Glu Ser Ile Leu His Arg Ile Tyr Thr
    610                 615                 620

His Gln Ser Asp Val Trp Ser Tyr Gly Val Thr Val Trp Glu Leu Met
625                 630                 635                 640

Thr Phe Gly Ser Lys Pro Tyr Asp Gly Ile Pro Ala Ser Glu Ile Ser
                645                 650                 655

Ser Ile Leu Glu Lys Gly Glu Arg Leu Pro Gln Pro Pro Ile Cys Thr
            660                 665                 670

Ile Asp Val Tyr Met Ile Met Val Lys Cys Trp Met Ile Asp Ala Asp
        675                 680                 685

Ser Arg Pro Lys Phe Arg Glu Leu Ile Ile Glu Phe Ser Lys Met Ala
    690                 695                 700

Arg Asp Pro Gln Arg Tyr Leu Val Ile Gln Gly Asp Glu Arg Met His

705                 710                 715                 720

Leu Pro Ser Pro Thr Asp Ser Asn Phe Tyr Arg Ala Leu Met Asp Glu
                725                 730                 735

Glu Asp Met Asp Asp Val Val Asp Ala Asp Glu Tyr Leu Ile Pro Gln
            740                 745                 750

Gln Gly Phe Phe Ser Ser Pro Ser Thr Ser Arg Thr Pro Leu Leu Ser
        755                 760                 765

Ser Leu Ser Ala Thr Ser Asn Asn Ser Thr Val Ala Cys Ile Asp Arg
    770                 775                 780

Asn Gly Leu Gln Ser Cys Pro Ile Lys Glu Asp Ser Phe Leu Gln Arg
785                 790                 795                 800

Tyr Ser Ser Asp Pro Thr Gly Ala Leu Thr Glu Asp Ser Ile Asp Asp
                805                 810                 815

Thr Phe Leu Pro Val Pro Glu Tyr Ile Asn Gln Ser Val Pro Lys Arg
            820                 825                 830

Pro Ala Gly Ser Val Gln Asn Pro Val Tyr His Asn Gln Pro Leu Asn
        835                 840                 845

Pro Ala Pro Ser Arg Asp Pro His Tyr Gln Asp Pro His Ser Thr Ala
    850                 855                 860

Val Gly Asn Pro Glu Tyr Leu Asn Thr Val Gln Pro Thr Cys Val Asn
865                 870                 875                 880

Ser Thr Phe Asp Ser Pro Ala His Trp Ala Gln Lys Gly Ser His Gln
                885                 890                 895

Ile Ser Leu Asp Asn Pro Asp Tyr Gln Gln Asp Phe Phe Pro Lys Glu
            900                 905                 910

Ala Lys Pro Asn Gly Ile Phe Lys Gly Ser Thr Ala Glu Asn Ala Glu
        915                 920                 925

Tyr Leu Arg Val Ala Pro Gln Ser Ser Glu Phe Ile Gly Ala
    930                 935                 940

<210>  13

<211>  3633

<212>  DNA

<213>  Ð¡Êó(Mus musculus)

<220>

<221>  CDS

<222>  (1)..(3630)

<223>

<400>  13
atg cga ccc tca ggg acc gcg aga acc aca ctg ctg gtg ctg ctg acc      48
Met Arg Pro Ser Gly Thr Ala Arg Thr Thr Leu Leu Val Leu Leu Thr
1               5                   10                  15

gcg ctc tgc gcc gca ggt ggg gcg ttg gag gaa aag aaa gtc tgc caa      96
Ala Leu Cys Ala Ala Gly Gly Ala Leu Glu Glu Lys Lys Val Cys Gln
            20                  25                  30

ggc aca agt aac agg ctc acc caa ctg ggc act ttt gaa gac cac ttt      144
Gly Thr Ser Asn Arg Leu Thr Gln Leu Gly Thr Phe Glu Asp His Phe
        35                  40                  45

ctg agc ctg cag agg atg tac aac aac tgt gaa gtg gtc ctt ggg aac      192
Leu Ser Leu Gln Arg Met Tyr Asn Asn Cys Glu Val Val Leu Gly Asn
    50                  55                  60

ttg gaa att acc tat gtg caa agg aat tac gac ctt tcc ttc tta aag      240
Leu Glu Ile Thr Tyr Val Gln Arg Asn Tyr Asp Leu Ser Phe Leu Lys
65                  70                  75                  80

acc atc cag gag gtg gcc ggc tat gtc ctc att gcc ctc aac acc gtg      288
Thr Ile Gln Glu Val Ala Gly Tyr Val Leu Ile Ala Leu Asn Thr Val
                85                  90                  95

gag aga atc cct ttg gag aac ctg cag atc atc agg gga aat gct ctt      336
Glu Arg Ile Pro Leu Glu Asn Leu Gln Ile Ile Arg Gly Asn Ala Leu
            100                 105                 110

tat gaa aac acc tat gcc tta gcc atc ctg tcc aac tat ggg aca aac      384
Tyr Glu Asn Thr Tyr Ala Leu Ala Ile Leu Ser Asn Tyr Gly Thr Asn
        115                 120                 125

aga act ggg ctt agg gaa ctg ccc atg cgg aac tta cag gaa atc ctg      432
Arg Thr Gly Leu Arg Glu Leu Pro Met Arg Asn Leu Gln Glu Ile Leu
    130                 135                 140

att ggt gct gtg cga ttc agc aac aac ccc atc ctc tgc aat atg gat      480
Ile Gly Ala Val Arg Phe Ser Asn Asn Pro Ile Leu Cys Asn Met Asp
145                 150                 155                 160

act atc cag tgg agg gac atc gtc caa aac gtc ttt atg agc aac atg      528
Thr Ile Gln Trp Arg Asp Ile Val Gln Asn Val Phe Met Ser Asn Met
                165                 170                 175

tca atg gac tta cag agc cat ccg agc agt tgc ccc aaa tgt gat cca      576
Ser Met Asp Leu Gln Ser His Pro Ser Ser Cys Pro Lys Cys Asp Pro
            180                 185                 190

agc tgt ccc aat gga agc tgc tgg gga gga gga gag gag aac tgc cag      624
Ser Cys Pro Asn Gly Ser Cys Trp Gly Gly Gly Glu Glu Asn Cys Gln
        195                 200                 205

aaa ttg acc aaa atc atc tgt gcc cag caa tgt tcc cat cgc tgt cgt      672
Lys Leu Thr Lys Ile Ile Cys Ala Gln Gln Cys Ser His Arg Cys Arg
    210                 215                 220

ggc agg tcc ccc agt gac tgc tgc cac aac caa tgt gct gcg ggg tgt      720
Gly Arg Ser Pro Ser Asp Cys Cys His Asn Gln Cys Ala Ala Gly Cys

225                 230                 235                 240

aca ggg ccc cga gag agt gac tgt ctg gtc tgc caa aag ttc caa gat      768
Thr Gly Pro Arg Glu Ser Asp Cys Leu Val Cys Gln Lys Phe Gln Asp
                245                 250                 255

gag gcc aca tgc aaa gac acc tgc cca cca ctc atg ctg tac aac ccc      816
Glu Ala Thr Cys Lys Asp Thr Cys Pro Pro Leu Met Leu Tyr Asn Pro
            260                 265                 270

acc acc tat cag atg gat gtc aac cct gaa ggg aag tac agc ttt ggt      864
Thr Thr Tyr Gln Met Asp Val Asn Pro Glu Gly Lys Tyr Ser Phe Gly
        275                 280                 285

gcc acc tgt gtg aag aag tgc ccc cga aac tac gtg gtg aca gat cat      912
Ala Thr Cys Val Lys Lys Cys Pro Arg Asn Tyr Val Val Thr Asp His
    290                 295                 300

ggc tca tgt gtc cga gcc tgt ggg cct gac tac tac gaa gtg gaa gaa      960
Gly Ser Cys Val Arg Ala Cys Gly Pro Asp Tyr Tyr Glu Val Glu Glu
305                 310                 315                 320

gat ggc atc cgc aag tgt aaa aaa tgt gat ggg ccc tgt cgc aaa gtt     1008
Asp Gly Ile Arg Lys Cys Lys Lys Cys Asp Gly Pro Cys Arg Lys Val
                325                 330                 335

tgt aat ggc ata ggc att ggt gaa ttt aaa gac aca ctc tcc ata aat     1056
Cys Asn Gly Ile Gly Ile Gly Glu Phe Lys Asp Thr Leu Ser Ile Asn
            340                 345                 350

gct aca aac atc aaa cac ttc aaa tac tgc act gcc atc agc ggg gac     1104
Ala Thr Asn Ile Lys His Phe Lys Tyr Cys Thr Ala Ile Ser Gly Asp
        355                 360                 365

ctt cac atc ctg cca gtg gcc ttt aag ggg gat tct ttc acg cgc act     1152
Leu His Ile Leu Pro Val Ala Phe Lys Gly Asp Ser Phe Thr Arg Thr
    370                 375                 380

cct cct cta gac cca cga gaa cta gaa att cta aaa acc gta aag gaa     1200
Pro Pro Leu Asp Pro Arg Glu Leu Glu Ile Leu Lys Thr Val Lys Glu
385                 390                 395                 400

ata aca ggc ttt ttg ctg att cag gct tgg cct gat aac tgg act gac     1248
Ile Thr Gly Phe Leu Leu Ile Gln Ala Trp Pro Asp Asn Trp Thr Asp
                405                 410                 415

ctc cat gct ttc gag aac cta gaa ata ata cgt ggc aga aca aag caa     1296
Leu His Ala Phe Glu Asn Leu Glu Ile Ile Arg Gly Arg Thr Lys Gln
            420                 425                 430

cat ggt cag ttt tct ttg gcg gtc gtt ggc ctg aac atc aca tca ctg     1344
His Gly Gln Phe Ser Leu Ala Val Val Gly Leu Asn Ile Thr Ser Leu
        435                 440                 445

ggg ctg cgt tcc ctc aag gag atc agt gat ggg gat gtg atc att tct     1392
Gly Leu Arg Ser Leu Lys Glu Ile Ser Asp Gly Asp Val Ile Ile Ser
    450                 455                 460

gga aac cga aat ttg tgc tac gca aac aca ata aac tgg aaa aaa ctc     1440
Gly Asn Arg Asn Leu Cys Tyr Ala Asn Thr Ile Asn Trp Lys Lys Leu
465                 470                 475                 480

ttc ggg aca ccc aat cag aaa acc aaa atc atg aac aac aga gct gag     1488
Phe Gly Thr Pro Asn Gln Lys Thr Lys Ile Met Asn Asn Arg Ala Glu
                485                 490                 495

aaa gac tgc aag gcc gtg aac cac gtc tgc aat cct tta tgc tcc tcg     1536
Lys Asp Cys Lys Ala Val Asn His Val Cys Asn Pro Leu Cys Ser Ser
            500                 505                 510

gaa ggc tgc tgg ggc cct gag ccc agg gac tgt gtc tcc tgc cag aat 1584
Glu Gly Cys Trp Gly Pro Glu Pro Arg Asp Cys Val Ser Cys Gln Asn
        515                 520                 525

gtg agc aga ggc agg gag tgc gtg gag aaa tgc aac atc ctg gag ggg 1632
Val Ser Arg Gly Arg Glu Cys Val Glu Lys Cys Asn Ile Leu Glu Gly
    530                 535                 540

gaa cca agg gag ttt gtg gaa aat tct gaa tgc atc cag tgc cat cca 1680
Glu Pro Arg Glu Phe Val Glu Asn Ser Glu Cys Ile Gln Cys His Pro
545                 550                 555                 560

gaa tgt ctg ccc cag gcc atg aac atc acc tgt aca ggc agg ggg cca 1728
Glu Cys Leu Pro Gln Ala Met Asn Ile Thr Cys Thr Gly Arg Gly Pro
                565                 570                 575

gac aac tgc atc cag tgt gcc cac tac att gat ggc cca cac tgt gtc 1776
Asp Asn Cys Ile Gln Cys Ala His Tyr Ile Asp Gly Pro His Cys Val
            580                 585                 590

aag acc tgc cca gct ggc atc atg gga gag aac aac act ctg gtc tgg 1824
Lys Thr Cys Pro Ala Gly Ile Met Gly Glu Asn Asn Thr Leu Val Trp
        595                 600                 605

aag tat gca gat gcc aat aat gtc tgc cac cta tgc cac gcc aac tgt 1872
Lys Tyr Ala Asp Ala Asn Asn Val Cys His Leu Cys His Ala Asn Cys
    610                 615                 620

acc tat gga tgt gct ggg cca ggt ctt caa gga tgt gaa gtg tgg cca 1920
Thr Tyr Gly Cys Ala Gly Pro Gly Leu Gln Gly Cys Glu Val Trp Pro
625                 630                 635                 640

tct ggg cca aag ata cca tct att gcc act ggg att gtg ggt ggc ctc 1968
Ser Gly Pro Lys Ile Pro Ser Ile Ala Thr Gly Ile Val Gly Gly Leu
                645                 650                 655

ctc ttc ata gtg gtg gtg gcc ctt ggg att ggc cta ttc atg cga aga 2016
Leu Phe Ile Val Val Val Ala Leu Gly Ile Gly Leu Phe Met Arg Arg
            660                 665                 670

cgt cac att gtt cga aag cgt aca cta cgc cgc ctg ctt caa gag aga 2064
Arg His Ile Val Arg Lys Arg Thr Leu Arg Arg Leu Leu Gln Glu Arg
        675                 680                 685

gag ctc gtg gaa cct ctc aca ccc agc gga gaa gct cca aac caa gcc 2112
Glu Leu Val Glu Pro Leu Thr Pro Ser Gly Glu Ala Pro Asn Gln Ala
    690                 695                 700

cac ttg agg ata tta aag gaa aca gaa ttc aaa aag atc aaa gtt ctg 2160
His Leu Arg Ile Leu Lys Glu Thr Glu Phe Lys Lys Ile Lys Val Leu
705                 710                 715                 720

ggt tcg gga gca ttt ggc aca gtg tat aag ggt ctc tgg atc cca gaa 2208
Gly Ser Gly Ala Phe Gly Thr Val Tyr Lys Gly Leu Trp Ile Pro Glu
                725                 730                 735

ggt gag aaa gta aaa atc ccg gtg gcc atc aag gag tta aga gaa gcc 2256
Gly Glu Lys Val Lys Ile Pro Val Ala Ile Lys Glu Leu Arg Glu Ala
            740                 745                 750

aca tct cca aaa gcc aac aaa gaa atc ctt gac gaa gcc tat gtg atg 2304
Thr Ser Pro Lys Ala Asn Lys Glu Ile Leu Asp Glu Ala Tyr Val Met
        755                 760                 765

gct agt gtg gac aac cct cat gta tgc cgc ctc ctg ggc atc tgt ctg 2352
Ala Ser Val Asp Asn Pro His Val Cys Arg Leu Leu Gly Ile Cys Leu
    770                 775                 780

acc tcc act gtc cag ctc att aca cag ctc atg ccc tac ggt tgc ctc 2400
Thr Ser Thr Val Gln Leu Ile Thr Gln Leu Met Pro Tyr Gly Cys Leu
785                 790                 795                 800

ctg gac tac gtc cga gaa cac aag gac aac att ggc tcc cag tac ctc 2448
Leu Asp Tyr Val Arg Glu His Lys Asp Asn Ile Gly Ser Gln Tyr Leu
                805                 810                 815

ctc aac tgg tgt gtg cag att gca aag ggc atg aac tac ctg gaa gat 2496
Leu Asn Trp Cys Val Gln Ile Ala Lys Gly Met Asn Tyr Leu Glu Asp
            820                 825                 830

cgg cgt ttg gtg cac cgt gac ttg gca gcc agg aat gta ctg gtg aag 2544
Arg Arg Leu Val His Arg Asp Leu Ala Ala Arg Asn Val Leu Val Lys
        835                 840                 845

aca cca cag cat gtc aag atc aca gat ttt ggg ctg gcc aaa ctg ctt 2592
Thr Pro Gln His Val Lys Ile Thr Asp Phe Gly Leu Ala Lys Leu Leu
    850                 855                 860

ggt gct gaa gag aaa gaa tat cat gcc gag ggg ggc aaa gtg cct atc 2640
Gly Ala Glu Glu Lys Glu Tyr His Ala Glu Gly Gly Lys Val Pro Ile
865                 870                 875                 880

aag tgg atg gct ttg gaa tca att tta cac cga att tat aca cac caa 2688
Lys Trp Met Ala Leu Glu Ser Ile Leu His Arg Ile Tyr Thr His Gln
                885                 890                 895

agt gat gtc tgg agc tat ggt gtc act gtg tgg gaa ctg atg acc ttt 2736
Ser Asp Val Trp Ser Tyr Gly Val Thr Val Trp Glu Leu Met Thr Phe
            900                 905                 910

ggg tcc aag cct tat gat gga atc cca gca agt gac atc tca tcc atc 2784
Gly Ser Lys Pro Tyr Asp Gly Ile Pro Ala Ser Asp Ile Ser Ser Ile
        915                 920                 925

cta gag aaa gga gaa cgc ctt cca cag cca cct atc tgc acc atc gat 2832
Leu Glu Lys Gly Glu Arg Leu Pro Gln Pro Pro Ile Cys Thr Ile Asp
    930                 935                 940

gtc tac atg atc atg gtc aag tgc tgg atg ata gat gct gat agc cgc 2880
Val Tyr Met Ile Met Val Lys Cys Trp Met Ile Asp Ala Asp Ser Arg
945                 950                 955                 960

cca aag ttc cga gag ttg att ctt gaa ttc tcc aaa atg gcc cga gac 2928
Pro Lys Phe Arg Glu Leu Ile Leu Glu Phe Ser Lys Met Ala Arg Asp
                965                 970                 975

cca cag cgc tac ctt gtt atc cag ggg gat gaa aga atg cat ttg cca 2976
Pro Gln Arg Tyr Leu Val Ile Gln Gly Asp Glu Arg Met His Leu Pro
            980                 985                 990

agc cct aca gac tcc aac ttt tac cga gcc ctg atg gat gaa gag gac 3024
Ser Pro Thr Asp Ser Asn Phe Tyr Arg Ala Leu Met Asp Glu Glu Asp
        995                 1000                1005

atg gag gat gta gtt gat gct gat gag tat ctt acc cca cag caa 3069
Met Glu Asp Val Val Asp Ala Asp Glu Tyr Leu Thr Pro Gln Gln
    1010                1015                1020

ggc ttc ttc aac agc ccg tcc acg tcg agg act ccc ctc ttg agt 3114
Gly Phe Phe Asn Ser Pro Ser Thr Ser Arg Thr Pro Leu Leu Ser
    1025                1030                1035

tct ctg agt gca act agc aac aat tcc act gtg gct tgc att aat 3159
Ser Leu Ser Ala Thr Ser Asn Asn Ser Thr Val Ala Cys Ile Asn
    1040                1045                1050

aga aat ggg agc tgc cgt gtc aaa gaa gac gcc ttc ttg cag cgg 3204

Arg Asn Gly Ser Cys Arg Val Lys Glu Asp Ala Phe Leu Gln Arg
    1055                1060                1065

tac agc tcc gac ccc aca ggt gct gta aca gag gac aac ata gat 3249
Tyr Ser Ser Asp Pro Thr Gly Ala Val Thr Glu Asp Asn Ile Asp
    1070                1075                1080

gac gca ttc ctc cct gta cct gaa tat gta aac caa tct gtt ccc 3294
Asp Ala Phe Leu Pro Val Pro Glu Tyr Val Asn Gln Ser Val Pro
    1085                1090                1095

aag agg cca gca ggc tct gtg cag aac cct gtc tat cac aat cag 3339
Lys Arg Pro Ala Gly Ser Val Gln Asn Pro Val Tyr His Asn Gln
    1100                1105                1110

ccc ctg cat cca gct cct gga aga gac ctg cat tat caa aat ccc 3384
Pro Leu His Pro Ala Pro Gly Arg Asp Leu His Tyr Gln Asn Pro
    1115                1120                1125

cac agc aat gca gtg ggc aac cct gag tat ctc aac act gcc cag 3429
His Ser Asn Ala Val Gly Asn Pro Glu Tyr Leu Asn Thr Ala Gln
    1130                1135                1140

cct acc tgt ctc agt agt ggg ttt aac agc cct gca ctc tgg atc 3474
Pro Thr Cys Leu Ser Ser Gly Phe Asn Ser Pro Ala Leu Trp Ile
    1145                1150                1155

cag aaa ggc agt cac caa atg agc cta gac aac cct gac tac cag 3519
Gln Lys Gly Ser His Gln Met Ser Leu Asp Asn Pro Asp Tyr Gln
    1160                1165                1170

cag gac ttc ttc ccc aag gaa acc aag cca aat ggc ata ttt aag 3564
Gln Asp Phe Phe Pro Lys Glu Thr Lys Pro Asn Gly Ile Phe Lys
    1175                1180                1185

ggc ccc aca gct gaa aat gca gag tac cta cgg gtg gca cct cca 3609
Gly Pro Thr Ala Glu Asn Ala Glu Tyr Leu Arg Val Ala Pro Pro
    1190                1195                1200

agc agc gag ttt att gga gca tga 3633
Ser Ser Glu Phe Ile Gly Ala
    1205                1210

<210> 14

<211> 1210

<212> PRT

<213> Ð¡Êó(Mus musculus)

<400> 14

Met Arg Pro Ser Gly Thr Ala Arg Thr Thr Leu Leu Val Leu Leu Thr
1               5                   10                  15

Ala Leu Cys Ala Ala Gly Gly Ala Leu Glu Glu Lys Lys Val Cys Gln
            20                  25                  30

Gly Thr Ser Asn Arg Leu Thr Gln Leu Gly Thr Phe Glu Asp His Phe
        35                  40                  45

Leu Ser Leu Gln Arg Met Tyr Asn Asn Cys Glu Val Val Leu Gly Asn

50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Ala Leu
            100                 105                 110

Tyr Glu Asn Thr Tyr Ala Leu Ala Ile Leu Ser Asn Tyr Gly Thr Asn
        115                 120                 125

Arg Thr Gly Leu Arg Glu Leu Pro Met Arg Asn Leu Gln Glu Ile Leu
    130                 135                 140

Ile Gly Ala Val Arg Phe Ser Asn Asn Pro Ile Leu Cys Asn Met Asp
145                 150                 155                 160

Thr Ile Gln Trp Arg Asp Ile Val Gln Asn Val Phe Met Ser Asn Met
                165                 170                 175

Ser Met Asp Leu Gln Ser His Pro Ser Ser Cys Pro Lys Cys Asp Pro
            180                 185                 190

Ser Cys Pro Asn Gly Ser Cys Trp Gly Gly Gly Glu Glu Asn Cys Gln
        195                 200                 205

Lys Leu Thr Lys Ile Ile Cys Ala Gln Gln Cys Ser His Arg Cys Arg
    210                 215                 220

Gly Arg Ser Pro Ser Asp Cys Cys His Asn Gln Cys Ala Ala Gly Cys
225                 230                 235                 240

Thr Gly Pro Arg Glu Ser Asp Cys Leu Val Cys Gln Lys Phe Gln Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Pro Asp Tyr Tyr Glu Val Glu Glu
305                 310                 315                 320

Asp Gly Ile Arg Lys Cys Lys Lys Cys Asp Gly Pro Cys Arg Lys Val
                325                 330                 335

Cys Asn Gly Ile Gly Ile Gly Glu Phe Lys Asp Thr Leu Ser Ile Asn
            340                 345                 350

Ala Thr Asn Ile Lys His Phe Lys Tyr Cys Thr Ala Ile Ser Gly Asp
        355                 360                 365

Leu His Ile Leu Pro Val Ala Phe Lys Gly Asp Ser Phe Thr Arg Thr
    370                 375                 380

Pro Pro Leu Asp Pro Arg Glu Leu Glu Ile Leu Lys Thr Val Lys Glu
385                 390                 395                 400

Ile Thr Gly Phe Leu Leu Ile Gln Ala Trp Pro Asp Asn Trp Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Gly Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Arg Asn Leu Cys Tyr Ala Asn Thr Ile Asn Trp Lys Lys Leu
465                 470                 475                 480

Phe Gly Thr Pro Asn Gln Lys Thr Lys Ile Met Asn Asn Arg Ala Glu
                485                 490                 495

Lys Asp Cys Lys Ala Val Asn His Val Cys Asn Pro Leu Cys Ser Ser
            500                 505                 510

Glu Gly Cys Trp Gly Pro Glu Pro Arg Asp Cys Val Ser Cys Gln Asn
        515                 520                 525

Val Ser Arg Gly Arg Glu Cys Val Glu Lys Cys Asn Ile Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Ile Met Gly Glu Asn Asn Thr Leu Val Trp
        595                 600                 605

Lys Tyr Ala Asp Ala Asn Asn Val Cys His Leu Cys His Ala Asn Cys
    610                 615                 620

Thr Tyr Gly Cys Ala Gly Pro Gly Leu Gln Gly Cys Glu Val Trp Pro
625                 630                 635                 640

Ser Gly Pro Lys Ile Pro Ser Ile Ala Thr Gly Ile Val Gly Gly Leu
                645                 650                 655

Leu Phe Ile Val Val Val Ala Leu Gly Ile Gly Leu Phe Met Arg Arg
            660                 665                 670

Arg His Ile Val Arg Lys Arg Thr Leu Arg Arg Leu Leu Gln Glu Arg
        675                 680                 685

Glu Leu Val Glu Pro Leu Thr Pro Ser Gly Glu Ala Pro Asn Gln Ala
    690                 695                 700

His Leu Arg Ile Leu Lys Glu Thr Glu Phe Lys Lys Ile Lys Val Leu
705                 710                 715                 720

Gly Ser Gly Ala Phe Gly Thr Val Tyr Lys Gly Leu Trp Ile Pro Glu
                725                 730                 735

Gly Glu Lys Val Lys Ile Pro Val Ala Ile Lys Glu Leu Arg Glu Ala
            740                 745                 750

Thr Ser Pro Lys Ala Asn Lys Glu Ile Leu Asp Glu Ala Tyr Val Met
        755                 760                 765

Ala Ser Val Asp Asn Pro His Val Cys Arg Leu Leu Gly Ile Cys Leu
    770                 775                 780

Thr Ser Thr Val Gln Leu Ile Thr Gln Leu Met Pro Tyr Gly Cys Leu
785                 790                 795                 800

Leu Asp Tyr Val Arg Glu His Lys Asp Asn Ile Gly Ser Gln Tyr Leu
                805                 810                 815

Leu Asn Trp Cys Val Gln Ile Ala Lys Gly Met Asn Tyr Leu Glu Asp
            820                 825                 830

Arg Arg Leu Val His Arg Asp Leu Ala Ala Arg Asn Val Leu Val Lys
        835                 840                 845

Thr Pro Gln His Val Lys Ile Thr Asp Phe Gly Leu Ala Lys Leu Leu
    850                 855                 860

Gly Ala Glu Glu Lys Glu Tyr His Ala Glu Gly Gly Lys Val Pro Ile
865                 870                 875                 880

Lys Trp Met Ala Leu Glu Ser Ile Leu His Arg Ile Tyr Thr His Gln
                885                 890                 895

Ser Asp Val Trp Ser Tyr Gly Val Thr Val Trp Glu Leu Met Thr Phe
            900                 905                 910

Gly Ser Lys Pro Tyr Asp Gly Ile Pro Ala Ser Asp Ile Ser Ser Ile
        915                 920                 925

Leu Glu Lys Gly Glu Arg Leu Pro Gln Pro Pro Ile Cys Thr Ile Asp
    930                 935                 940

Val Tyr Met Ile Met Val Lys Cys Trp Met Ile Asp Ala Asp Ser Arg
945                 950                 955                 960

Pro Lys Phe Arg Glu Leu Ile Leu Glu Phe Ser Lys Met Ala Arg Asp
                965                 970                 975

Pro Gln Arg Tyr Leu Val Ile Gln Gly Asp Glu Arg Met His Leu Pro
            980                 985                 990

Ser Pro Thr Asp Ser Asn Phe Tyr  Arg Ala Leu Met Asp  Glu Glu Asp
        995                 1000                1005

Met Glu  Asp Val Val Asp Ala  Asp Glu Tyr Leu Thr  Pro Gln Gln
    1010                 1015                 1020

Gly Phe  Phe Asn Ser Pro Ser  Thr Ser Arg Thr Pro  Leu Leu Ser
    1025                 1030                 1035

Ser Leu  Ser Ala Thr Ser Asn  Asn Ser Thr Val Ala  Cys Ile Asn
    1040                 1045                 1050

Arg Asn  Gly Ser Cys Arg Val  Lys Glu Asp Ala Phe  Leu Gln Arg
    1055                 1060                 1065

Tyr Ser  Ser Asp Pro Thr Gly  Ala Val Thr Glu Asp  Asn Ile Asp
    1070                 1075                 1080

Asp Ala  Phe Leu Pro Val Pro  Glu Tyr Val Asn Gln  Ser Val Pro
    1085                 1090                 1095

Lys Arg  Pro Ala Gly Ser Val  Gln Asn Pro Val Tyr  His Asn Gln
    1100                 1105                 1110

Pro Leu  His Pro Ala Pro Gly  Arg Asp Leu His Tyr  Gln Asn Pro
    1115                 1120                 1125

His Ser  Asn Ala Val Gly Asn  Pro Glu Tyr Leu Asn  Thr Ala Gln
    1130                 1135                 1140

Pro Thr  Cys Leu Ser Ser Gly  Phe Asn Ser Pro Ala  Leu Trp Ile

1145                    1150                    1155

Gln Lys  Gly Ser His Gln Met  Ser Leu Asp Asn Pro  Asp Tyr Gln
    1160                    1165                    1170

Gln Asp  Phe Phe Pro Lys Glu  Thr Lys Pro Asn Gly  Ile Phe Lys
    1175                    1180                    1185

Gly Pro  Thr Ala Glu Asn Ala  Glu Tyr Leu Arg Val  Ala Pro Pro
    1190                    1195                    1200

Ser Ser  Glu Phe Ile Gly Ala
    1205                    1210

<210>  15

<211>  1932

<212>  DNA

<213>  Ð¡Êó(Mus musculus)

<220>

<221>  CDS

<222>  (1)..(1929)

<223>

<400>  15
atg cga ccc tca ggg acc gcg aga acc aca ctg ctg gtg ctg ctg acc        48
Met Arg Pro Ser Gly Thr Ala Arg Thr Thr Leu Leu Val Leu Leu Thr
1               5                   10                  15

gcg ctc tgc gcc gca ggt ggg gcg ttg gag gaa aag aaa gtc tgc caa        96
Ala Leu Cys Ala Ala Gly Gly Ala Leu Glu Glu Lys Lys Val Cys Gln
            20                  25                  30

ggc aca agt aac agg ctc acc caa ctg ggc act ttt gaa gac cac ttt       144
Gly Thr Ser Asn Arg Leu Thr Gln Leu Gly Thr Phe Glu Asp His Phe
        35                  40                  45

ctg agc ctg cag agg atg tac aac aac tgt gaa gtg gtc ctt ggg aac       192
Leu Ser Leu Gln Arg Met Tyr Asn Asn Cys Glu Val Val Leu Gly Asn
    50                  55                  60

ttg gaa att acc tat gtg caa agg aat tac gac ctt tcc ttc tta aag       240
Leu Glu Ile Thr Tyr Val Gln Arg Asn Tyr Asp Leu Ser Phe Leu Lys
65                  70                  75                  80

acc atc cag gag gtg gcc ggc tat gtc ctc att gcc ctc aac acc gtg       288
Thr Ile Gln Glu Val Ala Gly Tyr Val Leu Ile Ala Leu Asn Thr Val
                85                  90                  95

gag aga atc cct ttg gag aac ctg cag atc atc agg gga aat gct ctt       336
Glu Arg Ile Pro Leu Glu Asn Leu Gln Ile Ile Arg Gly Asn Ala Leu
            100                 105                 110

tat gaa aac acc tat gcc tta gcc atc ctg tcc aac tat ggg aca aac       384

Tyr Glu Asn Thr Tyr Ala Leu Ala Ile Leu Ser Asn Tyr Gly Thr Asn
        115                 120                 125

aga act ggg ctt agg gaa ctg ccc atg cgg aac tta cag gaa atc ctg 432
Arg Thr Gly Leu Arg Glu Leu Pro Met Arg Asn Leu Gln Glu Ile Leu
    130                 135                 140

att ggt gct gtg cga ttc agc aac aac ccc atc ctc tgc aat atg gat 480
Ile Gly Ala Val Arg Phe Ser Asn Asn Pro Ile Leu Cys Asn Met Asp
145                 150                 155                 160

act atc cag tgg agg gac atc gtc caa aac gtc ttt atg agc aac atg 528
Thr Ile Gln Trp Arg Asp Ile Val Gln Asn Val Phe Met Ser Asn Met
                165                 170                 175

tca atg gac tta cag agc cat ccg agc agt tgc ccc aaa tgt gat cca 576
Ser Met Asp Leu Gln Ser His Pro Ser Ser Cys Pro Lys Cys Asp Pro
            180                 185                 190

agc tgt ccc aat gga agc tgc tgg gga gga gga gag gag aac tgc cag 624
Ser Cys Pro Asn Gly Ser Cys Trp Gly Gly Gly Glu Glu Asn Cys Gln
        195                 200                 205

aaa ttg acc aaa atc atc tgt gcc cag caa tgt tcc cat cgc tgt cgt 672
Lys Leu Thr Lys Ile Ile Cys Ala Gln Gln Cys Ser His Arg Cys Arg
    210                 215                 220

ggc agg tcc ccc agt gac tgc tgc cac aac caa tgt gct gcg ggg tgt 720
Gly Arg Ser Pro Ser Asp Cys Cys His Asn Gln Cys Ala Ala Gly Cys
225                 230                 235                 240

aca ggg ccc cga gag agt gac tgt ctg gtc tgc caa aag ttc caa gat 768
Thr Gly Pro Arg Glu Ser Asp Cys Leu Val Cys Gln Lys Phe Gln Asp
                245                 250                 255

gag gcc aca tgc aaa gac acc tgc cca cca ctc atg ctg tac aac ccc 816
Glu Ala Thr Cys Lys Asp Thr Cys Pro Pro Leu Met Leu Tyr Asn Pro
            260                 265                 270

acc acc tat cag atg gat gtc aac cct gaa ggg aag tac agc ttt ggt 864
Thr Thr Tyr Gln Met Asp Val Asn Pro Glu Gly Lys Tyr Ser Phe Gly
        275                 280                 285

gcc acc tgt gtg aag aag tgc ccc cga aac tac gtg gtg aca gat cat 912
Ala Thr Cys Val Lys Lys Cys Pro Arg Asn Tyr Val Val Thr Asp His
    290                 295                 300

ggc tca tgt gtc cga gcc tgt ggg cct gac tac tac gaa gtg gaa gaa 960
Gly Ser Cys Val Arg Ala Cys Gly Pro Asp Tyr Tyr Glu Val Glu Glu
305                 310                 315                 320

gat ggc atc cgc aag tgt aaa aaa tgt gat ggg ccc tgt cgc aaa gtt 1008
Asp Gly Ile Arg Lys Cys Lys Lys Cys Asp Gly Pro Cys Arg Lys Val
                325                 330                 335

tgt aat ggc ata ggc att ggt gaa ttt aaa gac aca ctc tcc ata aat 1056
Cys Asn Gly Ile Gly Ile Gly Glu Phe Lys Asp Thr Leu Ser Ile Asn
            340                 345                 350

gct aca aac atc aaa cac ttc aaa tac tgc act gcc atc agc ggg gac 1104
Ala Thr Asn Ile Lys His Phe Lys Tyr Cys Thr Ala Ile Ser Gly Asp
        355                 360                 365

ctt cac atc ctg cca gtg gcc ttt aag ggg gat tct ttc acg cgc act 1152
Leu His Ile Leu Pro Val Ala Phe Lys Gly Asp Ser Phe Thr Arg Thr
    370                 375                 380

cct cct cta gac cca cga gaa cta gaa att cta aaa acc gta aag gaa 1200
Pro Pro Leu Asp Pro Arg Glu Leu Glu Ile Leu Lys Thr Val Lys Glu

385                 390                 395                 400

ata aca ggc ttt ttg ctg att cag gct tgg cct gat aac tgg act gac      1248
Ile Thr Gly Phe Leu Leu Ile Gln Ala Trp Pro Asp Asn Trp Thr Asp
            405                 410                 415

ctc cat gct ttc gag aac cta gaa ata ata cgt ggc aga aca aag caa      1296
Leu His Ala Phe Glu Asn Leu Glu Ile Ile Arg Gly Arg Thr Lys Gln
        420                 425                 430

cat ggt cag ttt tct ttg gcg gtc gtt ggc ctg aac atc aca tca ctg      1344
His Gly Gln Phe Ser Leu Ala Val Val Gly Leu Asn Ile Thr Ser Leu
    435                 440                 445

ggg ctg cgt tcc ctc aag gag atc agt gat ggg gat gtg atc att tct      1392
Gly Leu Arg Ser Leu Lys Glu Ile Ser Asp Gly Asp Val Ile Ile Ser
    450                 455                 460

gga aac cga aat ttg tgc tac gca aac aca ata aac tgg aaa aaa ctc      1440
Gly Asn Arg Asn Leu Cys Tyr Ala Asn Thr Ile Asn Trp Lys Lys Leu
465                 470                 475                 480

ttc ggg aca ccc aat cag aaa acc aaa atc atg aac aac aga gct gag      1488
Phe Gly Thr Pro Asn Gln Lys Thr Lys Ile Met Asn Asn Arg Ala Glu
            485                 490                 495

aaa gac tgc aag gcc gtg aac cac gtc tgc aat cct tta tgc tcc tcg      1536
Lys Asp Cys Lys Ala Val Asn His Val Cys Asn Pro Leu Cys Ser Ser
        500                 505                 510

gaa ggc tgc tgg ggc cct gag ccc agg gac tgt gtc tcc tgc cag aat      1584
Glu Gly Cys Trp Gly Pro Glu Pro Arg Asp Cys Val Ser Cys Gln Asn
    515                 520                 525

gtg agc aga ggc agg gag tgc gtg gag aaa tgc aac atc ctg gag ggg      1632
Val Ser Arg Gly Arg Glu Cys Val Glu Lys Cys Asn Ile Leu Glu Gly
    530                 535                 540

gaa cca agg gag ttt gtg gaa aat tct gaa tgc atc cag tgc cat cca      1680
Glu Pro Arg Glu Phe Val Glu Asn Ser Glu Cys Ile Gln Cys His Pro
545                 550                 555                 560

gaa tgt ctg ccc cag gcc atg aac atc acc tgt aca ggc agg ggg cca      1728
Glu Cys Leu Pro Gln Ala Met Asn Ile Thr Cys Thr Gly Arg Gly Pro
            565                 570                 575

gac aac tgc atc cag tgt gcc cac tac att gat ggc cca cac tgt gtc      1776
Asp Asn Cys Ile Gln Cys Ala His Tyr Ile Asp Gly Pro His Cys Val
        580                 585                 590

aag acc tgc cca gct ggc atc atg gga gag aac aac act ctg gtc tgg      1824
Lys Thr Cys Pro Ala Gly Ile Met Gly Glu Asn Asn Thr Leu Val Trp
    595                 600                 605

aag tat gca gat gcc aat aat gtc tgc cac cta tgc cac gcc aac tgt      1872
Lys Tyr Ala Asp Ala Asn Asn Val Cys His Leu Cys His Ala Asn Cys
    610                 615                 620

acc tat gga gta atg gtt cct gaa atg ttg ctc caa agt atc att tta      1920
Thr Tyr Gly Val Met Val Pro Glu Met Leu Leu Gln Ser Ile Ile Leu
625                 630                 635                 640

aaa cct att tga                                                      1932
Lys Pro Ile

<210>  16

<211>  643

<212>  PRT

<213>  Ð¡Êó(Mus musculus)

<400>  16

Met Arg Pro Ser Gly Thr Ala Arg Thr Thr Leu Leu Val Leu Leu Thr
1               5                   10                  15

Ala Leu Cys Ala Ala Gly Gly Ala Leu Glu Glu Lys Lys Val Cys Gln
            20                  25                  30

Gly Thr Ser Asn Arg Leu Thr Gln Leu Gly Thr Phe Glu Asp His Phe
        35                  40                  45

Leu Ser Leu Gln Arg Met Tyr Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Ala Leu
            100                 105                 110

Tyr Glu Asn Thr Tyr Ala Leu Ala Ile Leu Ser Asn Tyr Gly Thr Asn
        115                 120                 125

Arg Thr Gly Leu Arg Glu Leu Pro Met Arg Asn Leu Gln Glu Ile Leu
    130                 135                 140

Ile Gly Ala Val Arg Phe Ser Asn Asn Pro Ile Leu Cys Asn Met Asp
145                 150                 155                 160

Thr Ile Gln Trp Arg Asp Ile Val Gln Asn Val Phe Met Ser Asn Met
                165                 170                 175

Ser Met Asp Leu Gln Ser His Pro Ser Ser Cys Pro Lys Cys Asp Pro
            180                 185                 190

Ser Cys Pro Asn Gly Ser Cys Trp Gly Gly Gly Glu Glu Asn Cys Gln
        195                 200                 205

Lys Leu Thr Lys Ile Ile Cys Ala Gln Gln Cys Ser His Arg Cys Arg
    210                 215                 220

Gly Arg Ser Pro Ser Asp Cys Cys His Asn Gln Cys Ala Ala Gly Cys
225                 230                 235                 240

Thr Gly Pro Arg Glu Ser Asp Cys Leu Val Cys Gln Lys Phe Gln Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Pro Asp Tyr Tyr Glu Val Glu Glu
305                 310                 315                 320

Asp Gly Ile Arg Lys Cys Lys Lys Cys Asp Gly Pro Cys Arg Lys Val
                325                 330                 335

Cys Asn Gly Ile Gly Ile Gly Glu Phe Lys Asp Thr Leu Ser Ile Asn
            340                 345                 350

Ala Thr Asn Ile Lys His Phe Lys Tyr Cys Thr Ala Ile Ser Gly Asp
        355                 360                 365

Leu His Ile Leu Pro Val Ala Phe Lys Gly Asp Ser Phe Thr Arg Thr
    370                 375                 380

Pro Pro Leu Asp Pro Arg Glu Leu Glu Ile Leu Lys Thr Val Lys Glu
385                 390                 395                 400

Ile Thr Gly Phe Leu Leu Ile Gln Ala Trp Pro Asp Asn Trp Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Gly Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Arg Asn Leu Cys Tyr Ala Asn Thr Ile Asn Trp Lys Lys Leu
465                 470                 475                 480

Phe Gly Thr Pro Asn Gln Lys Thr Lys Ile Met Asn Asn Arg Ala Glu
                485                 490                 495

Lys Asp Cys Lys Ala Val Asn His Val Cys Asn Pro Leu Cys Ser Ser
            500                 505                 510

Glu Gly Cys Trp Gly Pro Glu Pro Arg Asp Cys Val Ser Cys Gln Asn
        515                 520                 525

Val Ser Arg Gly Arg Glu Cys Val Glu Lys Cys Asn Ile Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Ile Met Gly Glu Asn Asn Thr Leu Val Trp
        595                 600                 605

Lys Tyr Ala Asp Ala Asn Asn Val Cys His Leu Cys His Ala Asn Cys
    610                 615                 620

Thr Tyr Gly Val Met Val Pro Glu Met Leu Leu Gln Ser Ile Ile Leu
625                 630                 635                 640

Lys Pro Ile

<210>  17

<211>  1968

<212>  DNA

<213>  Ð¡Êó(Mus musculus)

<220>

<221>  CDS

<222>  (1)..(1965)

<223>

<400>  17
atg cga ccc tca ggg acc gcg aga acc aca ctg ctg gtg ctg ctg acc      48
Met Arg Pro Ser Gly Thr Ala Arg Thr Thr Leu Leu Val Leu Leu Thr
1               5                   10                  15

gcg ctc tgc gcc gca ggt ggg gcg ttg gag gaa aag aaa gtc tgc caa      96
Ala Leu Cys Ala Ala Gly Gly Ala Leu Glu Glu Lys Lys Val Cys Gln
            20                  25                  30

ggc aca agt aac agg ctc acc caa ctg ggc act ttt gaa gac cac ttt      144
Gly Thr Ser Asn Arg Leu Thr Gln Leu Gly Thr Phe Glu Asp His Phe
        35                  40                  45

ctg agc ctg cag agg atg tac aac aac tgt gaa gtg gtc ctt ggg aac 192
Leu Ser Leu Gln Arg Met Tyr Asn Asn Cys Glu Val Val Leu Gly Asn
    50                  55                  60

ttg gaa att acc tat gtg caa agg aat tac gac ctt tcc ttc tta aag 240
Leu Glu Ile Thr Tyr Val Gln Arg Asn Tyr Asp Leu Ser Phe Leu Lys
65                  70                  75                  80

acc atc cag gag gtg gcc ggc tat gtc ctc att gcc ctc aac acc gtg 288
Thr Ile Gln Glu Val Ala Gly Tyr Val Leu Ile Ala Leu Asn Thr Val
                85                  90                  95

gag aga atc cct ttg gag aac ctg cag atc atc agg gga aat gct ctt 336
Glu Arg Ile Pro Leu Glu Asn Leu Gln Ile Ile Arg Gly Asn Ala Leu
            100                 105                 110

tat gaa aac acc tat gcc tta gcc atc ctg tcc aac tat ggg aca aac 384
Tyr Glu Asn Thr Tyr Ala Leu Ala Ile Leu Ser Asn Tyr Gly Thr Asn
        115                 120                 125

aga act ggg ctt agg gaa ctg ccc atg cgg aac tta cag gaa atc ctg 432
Arg Thr Gly Leu Arg Glu Leu Pro Met Arg Asn Leu Gln Glu Ile Leu
    130                 135                 140

att ggt gct gtg cga ttc agc aac aac ccc atc ctc tgc aat atg gat 480
Ile Gly Ala Val Arg Phe Ser Asn Asn Pro Ile Leu Cys Asn Met Asp
145                 150                 155                 160

act atc cag tgg agg gac atc gtc caa aac gtc ttt atg agc aac atg 528
Thr Ile Gln Trp Arg Asp Ile Val Gln Asn Val Phe Met Ser Asn Met
                165                 170                 175

tca atg gac tta cag agc cat ccg agc agt tgc ccc aaa tgt gat cca 576
Ser Met Asp Leu Gln Ser His Pro Ser Ser Cys Pro Lys Cys Asp Pro
            180                 185                 190

agc tgt ccc aat gga agc tgc tgg gga gga gga gag gag aac tgc cag 624
Ser Cys Pro Asn Gly Ser Cys Trp Gly Gly Gly Glu Glu Asn Cys Gln
        195                 200                 205

aaa ttg acc aaa atc atc tgt gcc cag caa tgt tcc cat cgc tgt cgt 672
Lys Leu Thr Lys Ile Ile Cys Ala Gln Gln Cys Ser His Arg Cys Arg
    210                 215                 220

ggc agg tcc ccc agt gac tgc tgc cac aac caa tgt gct gcg ggg tgt 720
Gly Arg Ser Pro Ser Asp Cys Cys His Asn Gln Cys Ala Ala Gly Cys
225                 230                 235                 240

aca ggg ccc cga gag agt gac tgt ctg gtc tgc caa aag ttc caa gat 768
Thr Gly Pro Arg Glu Ser Asp Cys Leu Val Cys Gln Lys Phe Gln Asp
                245                 250                 255

gag gcc aca tgc aaa gac acc tgc cca cca ctc atg ctg tac aac ccc 816
Glu Ala Thr Cys Lys Asp Thr Cys Pro Pro Leu Met Leu Tyr Asn Pro
            260                 265                 270

acc acc tat cag atg gat gtc aac cct gaa ggg aag tac agc ttt ggt 864
Thr Thr Tyr Gln Met Asp Val Asn Pro Glu Gly Lys Tyr Ser Phe Gly
        275                 280                 285

gcc acc tgt gtg aag aag tgc ccc cga aac tac gtg gtg aca gat cat 912
Ala Thr Cys Val Lys Lys Cys Pro Arg Asn Tyr Val Val Thr Asp His
    290                 295                 300

ggc tca tgt gtc cga gcc tgt ggg cct gac tac tac gaa gtg gaa gaa 960
Gly Ser Cys Val Arg Ala Cys Gly Pro Asp Tyr Tyr Glu Val Glu Glu
305                 310                 315                 320

gat ggc atc cgc aag tgt aaa aaa tgt gat ggg ccc tgt cgc aaa gtt 1008

Asp Gly Ile Arg Lys Cys Lys Lys Cys Asp Gly Pro Cys Arg Lys Val
                325                 330                 335

tgt aat ggc ata ggc att ggt gaa ttt aaa gac aca ctc tcc ata aat 1056
Cys Asn Gly Ile Gly Ile Gly Glu Phe Lys Asp Thr Leu Ser Ile Asn
            340                 345                 350

gct aca aac atc aaa cac ttc aaa tac tgc act gcc atc agc ggg gac 1104
Ala Thr Asn Ile Lys His Phe Lys Tyr Cys Thr Ala Ile Ser Gly Asp
        355                 360                 365

ctt cac atc ctg cca gtg gcc ttt aag ggg gat tct ttc acg cgc act 1152
Leu His Ile Leu Pro Val Ala Phe Lys Gly Asp Ser Phe Thr Arg Thr
    370                 375                 380

cct cct cta gac cca cga gaa cta gaa att cta aaa acc gta aag gaa 1200
Pro Pro Leu Asp Pro Arg Glu Leu Glu Ile Leu Lys Thr Val Lys Glu
385                 390                 395                 400

ata aca ggc ttt ttg ctg att cag gct tgg cct gat aac tgg act gac 1248
Ile Thr Gly Phe Leu Leu Ile Gln Ala Trp Pro Asp Asn Trp Thr Asp
                405                 410                 415

ctc cat gct ttc gag aac cta gaa ata ata cgt ggc aga aca aag caa 1296
Leu His Ala Phe Glu Asn Leu Glu Ile Ile Arg Gly Arg Thr Lys Gln
            420                 425                 430

cat ggt cag ttt tct ttg gcg gtc gtt ggc ctg aac atc aca tca ctg 1344
His Gly Gln Phe Ser Leu Ala Val Val Gly Leu Asn Ile Thr Ser Leu
        435                 440                 445

ggg ctg cgt tcc ctc aag gag atc agt gat ggg gat gtg atc att tct 1392
Gly Leu Arg Ser Leu Lys Glu Ile Ser Asp Gly Asp Val Ile Ile Ser
    450                 455                 460

gga aac cga aat ttg tgc tac gca aac aca ata aac tgg aaa aaa ctc 1440
Gly Asn Arg Asn Leu Cys Tyr Ala Asn Thr Ile Asn Trp Lys Lys Leu
465                 470                 475                 480

ttc ggg aca ccc aat cag aaa acc aaa atc atg aac aac aga gct gag 1488
Phe Gly Thr Pro Asn Gln Lys Thr Lys Ile Met Asn Asn Arg Ala Glu
                485                 490                 495

aaa gac tgc aag gcc gtg aac cac gtc tgc aat cct tta tgc tcc tcg 1536
Lys Asp Cys Lys Ala Val Asn His Val Cys Asn Pro Leu Cys Ser Ser
            500                 505                 510

gaa ggc tgc tgg ggc cct gag ccc agg gac tgt gtc tcc tgc cag aat 1584
Glu Gly Cys Trp Gly Pro Glu Pro Arg Asp Cys Val Ser Cys Gln Asn
        515                 520                 525

gtg agc aga ggc agg gag tgc gtg gag aaa tgc aac atc ctg gag ggg 1632
Val Ser Arg Gly Arg Glu Cys Val Glu Lys Cys Asn Ile Leu Glu Gly
    530                 535                 540

gaa cca agg gag ttt gtg gaa aat tct gaa tgc atc cag tgc cat cca 1680
Glu Pro Arg Glu Phe Val Glu Asn Ser Glu Cys Ile Gln Cys His Pro
545                 550                 555                 560

gaa tgt ctg ccc cag gcc atg aac atc acc tgt aca ggc agg ggg cca 1728
Glu Cys Leu Pro Gln Ala Met Asn Ile Thr Cys Thr Gly Arg Gly Pro
                565                 570                 575

gac aac tgc atc cag tgt gcc cac tac att gat ggc cca cac tgt gtc 1776
Asp Asn Cys Ile Gln Cys Ala His Tyr Ile Asp Gly Pro His Cys Val
            580                 585                 590

aag acc tgc cca gct ggc atc atg gga gag aac aac act ctg gtc tgg 1824
Lys Thr Cys Pro Ala Gly Ile Met Gly Glu Asn Asn Thr Leu Val Trp

        595                 600                 605

aag tat gca gat gcc aat aat gtc tgc cac cta tgc cac gcc aac tgt 1872
Lys Tyr Ala Asp Ala Asn Asn Val Cys His Leu Cys His Ala Asn Cys
    610                 615                 620

acc tat gga tgt gct ggg cca ggt ctt caa gga tgt gaa gtg tgg cca 1920
Thr Tyr Gly Cys Ala Gly Pro Gly Leu Gln Gly Cys Glu Val Trp Pro
625                 630                 635                 640

tct ggg tac gtt caa tgg cag tgg atc tta aag acc ttt tgg atc taa 1968
Ser Gly Tyr Val Gln Trp Gln Trp Ile Leu Lys Thr Phe Trp Ile
                645                 650                 655

<210> 18

<211> 655

<212> PRT

<213> Ð¡Êó(Mus musculus)

<400> 18

Met Arg Pro Ser Gly Thr Ala Arg Thr Thr Leu Leu Val Leu Leu Thr
1               5                   10                  15

Ala Leu Cys Ala Ala Gly Gly Ala Leu Glu Glu Lys Lys Val Cys Gln
            20                  25                  30

Gly Thr Ser Asn Arg Leu Thr Gln Leu Gly Thr Phe Glu Asp His Phe
        35                  40                  45

Leu Ser Leu Gln Arg Met Tyr Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
                85                  90                  95

Glu Arg Ile Pro Leu Glu Asn Leu Gln Ile Ile Arg Gly Asn Ala Leu
            100                 105                 110

Tyr Glu Asn Thr Tyr Ala Leu Ala Ile Leu Ser Asn Tyr Gly Thr Asn
        115                 120                 125

Arg Thr Gly Leu Arg Glu Leu Pro Met Arg Asn Leu Gln Glu Ile Leu
    130                 135                 140

Ile Gly Ala Val Arg Phe Ser Asn Asn Pro Ile Leu Cys Asn Met Asp
145                 150                 155                 160

Thr Ile Gln Trp Arg Asp Ile Val Gln Asn Val Phe Met Ser Asn Met
                165                 170                 175

Ser Met Asp Leu Gln Ser His Pro Ser Ser Cys Pro Lys Cys Asp Pro
            180                 185                 190

Ser Cys Pro Asn Gly Ser Cys Trp Gly Gly Gly Glu Glu Asn Cys Gln
        195                 200                 205

Lys Leu Thr Lys Ile Ile Cys Ala Gln Gln Cys Ser His Arg Cys Arg
    210                 215                 220

Gly Arg Ser Pro Ser Asp Cys Cys His Asn Gln Cys Ala Ala Gly Cys
225                 230                 235                 240

Thr Gly Pro Arg Glu Ser Asp Cys Leu Val Cys Gln Lys Phe Gln Asp
                245                 250                 255

Glu Ala Thr Cys Lys Asp Thr Cys Pro Pro Leu Met Leu Tyr Asn Pro
            260                 265                 270

Thr Thr Tyr Gln Met Asp Val Asn Pro Glu Gly Lys Tyr Ser Phe Gly
        275                 280                 285

Ala Thr Cys Val Lys Lys Cys Pro Arg Asn Tyr Val Val Thr Asp His
    290                 295                 300

Gly Ser Cys Val Arg Ala Cys Gly Pro Asp Tyr Tyr Glu Val Glu Glu
305                 310                 315                 320

Asp Gly Ile Arg Lys Cys Lys Lys Cys Asp Gly Pro Cys Arg Lys Val
                325                 330                 335

Cys Asn Gly Ile Gly Ile Gly Glu Phe Lys Asp Thr Leu Ser Ile Asn
            340                 345                 350

Ala Thr Asn Ile Lys His Phe Lys Tyr Cys Thr Ala Ile Ser Gly Asp
        355                 360                 365

Leu His Ile Leu Pro Val Ala Phe Lys Gly Asp Ser Phe Thr Arg Thr
    370                 375                 380

Pro Pro Leu Asp Pro Arg Glu Leu Glu Ile Leu Lys Thr Val Lys Glu
385                 390                 395                 400

Ile Thr Gly Phe Leu Leu Ile Gln Ala Trp Pro Asp Asn Trp Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Gly Leu Asn Ile Thr Ser Leu
        435                 440                 445

Gly Leu Arg Ser Leu Lys Glu Ile Ser Asp Gly Asp Val Ile Ile Ser
    450                 455                 460

Gly Asn Arg Asn Leu Cys Tyr Ala Asn Thr Ile Asn Trp Lys Lys Leu
465                 470                 475                 480

Phe Gly Thr Pro Asn Gln Lys Thr Lys Ile Met Asn Asn Arg Ala Glu
                485                 490                 495

Lys Asp Cys Lys Ala Val Asn His Val Cys Asn Pro Leu Cys Ser Ser
            500                 505                 510

Glu Gly Cys Trp Gly Pro Glu Pro Arg Asp Cys Val Ser Cys Gln Asn
        515                 520                 525

Val Ser Arg Gly Arg Glu Cys Val Glu Lys Cys Asn Ile Leu Glu Gly
    530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575

Asp Asn Cys Ile Gln Cys Ala His Tyr Ile Asp Gly Pro His Cys Val
            580                 585                 590

Lys Thr Cys Pro Ala Gly Ile Met Gly Glu Asn Asn Thr Leu Val Trp
        595                 600                 605

Lys Tyr Ala Asp Ala Asn Asn Val Cys His Leu Cys His Ala Asn Cys
    610                 615                 620

Thr Tyr Gly Cys Ala Gly Pro Gly Leu Gln Gly Cys Glu Val Trp Pro
625                 630                 635                 640

Ser Gly Tyr Val Gln Trp Gln Trp Ile Leu Lys Thr Phe Trp Ile
                645                 650                 655

<210>  19

<211>  1962

<212>  DNA

<213>  ¼¦

<220>

<221>  CDS

<222>  (1)..(1962)

<223>

<400> 19
atg ggt gta cgc agc ccc ctg tcc gcc tct ggg cct cgc ggg gcc gct 48
Met Gly Val Arg Ser Pro Leu Ser Ala Ser Gly Pro Arg Gly Ala Ala
1               5                   10                  15

gtc ctg gtg cta ctg ctg ctg ggc gtc gcg ctg tgt tcc gcc gtg gag 96
Val Leu Val Leu Leu Leu Leu Gly Val Ala Leu Cys Ser Ala Val Glu
            20                  25                  30

gag aag aaa gtt tgt caa ggg aca aat aac aag ttg acc cag ctg ggg 144
Glu Lys Lys Val Cys Gln Gly Thr Asn Asn Lys Leu Thr Gln Leu Gly
        35                  40                  45

cac gtg gaa gac cat ttc acc agc ctg cag aga atg tac aac aac tgc 192
His Val Glu Asp His Phe Thr Ser Leu Gln Arg Met Tyr Asn Asn Cys
    50                  55                  60

gaa gtg gta ctg agt aac ctg gag att acc tac gtg gag cat aat cgc 240
Glu Val Val Leu Ser Asn Leu Glu Ile Thr Tyr Val Glu His Asn Arg
65                  70                  75                  80

gat ctc acc ttc ctt aag acc ata cag gag gtt gca ggc tat gtg ctc 288
Asp Leu Thr Phe Leu Lys Thr Ile Gln Glu Val Ala Gly Tyr Val Leu
                85                  90                  95

att gcg ctt aac atg gtg gac gtc att ccc tta gaa aac ctc cag att 336
Ile Ala Leu Asn Met Val Asp Val Ile Pro Leu Glu Asn Leu Gln Ile
            100                 105                 110

atc cga ggg aat gtg ctt tat gac aac tct ttt gcc ctg gca gtt tta 384
Ile Arg Gly Asn Val Leu Tyr Asp Asn Ser Phe Ala Leu Ala Val Leu
        115                 120                 125

tcc aat tac cac atg aat aaa acc cag gga ctt cga gag ctg cca atg 432
Ser Asn Tyr His Met Asn Lys Thr Gln Gly Leu Arg Glu Leu Pro Met
    130                 135                 140

aaa cgg cta tca gaa att ctc aat gga ggt gtt aaa atc agc aac aac 480
Lys Arg Leu Ser Glu Ile Leu Asn Gly Gly Val Lys Ile Ser Asn Asn
145                 150                 155                 160

ccc aaa ctg tgc aac atg gac act gtt ctc tgg aat gac atc att gat 528
Pro Lys Leu Cys Asn Met Asp Thr Val Leu Trp Asn Asp Ile Ile Asp
                165                 170                 175

aca agc agg aag cct ctc aca gta ctt gac ttt gca agc aat ctt tct 576
Thr Ser Arg Lys Pro Leu Thr Val Leu Asp Phe Ala Ser Asn Leu Ser
            180                 185                 190

tct tgt cca aaa tgc cat ccg aac tgc aca gaa gac cac tgc tgg ggt 624
Ser Cys Pro Lys Cys His Pro Asn Cys Thr Glu Asp His Cys Trp Gly
        195                 200                 205

gct ggt gaa cag aac tgc cag act tta aca aaa gtc atc tgt gcc cag 672
Ala Gly Glu Gln Asn Cys Gln Thr Leu Thr Lys Val Ile Cys Ala Gln
    210                 215                 220

caa tgc tct ggc cgg tgc aga gga aag gtg ccc agt gac tgc tgc cac 720
Gln Cys Ser Gly Arg Cys Arg Gly Lys Val Pro Ser Asp Cys Cys His
225                 230                 235                 240

aat cag tgt gct gca ggg tgc aca gga cct cgg gag agt gac tgc ctg 768
Asn Gln Cys Ala Ala Gly Cys Thr Gly Pro Arg Glu Ser Asp Cys Leu
                245                 250                 255

gca tgc cgc aag ttt cgg gat gat gct acc tgc aag gac aca tgt ccc 816
Ala Cys Arg Lys Phe Arg Asp Asp Ala Thr Cys Lys Asp Thr Cys Pro
            260                 265                 270

cca ctg gtc ctc tat aac ccc acc acc tat caa atg gat gtc aac cct 864
Pro Leu Val Leu Tyr Asn Pro Thr Thr Tyr Gln Met Asp Val Asn Pro
        275                 280                 285

gag gga aaa tac agc ttt gga gcc act tgt gtg agg gaa tgt cca cac 912
Glu Gly Lys Tyr Ser Phe Gly Ala Thr Cys Val Arg Glu Cys Pro His
    290                 295                 300

aac tat gtg gtg aca gat cat ggc tcc tgc gtt cgc tcg tgt aat act 960
Asn Tyr Val Val Thr Asp His Gly Ser Cys Val Arg Ser Cys Asn Thr
305                 310                 315                 320

gat act tac gaa gtg gaa gaa aat ggt gtt cgg aag tgt aaa aaa tgt 1008
Asp Thr Tyr Glu Val Glu Glu Asn Gly Val Arg Lys Cys Lys Lys Cys
                325                 330                 335

gat ggg cta tgt agc aaa gtg tgc aat ggc att gga ata ggt gaa ctt 1056
Asp Gly Leu Cys Ser Lys Val Cys Asn Gly Ile Gly Ile Gly Glu Leu
            340                 345                 350

aaa ggg atc cta tcc ata aat gcc aca aac atc gac tcc ttc aaa aac 1104
Lys Gly Ile Leu Ser Ile Asn Ala Thr Asn Ile Asp Ser Phe Lys Asn
        355                 360                 365

tgt acg aag atc aat ggg gat gtc agc att ctt cct gtt gca ttt cta 1152
Cys Thr Lys Ile Asn Gly Asp Val Ser Ile Leu Pro Val Ala Phe Leu
    370                 375                 380

ggg gat gcc ttc aca aag aca cta ccc ctt gac cct aag aag ctg gat 1200
Gly Asp Ala Phe Thr Lys Thr Leu Pro Leu Asp Pro Lys Lys Leu Asp
385                 390                 395                 400

gtc ttt aga aca gtc aaa gaa ata tca gga ttt ttg ttg att cag gcc 1248
Val Phe Arg Thr Val Lys Glu Ile Ser Gly Phe Leu Leu Ile Gln Ala
                405                 410                 415

tgg cct gat aat gct act gat ctc tat gct ttt gaa aat ctg gag att 1296
Trp Pro Asp Asn Ala Thr Asp Leu Tyr Ala Phe Glu Asn Leu Glu Ile
            420                 425                 430

atc cga ggc cga acc aag cag cac ggc cag tat tcc ctt gct gtt gtt 1344
Ile Arg Gly Arg Thr Lys Gln His Gly Gln Tyr Ser Leu Ala Val Val
        435                 440                 445

aac ttg aaa ata cag tcg ttg ggg ctg cgc tcc ctc aag gaa ata agt 1392
Asn Leu Lys Ile Gln Ser Leu Gly Leu Arg Ser Leu Lys Glu Ile Ser
    450                 455                 460

gat gga gac att gcc att atg aag aac aag aac ctc tgc tat gct gac 1440
Asp Gly Asp Ile Ala Ile Met Lys Asn Lys Asn Leu Cys Tyr Ala Asp
465                 470                 475                 480

acc atg aac tgg cgc agc ttg ttt gct act cag agt cag aaa aca aaa 1488
Thr Met Asn Trp Arg Ser Leu Phe Ala Thr Gln Ser Gln Lys Thr Lys
                485                 490                 495

att ata cag aac aga aat aaa aat gat tgt act gct gac agg cat gtg 1536
Ile Ile Gln Asn Arg Asn Lys Asn Asp Cys Thr Ala Asp Arg His Val
            500                 505                 510

tgt gac ccg ctg tgc tcg gac gtg ggc tgc tgg ggc cca ggg ccc ttc 1584
Cys Asp Pro Leu Cys Ser Asp Val Gly Cys Trp Gly Pro Gly Pro Phe
        515                 520                 525

cac tgc ttt tcc tgc agg ttt ttc agt cgc cag aag gag tgt gta aaa 1632

His Cys Phe Ser Cys Arg Phe Phe Ser Arg Gln Lys Glu Cys Val Lys
    530                 535                 540

cag tgc aac atc ctg caa ggg gag cca cgt gag ttt gaa aga gac tcc      1680
Gln Cys Asn Ile Leu Gln Gly Glu Pro Arg Glu Phe Glu Arg Asp Ser
545                 550                 555                 560

aaa tgc ctc ccc tgc cac tca gag tgt ctg gta cag aac tcc act gca      1728
Lys Cys Leu Pro Cys His Ser Glu Cys Leu Val Gln Asn Ser Thr Ala
                565                 570                 575

tac aac aca acc tgc tct gga ccg ggc cca gac cac tgc atg aag tgt      1776
Tyr Asn Thr Thr Cys Ser Gly Pro Gly Pro Asp His Cys Met Lys Cys
            580                 585                 590

gcc cat ttt ata gat ggt ccc cac tgt gtg aag gcc tgc ccc gct ggg      1824
Ala His Phe Ile Asp Gly Pro His Cys Val Lys Ala Cys Pro Ala Gly
        595                 600                 605

gtc ctg ggt gag aat gat acc ctg gtc tgg aag tat gca gat gcc aat      1872
Val Leu Gly Glu Asn Asp Thr Leu Val Trp Lys Tyr Ala Asp Ala Asn
    610                 615                 620

gct gtt tgc cag ctc tgc cat cca aac tgt aca cga ggg tgc aaa ggg      1920
Ala Val Cys Gln Leu Cys His Pro Asn Cys Thr Arg Gly Cys Lys Gly
625                 630                 635                 640

cca ggt ctt gaa gga tgt cca aat ggc tcc aaa act cca tct              1962
Pro Gly Leu Glu Gly Cys Pro Asn Gly Ser Lys Thr Pro Ser
                645                 650

<210>  20

<211>  654

<212>  PRT

<213>  ¼¦

<400>  20

Met Gly Val Arg Ser Pro Leu Ser Ala Ser Gly Pro Arg Gly Ala Ala
1               5                   10                  15

Val Leu Val Leu Leu Leu Leu Gly Val Ala Leu Cys Ser Ala Val Glu
            20                  25                  30

Glu Lys Lys Val Cys Gln Gly Thr Asn Asn Lys Leu Thr Gln Leu Gly
        35                  40                  45

His Val Glu Asp His Phe Thr Ser Leu Gln Arg Met Tyr Asn Asn Cys
    50                  55                  60

Glu Val Val Leu Ser Asn Leu Glu Ile Thr Tyr Val Glu His Asn Arg
65                  70                  75                  80

Asp Leu Thr Phe Leu Lys Thr Ile Gln Glu Val Ala Gly Tyr Val Leu
                85                  90                  95

Ile Ala Leu Asn Met Val Asp Val Ile Pro Leu Glu Asn Leu Gln Ile

            100                 105                 110

Ile Arg Gly Asn Val Leu Tyr Asp Asn Ser Phe Ala Leu Ala Val Leu
        115                 120                 125

Ser Asn Tyr His Met Asn Lys Thr Gln Gly Leu Arg Glu Leu Pro Met
    130                 135                 140

Lys Arg Leu Ser Glu Ile Leu Asn Gly Gly Val Lys Ile Ser Asn Asn
145                 150                 155                 160

Pro Lys Leu Cys Asn Met Asp Thr Val Leu Trp Asn Asp Ile Ile Asp
                165                 170                 175

Thr Ser Arg Lys Pro Leu Thr Val Leu Asp Phe Ala Ser Asn Leu Ser
            180                 185                 190

Ser Cys Pro Lys Cys His Pro Asn Cys Thr Glu Asp His Cys Trp Gly
        195                 200                 205

Ala Gly Glu Gln Asn Cys Gln Thr Leu Thr Lys Val Ile Cys Ala Gln
    210                 215                 220

Gln Cys Ser Gly Arg Cys Arg Gly Lys Val Pro Ser Asp Cys Cys His
225                 230                 235                 240

Asn Gln Cys Ala Ala Gly Cys Thr Gly Pro Arg Glu Ser Asp Cys Leu
                245                 250                 255

Ala Cys Arg Lys Phe Arg Asp Asp Ala Thr Cys Lys Asp Thr Cys Pro
            260                 265                 270

Pro Leu Val Leu Tyr Asn Pro Thr Thr Tyr Gln Met Asp Val Asn Pro
        275                 280                 285

Glu Gly Lys Tyr Ser Phe Gly Ala Thr Cys Val Arg Glu Cys Pro His
    290                 295                 300

Asn Tyr Val Val Thr Asp His Gly Ser Cys Val Arg Ser Cys Asn Thr
305                 310                 315                 320

Asp Thr Tyr Glu Val Glu Glu Asn Gly Val Arg Lys Cys Lys Lys Cys
                325                 330                 335

Asp Gly Leu Cys Ser Lys Val Cys Asn Gly Ile Gly Ile Gly Glu Leu
            340                 345                 350

Lys Gly Ile Leu Ser Ile Asn Ala Thr Asn Ile Asp Ser Phe Lys Asn
        355                 360                 365

Cys Thr Lys Ile Asn Gly Asp Val Ser Ile Leu Pro Val Ala Phe Leu
    370                 375                 380

Gly Asp Ala Phe Thr Lys Thr Leu Pro Leu Asp Pro Lys Lys Leu Asp
385                 390                 395                 400

Val Phe Arg Thr Val Lys Glu Ile Ser Gly Phe Leu Leu Ile Gln Ala
                405                 410                 415

Trp Pro Asp Asn Ala Thr Asp Leu Tyr Ala Phe Glu Asn Leu Glu Ile
            420                 425                 430

Ile Arg Gly Arg Thr Lys Gln His Gly Gln Tyr Ser Leu Ala Val Val
        435                 440                 445

Asn Leu Lys Ile Gln Ser Leu Gly Leu Arg Ser Leu Lys Glu Ile Ser
    450                 455                 460

Asp Gly Asp Ile Ala Ile Met Lys Asn Lys Asn Leu Cys Tyr Ala Asp
465                 470                 475                 480

Thr Met Asn Trp Arg Ser Leu Phe Ala Thr Gln Ser Gln Lys Thr Lys
                485                 490                 495

Ile Ile Gln Asn Arg Asn Lys Asn Asp Cys Thr Ala Asp Arg His Val
            500                 505                 510

Cys Asp Pro Leu Cys Ser Asp Val Gly Cys Trp Gly Pro Gly Pro Phe
        515                 520                 525

His Cys Phe Ser Cys Arg Phe Phe Ser Arg Gln Lys Glu Cys Val Lys
    530                 535                 540

Gln Cys Asn Ile Leu Gln Gly Glu Pro Arg Glu Phe Glu Arg Asp Ser
545                 550                 555                 560

Lys Cys Leu Pro Cys His Ser Glu Cys Leu Val Gln Asn Ser Thr Ala
                565                 570                 575

Tyr Asn Thr Thr Cys Ser Gly Pro Gly Pro Asp His Cys Met Lys Cys
            580                 585                 590

Ala His Phe Ile Asp Gly Pro His Cys Val Lys Ala Cys Pro Ala Gly
        595                 600                 605

Val Leu Gly Glu Asn Asp Thr Leu Val Trp Lys Tyr Ala Asp Ala Asn
    610                 615                 620

Ala Val Cys Gln Leu Cys His Pro Asn Cys Thr Arg Gly Cys Lys Gly
625                 630                 635                 640

Pro Gly Leu Glu Gly Cys Pro Asn Gly Ser Lys Thr Pro Ser
                645                 650

<210>  21

<211>  4134

<212>  DNA

<213>  'û6¬(Drosophila melanogaster)

<220>

<221>  CDS

<222>  (1)..(3630)

<223>

<400>  21
atg atg att atc agc atg tgg atg agc ata tcg cga gga ttg tgg gac      48
Met Met Ile Ile Ser Met Trp Met Ser Ile Ser Arg Gly Leu Trp Asp
1               5                   10                  15

agc agc tcc atc ttg tcg gtg ctg ctg atc ctc gcc tgc atg gca tcc      96
Ser Ser Ser Ile Leu Ser Val Leu Leu Ile Leu Ala Cys Met Ala Ser
            20                  25                  30

atc acc aca agc tca tcg gtc agc aat gcc ggc tat gtg gat aat ggc      144
Ile Thr Thr Ser Ser Ser Val Ser Asn Ala Gly Tyr Val Asp Asn Gly
        35                  40                  45

aat atg aaa gtc tgc atc ggc act aaa tct cgg ctc tcc gtg ccc tcc      192
Asn Met Lys Val Cys Ile Gly Thr Lys Ser Arg Leu Ser Val Pro Ser
    50                  55                  60

aac aag gaa cat cat tac cga aac ctc aga gat cgg tac acg aac tgt      240
Asn Lys Glu His His Tyr Arg Asn Leu Arg Asp Arg Tyr Thr Asn Cys
65                  70                  75                  80

acg tat gtg gat ggc aac ttg aaa ctg acc tgg cta ccc aac gag aat      288
Thr Tyr Val Asp Gly Asn Leu Lys Leu Thr Trp Leu Pro Asn Glu Asn
                85                  90                  95

ttg gac ctc agc ttc cta gac aac ata cgg gag gtc acc ggc tat att      336
Leu Asp Leu Ser Phe Leu Asp Asn Ile Arg Glu Val Thr Gly Tyr Ile
            100                 105                 110

ctg atc agt cat gtg gac gtt aag aaa gtg gtg ttt ccc aaa cta caa      384
Leu Ile Ser His Val Asp Val Lys Lys Val Val Phe Pro Lys Leu Gln
        115                 120                 125

atc att cgc gga cgc acg ctg ttc agc tta tcc gtg gag gag gag aag      432
Ile Ile Arg Gly Arg Thr Leu Phe Ser Leu Ser Val Glu Glu Glu Lys
    130                 135                 140

tat gcc ttg ttc gtc act tat tcc aaa atg tac acg ctg gag att ccc      480
Tyr Ala Leu Phe Val Thr Tyr Ser Lys Met Tyr Thr Leu Glu Ile Pro
145                 150                 155                 160

gat cta cgc gat gtc tta aat ggc caa gtg ggc ttc cac aac aac tac      528
Asp Leu Arg Asp Val Leu Asn Gly Gln Val Gly Phe His Asn Asn Tyr
                165                 170                 175

aat ctc tgc cac atg cga acg atc cag tgg tcg gag att gta tcc aac      576
Asn Leu Cys His Met Arg Thr Ile Gln Trp Ser Glu Ile Val Ser Asn
            180                 185                 190

ggc acg gat gca tac tac aac tac gac ttt act gct ccg gag cgc gag 624
Gly Thr Asp Ala Tyr Tyr Asn Tyr Asp Phe Thr Ala Pro Glu Arg Glu
        195                 200                 205

tgt ccc aag tgc cac gag agc tgc acg cac gga tgt tgg ggc gag ggt 672
Cys Pro Lys Cys His Glu Ser Cys Thr His Gly Cys Trp Gly Glu Gly
    210                 215                 220

ccc aag aat tgc cag aag ttc agc aag ctc acc tgc tcg cca cag tgt 720
Pro Lys Asn Cys Gln Lys Phe Ser Lys Leu Thr Cys Ser Pro Gln Cys
225                 230                 235                 240

gcc gga ggt cgt tgc tat gga cca aag ccg cgg gag tgt tgt cac ctc 768
Ala Gly Gly Arg Cys Tyr Gly Pro Lys Pro Arg Glu Cys Cys His Leu
                245                 250                 255

ttc tgc gcc gga gga tgc act ggt ccc acg caa aag gat tgc atc gcc 816
Phe Cys Ala Gly Gly Cys Thr Gly Pro Thr Gln Lys Asp Cys Ile Ala
            260                 265                 270

tgc aag aac ttc ttc gac gag gca gta tca aag gag gaa tgc ccg ccc 864
Cys Lys Asn Phe Phe Asp Glu Ala Val Ser Lys Glu Glu Cys Pro Pro
        275                 280                 285

atg cgc aag tac aat ccc acc acc tat gtt ctt gaa acg aat cct gag 912
Met Arg Lys Tyr Asn Pro Thr Thr Tyr Val Leu Glu Thr Asn Pro Glu
    290                 295                 300

gga aag tat gcc tat ggt gcc acc tgc gtc aag gag tgt ccc ggt cat 960
Gly Lys Tyr Ala Tyr Gly Ala Thr Cys Val Lys Glu Cys Pro Gly His
305                 310                 315                 320

ctg ttg cgg gat aat ggc gcc tgc gtg cgc agc tgt ccc cag gac aag 1008
Leu Leu Arg Asp Asn Gly Ala Cys Val Arg Ser Cys Pro Gln Asp Lys
                325                 330                 335

atg gac aag ggg ggc gag tgt gtg ccc tgc aat gga ccg tgc ccc aaa 1056
Met Asp Lys Gly Gly Glu Cys Val Pro Cys Asn Gly Pro Cys Pro Lys
            340                 345                 350

acc tgc ccg ggc gtt act gtc ctg cat gcc ggc aac att gac tcg ttc 1104
Thr Cys Pro Gly Val Thr Val Leu His Ala Gly Asn Ile Asp Ser Phe
        355                 360                 365

cgg aat tgt acg gtg atc gat ggc aac att cgc att ttg gat cag acc 1152
Arg Asn Cys Thr Val Ile Asp Gly Asn Ile Arg Ile Leu Asp Gln Thr
    370                 375                 380

ttc tcg ggc ttc cag gat gtc tat gcc aac tac acg atg gga cca cga 1200
Phe Ser Gly Phe Gln Asp Val Tyr Ala Asn Tyr Thr Met Gly Pro Arg
385                 390                 395                 400

tac ata ccg ctg gat ccc gag cga cgg gag gtg ttc tcc acg gtg aag 1248
Tyr Ile Pro Leu Asp Pro Glu Arg Arg Glu Val Phe Ser Thr Val Lys
                405                 410                 415

gag atc acc ggg tat ctg aat atc gag gga acc cac ccg cag ttc cgg 1296
Glu Ile Thr Gly Tyr Leu Asn Ile Glu Gly Thr His Pro Gln Phe Arg
            420                 425                 430

aat ctg tcg tac ttt cgc aat ctg gaa aca att cat ggc cgc cag ctg 1344
Asn Leu Ser Tyr Phe Arg Asn Leu Glu Thr Ile His Gly Arg Gln Leu
        435                 440                 445

atg gag agc atg ttt gcc gct ttg gcg atc gtt aag tca tcc ctg tac 1392
Met Glu Ser Met Phe Ala Ala Leu Ala Ile Val Lys Ser Ser Leu Tyr
    450                 455                 460

agc ctg gag atg cgc aat ctg aag cag att agt tcc ggc agt gtg gtc 1440
Ser Leu Glu Met Arg Asn Leu Lys Gln Ile Ser Ser Gly Ser Val Val
465                 470                 475                 480

atc cag cat aat aga gac ctc tgc tac gta agc aat atc cgt tgg ccg 1488
Ile Gln His Asn Arg Asp Leu Cys Tyr Val Ser Asn Ile Arg Trp Pro
                485                 490                 495

gcc att cag aag gag ccc gaa cag aag gtg tgg gtc aac gag aat ctc 1536
Ala Ile Gln Lys Glu Pro Glu Gln Lys Val Trp Val Asn Glu Asn Leu
            500                 505                 510

agg gcg gat cta tgc gag aaa aat gga acc att tgc tcg gat cag tgc 1584
Arg Ala Asp Leu Cys Glu Lys Asn Gly Thr Ile Cys Ser Asp Gln Cys
        515                 520                 525

aac gag gac ggc tgc tgg gga gct ggc acg gat cag tgc ctt acc tgc 1632
Asn Glu Asp Gly Cys Trp Gly Ala Gly Thr Asp Gln Cys Leu Thr Cys
    530                 535                 540

aag aac ttc aat ttc aat ggc acc tgc atc gcc gac tgt ggt tat ata 1680
Lys Asn Phe Asn Phe Asn Gly Thr Cys Ile Ala Asp Cys Gly Tyr Ile
545                 550                 555                 560

tcc aat gcc tac aag ttt gac aat aga acg tgc aag ata tgc cat cca 1728
Ser Asn Ala Tyr Lys Phe Asp Asn Arg Thr Cys Lys Ile Cys His Pro
                565                 570                 575

gag tgc cgg act tgc aat gga gct gga gca gat cac tgc cag gag tgc 1776
Glu Cys Arg Thr Cys Asn Gly Ala Gly Ala Asp His Cys Gln Glu Cys
            580                 585                 590

gtc cat gtg agg gac ggt cag cac tgt gtg tcc gag tgc ccg aag aac 1824
Val His Val Arg Asp Gly Gln His Cys Val Ser Glu Cys Pro Lys Asn
        595                 600                 605

aag tac aac gat cgt ggt gtc tgc cga gag tgc cac gcc acc tgc gat 1872
Lys Tyr Asn Asp Arg Gly Val Cys Arg Glu Cys His Ala Thr Cys Asp
    610                 615                 620

gga tgc act ggg ccc aag gac acc atc ggc att gga gcg tgt acg acg 1920
Gly Cys Thr Gly Pro Lys Asp Thr Ile Gly Ile Gly Ala Cys Thr Thr
625                 630                 635                 640

tgc aat ttg gcc att atc aac aat gac gcc aca gta aaa cgc tgc ctg 1968
Cys Asn Leu Ala Ile Ile Asn Asn Asp Ala Thr Val Lys Arg Cys Leu
                645                 650                 655

ctg aag gac gac aag tgc ccc gat ggg tat ttc tgg gag tat gtg cat 2016
Leu Lys Asp Asp Lys Cys Pro Asp Gly Tyr Phe Trp Glu Tyr Val His
            660                 665                 670

ccg caa gag cag gga tcg ctg aag cca ttg gcc ggc aga gca gtt tgc 2064
Pro Gln Glu Gln Gly Ser Leu Lys Pro Leu Ala Gly Arg Ala Val Cys
        675                 680                 685

cga aag tgc cat ccc ctt tgc gag ctg tgc acg aac tac gga tac cat 2112
Arg Lys Cys His Pro Leu Cys Glu Leu Cys Thr Asn Tyr Gly Tyr His
    690                 695                 700

gaa cag gtg tgc tcc aag tgc acc cac tac aag cga cgg gag cag tgc 2160
Glu Gln Val Cys Ser Lys Cys Thr His Tyr Lys Arg Arg Glu Gln Cys
705                 710                 715                 720

gag acc gag tgt ccg gcc gat cac tac acg gat gag gag cag cgc gag 2208
Glu Thr Glu Cys Pro Ala Asp His Tyr Thr Asp Glu Glu Gln Arg Glu
                725                 730                 735

tgc ttc cag cgc cac ccg gaa tgc aat ggt tgc acg ggt ccg ggt gcc 2256

Cys Phe Gln Arg His Pro Glu Cys Asn Gly Cys Thr Gly Pro Gly Ala
            740                 745                 750

gac gat tgc aag tct tgc cgc aac ttt aag ttg ttc gac gcg aat gag      2304
Asp Asp Cys Lys Ser Cys Arg Asn Phe Lys Leu Phe Asp Ala Asn Glu
        755                 760                 765

acg ggt ccc tat gtg aac tcc acg atg ttc aat tgc acc tcg aag tgt      2352
Thr Gly Pro Tyr Val Asn Ser Thr Met Phe Asn Cys Thr Ser Lys Cys
    770                 775                 780

ccc ttg gag atg cga cat gtg aac tat cag tac acg gcc att gga ccc      2400
Pro Leu Glu Met Arg His Val Asn Tyr Gln Tyr Thr Ala Ile Gly Pro
785                 790                 795                 800

tac tgc gca gct agt ccg ccg agg agc agc aag ata act gcc aat ctg      2448
Tyr Cys Ala Ala Ser Pro Pro Arg Ser Ser Lys Ile Thr Ala Asn Leu
                805                 810                 815

gat gtg aac atg atc ttc att atc act ggt gct gtt ctg gtg ccg acg      2496
Asp Val Asn Met Ile Phe Ile Ile Thr Gly Ala Val Leu Val Pro Thr
            820                 825                 830

atc tgc atc ctc tgc gtg gtc aca tac att tgt cgg caa aag caa aag      2544
Ile Cys Ile Leu Cys Val Val Thr Tyr Ile Cys Arg Gln Lys Gln Lys
        835                 840                 845

gcc aag aag gaa aca gtc aag atg acc atg gct ctg tct ggc tgc gag      2592
Ala Lys Lys Glu Thr Val Lys Met Thr Met Ala Leu Ser Gly Cys Glu
    850                 855                 860

gat tcc gag ccg ctg cgt ccc tcg aac att gga gcc aac cta tgc aag      2640
Asp Ser Glu Pro Leu Arg Pro Ser Asn Ile Gly Ala Asn Leu Cys Lys
865                 870                 875                 880

ttg cgc att gtc aag gac gcc gag ttg cgc aag ggc gga gtc ctt gga      2688
Leu Arg Ile Val Lys Asp Ala Glu Leu Arg Lys Gly Gly Val Leu Gly
                885                 890                 895

atg gga gcc ttt gga cga gtg tac aag ggc gtt tgg gtg ccg gag ggt      2736
Met Gly Ala Phe Gly Arg Val Tyr Lys Gly Val Trp Val Pro Glu Gly
            900                 905                 910

gag aac gtc aag att cca gtg gcc att aag gag ctg ctc aag tcc aca      2784
Glu Asn Val Lys Ile Pro Val Ala Ile Lys Glu Leu Leu Lys Ser Thr
        915                 920                 925

ggc gcc gag tca agc gaa gag ttc ctc cgc gaa gcc tac atc atg gcc      2832
Gly Ala Glu Ser Ser Glu Glu Phe Leu Arg Glu Ala Tyr Ile Met Ala
    930                 935                 940

tct gag gag cac gtt aat ctg ctg aag ctc ctg gcc gtg tgc atg tcc      2880
Ser Glu Glu His Val Asn Leu Leu Lys Leu Leu Ala Val Cys Met Ser
945                 950                 955                 960

tca caa atg atg cta atc acg caa ctg atg ccg ctt ggc tgc ctg ttg      2928
Ser Gln Met Met Leu Ile Thr Gln Leu Met Pro Leu Gly Cys Leu Leu
                965                 970                 975

gac tat gtg cga aat aac cgg gac aag atc ggc tct aag gct ctg ctc      2976
Asp Tyr Val Arg Asn Asn Arg Asp Lys Ile Gly Ser Lys Ala Leu Leu
            980                 985                 990

aac tgg agc acg caa atc gcc aag  ggc atg tcg tat ctg  gag gag aag      3024
Asn Trp Ser Thr Gln Ile Ala Lys  Gly Met Ser Tyr Leu  Glu Glu Lys
        995                 1000                 1005

cga ctg  gtc cac aga gac ttg  gct gcc cgc aat gtc  ctg gtg cag      3069
Arg Leu  Val His Arg Asp Leu  Ala Ala Arg Asn Val  Leu Val Gln

1010                    1015                    1020

act ccc tcg ctg gtg aag atc acc gac ttt ggg ctg gcc aag ttg          3114
Thr Pro Ser Leu Val Lys Ile Thr Asp Phe Gly Leu Ala Lys Leu
    1025                    1030                    1035

ctg agc agc gat tcc aat gag tac aag gct gct ggc ggc aag atg          3159
Leu Ser Ser Asp Ser Asn Glu Tyr Lys Ala Ala Gly Gly Lys Met
    1040                    1045                    1050

ccc atc aag tgg ttg gca ctg gag tgc atc cgc aat cgt gta ttc          3204
Pro Ile Lys Trp Leu Ala Leu Glu Cys Ile Arg Asn Arg Val Phe
    1055                    1060                    1065

acc agc aag tcc gat gtc tgg gcc ttt ggt gtg acc att tgg gaa          3249
Thr Ser Lys Ser Asp Val Trp Ala Phe Gly Val Thr Ile Trp Glu
    1070                    1075                    1080

ctg ctg acc ttt ggc cag cgt cca cac gag aac atc cca gct aag          3294
Leu Leu Thr Phe Gly Gln Arg Pro His Glu Asn Ile Pro Ala Lys
    1085                    1090                    1095

gat att ccc gat ctt att gaa gtc ggt ctg aag ctg gag cag ccg          3339
Asp Ile Pro Asp Leu Ile Glu Val Gly Leu Lys Leu Glu Gln Pro
    1100                    1105                    1110

gag att tgt tcg ctg gac att tac tgt aca ctg ctc tcg tgc tgg          3384
Glu Ile Cys Ser Leu Asp Ile Tyr Cys Thr Leu Leu Ser Cys Trp
    1115                    1120                    1125

cac ttg gat gcc gcc atg cgt cca acc ttc aag cag ctg act acg          3429
His Leu Asp Ala Ala Met Arg Pro Thr Phe Lys Gln Leu Thr Thr
    1130                    1135                    1140

gtc ttt gct gag ttc gcc aga gat ccg ggt cgc tat ctg gcc att          3474
Val Phe Ala Glu Phe Ala Arg Asp Pro Gly Arg Tyr Leu Ala Ile
    1145                    1150                    1155

ccc ggg gat aag ttc acc cgg ctg ccg gct tac acg agt cag gat          3519
Pro Gly Asp Lys Phe Thr Arg Leu Pro Ala Tyr Thr Ser Gln Asp
    1160                    1165                    1170

gag aag gat ctc atc cga aaa ttg gct ccc acc acc gat ggg tcc          3564
Glu Lys Asp Leu Ile Arg Lys Leu Ala Pro Thr Thr Asp Gly Ser
    1175                    1180                    1185

gaa gcc att gcg aaa ccc gat gac tac ctg caa ccc aag gca gca          3609
Glu Ala Ile Ala Lys Pro Asp Asp Tyr Leu Gln Pro Lys Ala Ala
    1190                    1195                    1200

cct ggt cct agt cac aga acc gactgcacgg atgagatgcc caagctgaac          3660
Pro Gly Pro Ser His Arg Thr
    1205                    1210

cgctactgca aggatcctag taacaagaat tcgagtaccg gagacgatga gagggattcg    3720

agtgcccggg aagtgggcgt gggtaatctg cgcctcgatc taccagtcga tgaggatgat    3780

tatctgatgc ccacttgcca accgggtccc aacaacaaca acaacatgaa taatcccaat    3840

caaaacaata tggcagctgt gggcgtggct gccggctaca tggatctcat cggagtgccc    3900

gttagtgtgg acaatccgga gtatctgcta aacgcgcaga cactgggagt tggggagtcg    3960

ccgataccca cccagaccat cgggataccg gtgatgggag gcccgggcac catggaggtc    4020

aaggtgccaa tgccaggcag tgagccaacc agctccgatc acgagtacta caatgatacc    4080

caacgggagt tgcagccact gcatcgaaac cgcaacacgg agacgagggt gtag          4134

<210> 22

<211> 1210

<212> PRT

<213> ¹ûÓ¬(Drosophila melanogaster)

<400> 22

Met Met Ile Ile Ser Met Trp Met Ser Ile Ser Arg Gly Leu Trp Asp
1               5                   10                  15

Ser Ser Ser Ile Leu Ser Val Leu Leu Ile Leu Ala Cys Met Ala Ser
            20                  25                  30

Ile Thr Thr Ser Ser Ser Val Ser Asn Ala Gly Tyr Val Asp Asn Gly
        35                  40                  45

Asn Met Lys Val Cys Ile Gly Thr Lys Ser Arg Leu Ser Val Pro Ser
    50                  55                  60

Asn Lys Glu His His Tyr Arg Asn Leu Arg Asp Arg Tyr Thr Asn Cys
65                  70                  75                  80

Thr Tyr Val Asp Gly Asn Leu Lys Leu Thr Trp Leu Pro Asn Glu Asn
                85                  90                  95

Leu Asp Leu Ser Phe Leu Asp Asn Ile Arg Glu Val Thr Gly Tyr Ile
            100                 105                 110

Leu Ile Ser His Val Asp Val Lys Lys Val Val Phe Pro Lys Leu Gln
        115                 120                 125

Ile Ile Arg Gly Arg Thr Leu Phe Ser Leu Ser Val Glu Glu Glu Lys
    130                 135                 140

Tyr Ala Leu Phe Val Thr Tyr Ser Lys Met Tyr Thr Leu Glu Ile Pro
145                 150                 155                 160

Asp Leu Arg Asp Val Leu Asn Gly Gln Val Gly Phe His Asn Asn Tyr
                165                 170                 175

Asn Leu Cys His Met Arg Thr Ile Gln Trp Ser Glu Ile Val Ser Asn
            180                 185                 190

Gly Thr Asp Ala Tyr Tyr Asn Tyr Asp Phe Thr Ala Pro Glu Arg Glu
        195                 200                 205

Cys Pro Lys Cys His Glu Ser Cys Thr His Gly Cys Trp Gly Glu Gly
    210                 215                 220

Pro Lys Asn Cys Gln Lys Phe Ser Lys Leu Thr Cys Ser Pro Gln Cys
225                 230                 235                 240

Ala Gly Gly Arg Cys Tyr Gly Pro Lys Pro Arg Glu Cys Cys His Leu
                245                 250                 255

Phe Cys Ala Gly Gly Cys Thr Gly Pro Thr Gln Lys Asp Cys Ile Ala
            260                 265                 270

Cys Lys Asn Phe Phe Asp Glu Ala Val Ser Lys Glu Glu Cys Pro Pro
        275                 280                 285

Met Arg Lys Tyr Asn Pro Thr Thr Tyr Val Leu Glu Thr Asn Pro Glu
    290                 295                 300

Gly Lys Tyr Ala Tyr Gly Ala Thr Cys Val Lys Glu Cys Pro Gly His
305                 310                 315                 320

Leu Leu Arg Asp Asn Gly Ala Cys Val Arg Ser Cys Pro Gln Asp Lys
                325                 330                 335

Met Asp Lys Gly Gly Glu Cys Val Pro Cys Asn Gly Pro Cys Pro Lys
            340                 345                 350

Thr Cys Pro Gly Val Thr Val Leu His Ala Gly Asn Ile Asp Ser Phe
        355                 360                 365

Arg Asn Cys Thr Val Ile Asp Gly Asn Ile Arg Ile Leu Asp Gln Thr
    370                 375                 380

Phe Ser Gly Phe Gln Asp Val Tyr Ala Asn Tyr Thr Met Gly Pro Arg
385                 390                 395                 400

Tyr Ile Pro Leu Asp Pro Glu Arg Arg Glu Val Phe Ser Thr Val Lys
                405                 410                 415

Glu Ile Thr Gly Tyr Leu Asn Ile Glu Gly Thr His Pro Gln Phe Arg
            420                 425                 430

Asn Leu Ser Tyr Phe Arg Asn Leu Glu Thr Ile His Gly Arg Gln Leu
        435                 440                 445

Met Glu Ser Met Phe Ala Ala Leu Ala Ile Val Lys Ser Ser Leu Tyr
    450                 455                 460

Ser Leu Glu Met Arg Asn Leu Lys Gln Ile Ser Ser Gly Ser Val Val
465                 470                 475                 480

Ile Gln His Asn Arg Asp Leu Cys Tyr Val Ser Asn Ile Arg Trp Pro
                485                 490                 495

Ala Ile Gln Lys Glu Pro Glu Gln Lys Val Trp Val Asn Glu Asn Leu
            500                 505                 510

Arg Ala Asp Leu Cys Glu Lys Asn Gly Thr Ile Cys Ser Asp Gln Cys
        515                 520                 525

Asn Glu Asp Gly Cys Trp Gly Ala Gly Thr Asp Gln Cys Leu Thr Cys
    530                 535                 540

Lys Asn Phe Asn Phe Asn Gly Thr Cys Ile Ala Asp Cys Gly Tyr Ile
545                 550                 555                 560

Ser Asn Ala Tyr Lys Phe Asp Asn Arg Thr Cys Lys Ile Cys His Pro
                565                 570                 575

Glu Cys Arg Thr Cys Asn Gly Ala Gly Ala Asp His Cys Gln Glu Cys
            580                 585                 590

Val His Val Arg Asp Gly Gln His Cys Val Ser Glu Cys Pro Lys Asn
        595                 600                 605

Lys Tyr Asn Asp Arg Gly Val Cys Arg Glu Cys His Ala Thr Cys Asp
    610                 615                 620

Gly Cys Thr Gly Pro Lys Asp Thr Ile Gly Ile Gly Ala Cys Thr Thr
625                 630                 635                 640

Cys Asn Leu Ala Ile Ile Asn Asn Asp Ala Thr Val Lys Arg Cys Leu
                645                 650                 655

Leu Lys Asp Asp Lys Cys Pro Asp Gly Tyr Phe Trp Glu Tyr Val His
            660                 665                 670

Pro Gln Glu Gln Gly Ser Leu Lys Pro Leu Ala Gly Arg Ala Val Cys
        675                 680                 685

Arg Lys Cys His Pro Leu Cys Glu Leu Cys Thr Asn Tyr Gly Tyr His
    690                 695                 700

Glu Gln Val Cys Ser Lys Cys Thr His Tyr Lys Arg Arg Glu Gln Cys
705                 710                 715                 720

Glu Thr Glu Cys Pro Ala Asp His Tyr Thr Asp Glu Glu Gln Arg Glu
                725                 730                 735

Cys Phe Gln Arg His Pro Glu Cys Asn Gly Cys Thr Gly Pro Gly Ala
            740                 745                 750

Asp Asp Cys Lys Ser Cys Arg Asn Phe Lys Leu Phe Asp Ala Asn Glu
        755                 760                 765

Thr Gly Pro Tyr Val Asn Ser Thr Met Phe Asn Cys Thr Ser Lys Cys

770                 775                 780

Pro Leu Glu Met Arg His Val Asn Tyr Gln Tyr Thr Ala Ile Gly Pro
785                 790                 795                 800

Tyr Cys Ala Ala Ser Pro Pro Arg Ser Ser Lys Ile Thr Ala Asn Leu
                805                 810                 815

Asp Val Asn Met Ile Phe Ile Ile Thr Gly Ala Val Leu Val Pro Thr
            820                 825                 830

Ile Cys Ile Leu Cys Val Val Thr Tyr Ile Cys Arg Gln Lys Gln Lys
        835                 840                 845

Ala Lys Lys Glu Thr Val Lys Met Thr Met Ala Leu Ser Gly Cys Glu
    850                 855                 860

Asp Ser Glu Pro Leu Arg Pro Ser Asn Ile Gly Ala Asn Leu Cys Lys
865                 870                 875                 880

Leu Arg Ile Val Lys Asp Ala Glu Leu Arg Lys Gly Gly Val Leu Gly
                885                 890                 895

Met Gly Ala Phe Gly Arg Val Tyr Lys Gly Val Trp Val Pro Glu Gly
            900                 905                 910

Glu Asn Val Lys Ile Pro Val Ala Ile Lys Glu Leu Leu Lys Ser Thr
        915                 920                 925

Gly Ala Glu Ser Ser Glu Glu Phe Leu Arg Glu Ala Tyr Ile Met Ala
    930                 935                 940

Ser Glu Glu His Val Asn Leu Leu Lys Leu Leu Ala Val Cys Met Ser
945                 950                 955                 960

Ser Gln Met Met Leu Ile Thr Gln Leu Met Pro Leu Gly Cys Leu Leu
                965                 970                 975

Asp Tyr Val Arg Asn Asn Arg Asp Lys Ile Gly Ser Lys Ala Leu Leu
            980                 985                 990

Asn Trp Ser Thr Gln Ile Ala Lys  Gly Met Ser Tyr Leu  Glu Glu Lys
        995                 1000                 1005

Arg Leu  Val His Arg Asp Leu  Ala Ala Arg Asn Val  Leu Val Gln
    1010                 1015                 1020

Thr Pro  Ser Leu Val Lys Ile  Thr Asp Phe Gly Leu  Ala Lys Leu
    1025                 1030                 1035

Leu Ser  Ser Asp Ser Asn Glu  Tyr Lys Ala Ala Gly  Gly Lys Met
    1040                 1045                 1050

Pro Ile Lys Trp Leu Ala Leu Glu Cys Ile Arg Asn Arg Val Phe
    1055                1060                1065

Thr Ser Lys Ser Asp Val Trp Ala Phe Gly Val Thr Ile Trp Glu
    1070                1075                1080

Leu Leu Thr Phe Gly Gln Arg Pro His Glu Asn Ile Pro Ala Lys
    1085                1090                1095

Asp Ile Pro Asp Leu Ile Glu Val Gly Leu Lys Leu Glu Gln Pro
    1100                1105                1110

Glu Ile Cys Ser Leu Asp Ile Tyr Cys Thr Leu Leu Ser Cys Trp
    1115                1120                1125

His Leu Asp Ala Ala Met Arg Pro Thr Phe Lys Gln Leu Thr Thr
    1130                1135                1140

Val Phe Ala Glu Phe Ala Arg Asp Pro Gly Arg Tyr Leu Ala Ile
    1145                1150                1155

Pro Gly Asp Lys Phe Thr Arg Leu Pro Ala Tyr Thr Ser Gln Asp
    1160                1165                1170

Glu Lys Asp Leu Ile Arg Lys Leu Ala Pro Thr Thr Asp Gly Ser
    1175                1180                1185

Glu Ala Ile Ala Lys Pro Asp Asp Tyr Leu Gln Pro Lys Ala Ala
    1190                1195                1200

Pro Gly Pro Ser His Arg Thr
    1205                1210

<210> 23
<211> 4281
<212> DNA
<213> 'ûÓ¬(Drosophila melanogaster)

<400> 23
atgctgctgc gacggcgcaa cggcccctgc cccttccccc tgctgcttct gctcctggcc 60
cactgcattt gcatttggcc cgcgtcggcg gcccgcgatc gctacgcccg ccagaacaat 120
cgccagcgcc atcaggatat agatcgcgat cgggatcgag atcgattcct ataccgcagc 180
agttcggccc aaaatcgaca gaggggcggg gccaacttcg ccctgggact gggagccaac 240
ggagtcacca ttcccaccag tctggaggat aagaacaaga acgagttcgt caaggggaaa 300
atctgcatcg gcactaaatc tcggctctcc gtgccctcca acaaggaaca tcattaccga 360

aacctcagag atcggtacac gaactgtacg tatgtggatg gcaacttgaa actgacctgg 420
ctacccaacg agaatttgga cctcagcttc ctagacaaca tacgggaggt caccggctat 480
attctgatca gtcatgtgga cgttaagaaa gtggtgtttc ccaaactaca aatcattcgc 540
ggacgcacgc tgttcagctt atccgtggag gaggagaagt atgccttgtt cgtcacttat 600
tccaaaatgt acacgctgga gattcccgat ctacgcgatg tcttaaatgg ccaagtgggc 660
ttccacaaca actacaatct ctgccacatg cgaacgatcc agtggtcgga gattgtatcc 720
aacggcacgg atgcatacta caactacgac tttactgctc cggagcgcga gtgtcccaag 780
tgccacgaga gctgcacgca cggatgttgg ggcgagggtc ccaagaattg ccagaagttc 840
agcaagctca cctgctcgcc acagtgtgcc ggaggtcgtt gctatggacc aaagccgcgg 900
gagtgttgtc acctcttctg cgccggagga tgcactggtc ccacgcaaaa ggattgcatc 960
gcctgcaaga acttcttcga cgaggcagta tcaaaggagg aatgcccgcc catgcgcaag 1020
tacaatccca ccacctatgt tcttgaaacg aatcctgagg gaaagtatgc ctatggtgcc 1080
acctgcgtca aggagtgtcc cggtcatctg ttgcgggata atggcgcctg cgtgcgcagc 1140
tgtccccagg acaagatgga caaggggggc gagtgtgtgc cctgcaatgg accgtgcccc 1200
aaaacctgcc cgggcgttac tgtcctgcat gccggcaaca ttgactcgtt ccggaattgt 1260
acggtgatcg atggcaacat tcgcattttg gatcagacct tctcgggctt ccaggatgtc 1320
tatgccaact acacgatggg accacgatac ataccgctgg atcccgagcg acgggaggtg 1380
ttctccacgg tgaaggagat caccgggtat ctgaatatcg agggaaccca cccgcagttc 1440
cggaatctgt cgtactttcg caatctggaa acaattcatg gccgccagct gatggagagc 1500
atgtttgccg ctttggcgat cgttaagtca tccctgtaca gcctggagat gcgcaatctg 1560
aagcagatta gttccggcag tgtggtcatc cagcataata gagacctctg ctacgtaagc 1620
aatatccgtt ggccggccat tcagaaggag cccgaacaga aggtgtgggt caacgagaat 1680
ctcagggcgg atctatgcga gaaaaatgga accatttgct cggatcagtg caacgaggac 1740
ggctgctggg gagctggcac ggatcagtgc cttacctgca agaacttcaa tttcaatggc 1800
acctgcatcg ccgactgtgg ttatatatcc aatgcctaca agtttgacaa tagaacgtgc 1860
aagatatgcc atccagagtg ccggacttgc aatggagctg gagcagatca ctgccaggag 1920
tgcgtccatg tgagggacgg tcagcactgt gtgtccgagt gcccgaagaa caagtacaac 1980
gatcgtggtg tctgccgaga gtgccacgcc acctgcgatg gatgcactgg gcccaaggac 2040
accatcggca ttggagcgtg tacgacgtgc aatttggcca ttatcaacaa tgacgccaca 2100
gtaaaacgct gcctgctgaa ggacgacaag tgccccgatg ggtatttctg ggagtatgtg 2160
catccgcaag agcagggatc gctgaagcca ttggccggca gagcagtttg ccgaaagtgc 2220
catccccttt gcgagctgtg cacgaactac ggataccatg aacaggtgtg ctccaagtgc 2280
acccactaca agcgacggga gcagtgcgag accgagtgtc cggccgatca ctacacggat 2340
gaggagcagc gcgagtgctt ccagcgccac ccggaatgca atggttgcac gggtccgggt 2400
gccgacgatt gcaagtcttg ccgcaacttt aagttgttcg acgcgaatga gacgggtccc 2460

tatgtgaact ccacgatgtt caattgcacc tcgaagtgtc ccttggagat gcgacatgtg 2520
aactatcagt acacggccat tggaccctac tgcgcagcta gtccgccgag gagcagcaag 2580
ataactgcca atctggatgt gaacatgatc ttcattatca ctggtgctgt tctggtgccg 2640
acgatctgca tcctctgcgt ggtcacatac atttgtcggc aaaagcaaaa ggccaagaag 2700
gaaacagtca agatgaccat ggctctgtct ggctgcgagg attccgagcc gctgcgtccc 2760
tcgaacattg gagccaacct atgcaagttg cgcattgtca aggacgccga gttgcgcaag 2820
ggcggagtcc ttggaatggg agcctttgga cgagtgtaca agggcgtttg ggtgccggag 2880
ggtgagaacg tcaagattcc agtggccatt aaggagctgc tcaagtccac aggcgccgag 2940
tcaagcgaag agttcctccg cgaagcctac atcatggcct ctgaggagca cgttaatctg 3000
ctgaagctcc tggccgtgtg catgtcctca caaatgatgc taatcacgca actgatgccg 3060
cttggctgcc tgttggacta tgtgcgaaat aaccgggaca agatcggctc taaggctctg 3120
ctcaactgga gcacgcaaat cgccaagggc atgtcgtatc tggaggagaa gcgactggtc 3180
cacagagact tggctgcccg caatgtcctg gtgcagactc cctcgctggt gaagatcacc 3240
gactttgggc tggccaagtt gctgagcagc gattccaatg agtacaaggc tgctggcggc 3300
aagatgccca tcaagtggtt ggcactggag tgcatccgca atcgtgtatt caccagcaag 3360
tccgatgtct gggcctttgg tgtgaccatt tgggaactgc tgacctttgg ccagcgtcca 3420
cacgagaaca tcccagctaa ggatattccc gatcttattg aagtcggtct gaagctggag 3480
cagccggaga tttgttcgct ggacatttac tgtacactgc tctcgtgctg gcacttggat 3540
gccgccatgc gtccaacctt caagcagctg actacggtct ttgctgagtt cgccagagat 3600
ccgggtcgct atctggccat tcccggggat aagttcaccc ggctgccggc ttacacgagt 3660
caggatgaga aggatctcat ccgaaaattg gctcccacca ccgatgggtc cgaagccatt 3720
gcgaaacccg atgactacct gcaacccaag gcagcacctg gtcctagtca cagaaccgac 3780
tgcacggatg agatgcccaa gctgaaccgc tactgcaagg atcctagtaa caagaattcg 3840
agtaccggag acgatgagag ggattcgagt gcccgggaag tgggcgtggg taatctgcgc 3900
ctcgatctac cagtcgatga ggatgattat ctgatgccca cttgccaacc gggtcccaac 3960
aacaacaaca acatgaataa tcccaatcaa aacaatatgg cagctgtggg cgtggctgcc 4020
ggctacatgg atctcatcgg agtgcccgtt agtgtggaca atccggagta tctgctaaac 4080
gcgcagacac tgggagttgg ggagtcgccg atacccaccc agaccatcgg gataccggtg 4140
atgggaggcc cgggcaccat ggaggtcaag gtgccaatgc caggcagtga gccaaccagc 4200
tccgatcacg agtactacaa tgatacccaa cgggagttgc agccactgca tcgaaaccgc 4260
aacacggaga cgagggtgta g 4281

<210> 24
<211> 4281
<212> DNA

<213>  ´óÊó(Rattus norvegicus)

<220>
<221>  CDS
<222>  (1)..(3627)
<223>

<400>  24
atg ctg ctg cga cgg cgc aac ggc ccc tgc ccc ttc ccc ctg ctg ctt      48
Met Leu Leu Arg Arg Arg Asn Gly Pro Cys Pro Phe Pro Leu Leu Leu
1               5                   10                  15

ctg ctc ctg gcc cac tgc att tgc att tgg ccc gcg tcg gcg gcc cgc      96
Leu Leu Leu Ala His Cys Ile Cys Ile Trp Pro Ala Ser Ala Ala Arg
            20                  25                  30

gat cgc tac gcc cgc cag aac aat cgc cag cgc cat cag gat ata gat      144
Asp Arg Tyr Ala Arg Gln Asn Asn Arg Gln Arg His Gln Asp Ile Asp
        35                  40                  45

cgc gat cgg gat cga gat cga ttc cta tac cgc agc agt tcg gcc caa      192
Arg Asp Arg Asp Arg Asp Arg Phe Leu Tyr Arg Ser Ser Ser Ala Gln
    50                  55                  60

aat cga cag agg ggc ggg gcc aac ttc gcc ctg gga ctg gga gcc aac      240
Asn Arg Gln Arg Gly Gly Ala Asn Phe Ala Leu Gly Leu Gly Ala Asn
65                  70                  75                  80

gga gtc acc att ccc acc agt ctg gag gat aag aac aag aac gag ttc      288
Gly Val Thr Ile Pro Thr Ser Leu Glu Asp Lys Asn Lys Asn Glu Phe
                85                  90                  95

gtc aag ggg aaa atc tgc atc ggc act aaa tct cgg ctc tcc gtg ccc      336
Val Lys Gly Lys Ile Cys Ile Gly Thr Lys Ser Arg Leu Ser Val Pro
            100                 105                 110

tcc aac aag gaa cat cat tac cga aac ctc aga gat cgg tac acg aac      384
Ser Asn Lys Glu His His Tyr Arg Asn Leu Arg Asp Arg Tyr Thr Asn
        115                 120                 125

tgt acg tat gtg gat ggc aac ttg aaa ctg acc tgg cta ccc aac gag      432
Cys Thr Tyr Val Asp Gly Asn Leu Lys Leu Thr Trp Leu Pro Asn Glu
    130                 135                 140

aat ttg gac ctc agc ttc cta gac aac ata cgg gag gtc acc ggc tat      480
Asn Leu Asp Leu Ser Phe Leu Asp Asn Ile Arg Glu Val Thr Gly Tyr
145                 150                 155                 160

att ctg atc agt cat gtg gac gtt aag aaa gtg gtg ttt ccc aaa cta      528
Ile Leu Ile Ser His Val Asp Val Lys Lys Val Val Phe Pro Lys Leu
                165                 170                 175

caa atc att cgc gga cgc acg ctg ttc agc tta tcc gtg gag gag gag      576
Gln Ile Ile Arg Gly Arg Thr Leu Phe Ser Leu Ser Val Glu Glu Glu
            180                 185                 190

aag tat gcc ttg ttc gtc act tat tcc aaa atg tac acg ctg gag att      624
Lys Tyr Ala Leu Phe Val Thr Tyr Ser Lys Met Tyr Thr Leu Glu Ile
        195                 200                 205

ccc gat cta cgc gat gtc tta aat ggc caa gtg ggc ttc cac aac aac      672

Pro Asp Leu Arg Asp Val Leu Asn Gly Gln Val Gly Phe His Asn Asn
    210                 215                 220

tac aat ctc tgc cac atg cga acg atc cag tgg tcg gag att gta tcc      720
Tyr Asn Leu Cys His Met Arg Thr Ile Gln Trp Ser Glu Ile Val Ser
225                 230                 235                 240

aac ggc acg gat gca tac tac aac tac gac ttt act gct ccg gag cgc      768
Asn Gly Thr Asp Ala Tyr Tyr Asn Tyr Asp Phe Thr Ala Pro Glu Arg
                245                 250                 255

gag tgt ccc aag tgc cac gag agc tgc acg cac gga tgt tgg ggc gag      816
Glu Cys Pro Lys Cys His Glu Ser Cys Thr His Gly Cys Trp Gly Glu
            260                 265                 270

ggt ccc aag aat tgc cag aag ttc agc aag ctc acc tgc tcg cca cag      864
Gly Pro Lys Asn Cys Gln Lys Phe Ser Lys Leu Thr Cys Ser Pro Gln
        275                 280                 285

tgt gcc gga ggt cgt tgc tat gga cca aag ccg cgg gag tgt tgt cac      912
Cys Ala Gly Gly Arg Cys Tyr Gly Pro Lys Pro Arg Glu Cys Cys His
    290                 295                 300

ctc ttc tgc gcc gga gga tgc act ggt ccc acg caa aag gat tgc atc      960
Leu Phe Cys Ala Gly Gly Cys Thr Gly Pro Thr Gln Lys Asp Cys Ile
305                 310                 315                 320

gcc tgc aag aac ttc ttc gac gag gca gta tca aag gag gaa tgc ccg     1008
Ala Cys Lys Asn Phe Phe Asp Glu Ala Val Ser Lys Glu Glu Cys Pro
                325                 330                 335

ccc atg cgc aag tac aat ccc acc acc tat gtt ctt gaa acg aat cct     1056
Pro Met Arg Lys Tyr Asn Pro Thr Thr Tyr Val Leu Glu Thr Asn Pro
            340                 345                 350

gag gga aag tat gcc tat ggt gcc acc tgc gtc aag gag tgt ccc ggt     1104
Glu Gly Lys Tyr Ala Tyr Gly Ala Thr Cys Val Lys Glu Cys Pro Gly
        355                 360                 365

cat ctg ttg cgg gat aat ggc gcc tgc gtg cgc agc tgt ccc cag gac     1152
His Leu Leu Arg Asp Asn Gly Ala Cys Val Arg Ser Cys Pro Gln Asp
    370                 375                 380

aag atg gac aag ggg ggc gag tgt gtg ccc tgc aat gga ccg tgc ccc     1200
Lys Met Asp Lys Gly Gly Glu Cys Val Pro Cys Asn Gly Pro Cys Pro
385                 390                 395                 400

aaa acc tgc ccg ggc gtt act gtc ctg cat gcc ggc aac att gac tcg     1248
Lys Thr Cys Pro Gly Val Thr Val Leu His Ala Gly Asn Ile Asp Ser
                405                 410                 415

ttc cgg aat tgt acg gtg atc gat ggc aac att cgc att ttg gat cag     1296
Phe Arg Asn Cys Thr Val Ile Asp Gly Asn Ile Arg Ile Leu Asp Gln
            420                 425                 430

acc ttc tcg ggc ttc cag gat gtc tat gcc aac tac acg atg gga cca     1344
Thr Phe Ser Gly Phe Gln Asp Val Tyr Ala Asn Tyr Thr Met Gly Pro
        435                 440                 445

cga tac ata ccg ctg gat ccc gag cga cgg gag gtg ttc tcc acg gtg     1392
Arg Tyr Ile Pro Leu Asp Pro Glu Arg Arg Glu Val Phe Ser Thr Val
    450                 455                 460

aag gag atc acc ggg tat ctg aat atc gag gga acc cac ccg cag ttc     1440
Lys Glu Ile Thr Gly Tyr Leu Asn Ile Glu Gly Thr His Pro Gln Phe
465                 470                 475                 480

cgg aat ctg tcg tac ttt cgc aat ctg gaa aca att cat ggc cgc cag     1488
Arg Asn Leu Ser Tyr Phe Arg Asn Leu Glu Thr Ile His Gly Arg Gln

                485                 490                 495

ctg atg gag agc atg ttt gcc gct ttg gcg atc gtt aag tca tcc ctg 1536
Leu Met Glu Ser Met Phe Ala Ala Leu Ala Ile Val Lys Ser Ser Leu
            500                 505                 510

tac agc ctg gag atg cgc aat ctg aag cag att agt tcc ggc agt gtg 1584
Tyr Ser Leu Glu Met Arg Asn Leu Lys Gln Ile Ser Ser Gly Ser Val
        515                 520                 525

gtc atc cag cat aat aga gac ctc tgc tac gta agc aat atc cgt tgg 1632
Val Ile Gln His Asn Arg Asp Leu Cys Tyr Val Ser Asn Ile Arg Trp
    530                 535                 540

ccg gcc att cag aag gag ccc gaa cag aag gtg tgg gtc aac gag aat 1680
Pro Ala Ile Gln Lys Glu Pro Glu Gln Lys Val Trp Val Asn Glu Asn
545                 550                 555                 560

ctc agg gcg gat cta tgc gag aaa aat gga acc att tgc tcg gat cag 1728
Leu Arg Ala Asp Leu Cys Glu Lys Asn Gly Thr Ile Cys Ser Asp Gln
                565                 570                 575

tgc aac gag gac ggc tgc tgg gga gct ggc acg gat cag tgc ctt acc 1776
Cys Asn Glu Asp Gly Cys Trp Gly Ala Gly Thr Asp Gln Cys Leu Thr
            580                 585                 590

tgc aag aac ttc aat ttc aat ggc acc tgc atc gcc gac tgt ggt tat 1824
Cys Lys Asn Phe Asn Phe Asn Gly Thr Cys Ile Ala Asp Cys Gly Tyr
        595                 600                 605

ata tcc aat gcc tac aag ttt gac aat aga acg tgc aag ata tgc cat 1872
Ile Ser Asn Ala Tyr Lys Phe Asp Asn Arg Thr Cys Lys Ile Cys His
    610                 615                 620

cca gag tgc cgg act tgc aat gga gct gga gca gat cac tgc cag gag 1920
Pro Glu Cys Arg Thr Cys Asn Gly Ala Gly Ala Asp His Cys Gln Glu
625                 630                 635                 640

tgc gtc cat gtg agg gac ggt cag cac tgt gtg tcc gag tgc ccg aag 1968
Cys Val His Val Arg Asp Gly Gln His Cys Val Ser Glu Cys Pro Lys
                645                 650                 655

aac aag tac aac gat cgt ggt gtc tgc cga gag tgc cac gcc acc tgc 2016
Asn Lys Tyr Asn Asp Arg Gly Val Cys Arg Glu Cys His Ala Thr Cys
            660                 665                 670

gat gga tgc act ggg ccc aag gac acc atc ggc att gga gcg tgt acg 2064
Asp Gly Cys Thr Gly Pro Lys Asp Thr Ile Gly Ile Gly Ala Cys Thr
        675                 680                 685

acg tgc aat ttg gcc att atc aac aat gac gcc aca gta aaa cgc tgc 2112
Thr Cys Asn Leu Ala Ile Ile Asn Asn Asp Ala Thr Val Lys Arg Cys
    690                 695                 700

ctg ctg aag gac gac aag tgc ccc gat ggg tat ttc tgg gag tat gtg 2160
Leu Leu Lys Asp Asp Lys Cys Pro Asp Gly Tyr Phe Trp Glu Tyr Val
705                 710                 715                 720

cat ccg caa gag cag gga tcg ctg aag cca ttg gcc ggc aga gca gtt 2208
His Pro Gln Glu Gln Gly Ser Leu Lys Pro Leu Ala Gly Arg Ala Val
                725                 730                 735

tgc cga aag tgc cat ccc ctt tgc gag ctg tgc acg aac tac gga tac 2256
Cys Arg Lys Cys His Pro Leu Cys Glu Leu Cys Thr Asn Tyr Gly Tyr
            740                 745                 750

cat gaa cag gtg tgc tcc aag tgc acc cac tac aag cga cgg gag cag 2304
His Glu Gln Val Cys Ser Lys Cys Thr His Tyr Lys Arg Arg Glu Gln
        755                 760                 765

tgc gag acc gag tgt ccg gcc gat cac tac acg gat gag gag cag cgc 2352
Cys Glu Thr Glu Cys Pro Ala Asp His Tyr Thr Asp Glu Glu Gln Arg
    770                 775                 780

gag tgc ttc cag cgc cac ccg gaa tgc aat ggt tgc acg ggt ccg ggt 2400
Glu Cys Phe Gln Arg His Pro Glu Cys Asn Gly Cys Thr Gly Pro Gly
785                 790                 795                 800

gcc gac gat tgc aag tct tgc cgc aac ttt aag ttg ttc gac gcg aat 2448
Ala Asp Asp Cys Lys Ser Cys Arg Asn Phe Lys Leu Phe Asp Ala Asn
                805                 810                 815

gag acg ggt ccc tat gtg aac tcc acg atg ttc aat tgc acc tcg aag 2496
Glu Thr Gly Pro Tyr Val Asn Ser Thr Met Phe Asn Cys Thr Ser Lys
            820                 825                 830

tgt ccc ttg gag atg cga cat gtg aac tat cag tac acg gcc att gga 2544
Cys Pro Leu Glu Met Arg His Val Asn Tyr Gln Tyr Thr Ala Ile Gly
        835                 840                 845

ccc tac tgc gca gct agt ccg ccg agg agc agc aag ata act gcc aat 2592
Pro Tyr Cys Ala Ala Ser Pro Pro Arg Ser Ser Lys Ile Thr Ala Asn
    850                 855                 860

ctg gat gtg aac atg atc ttc att atc act ggt gct gtt ctg gtg ccg 2640
Leu Asp Val Asn Met Ile Phe Ile Ile Thr Gly Ala Val Leu Val Pro
865                 870                 875                 880

acg atc tgc atc ctc tgc gtg gtc aca tac att tgt cgg caa aag caa 2688
Thr Ile Cys Ile Leu Cys Val Val Thr Tyr Ile Cys Arg Gln Lys Gln
                885                 890                 895

aag gcc aag aag gaa aca gtc aag atg acc atg gct ctg tct ggc tgc 2736
Lys Ala Lys Lys Glu Thr Val Lys Met Thr Met Ala Leu Ser Gly Cys
            900                 905                 910

gag gat tcc gag ccg ctg cgt ccc tcg aac att gga gcc aac cta tgc 2784
Glu Asp Ser Glu Pro Leu Arg Pro Ser Asn Ile Gly Ala Asn Leu Cys
        915                 920                 925

aag ttg cgc att gtc aag gac gcc gag ttg cgc aag ggc gga gtc ctt 2832
Lys Leu Arg Ile Val Lys Asp Ala Glu Leu Arg Lys Gly Gly Val Leu
    930                 935                 940

gga atg gga gcc ttt gga cga gtg tac aag ggc gtt tgg gtg ccg gag 2880
Gly Met Gly Ala Phe Gly Arg Val Tyr Lys Gly Val Trp Val Pro Glu
945                 950                 955                 960

ggt gag aac gtc aag att cca gtg gcc att aag gag ctg ctc aag tcc 2928
Gly Glu Asn Val Lys Ile Pro Val Ala Ile Lys Glu Leu Leu Lys Ser
                965                 970                 975

aca ggc gcc gag tca agc gaa gag ttc ctc cgc gaa gcc tac atc atg 2976
Thr Gly Ala Glu Ser Ser Glu Glu Phe Leu Arg Glu Ala Tyr Ile Met
            980                 985                 990

gcc tct gag gag cac gtt aat ctg ctg aag ctc ctg gcc gtg tgc atg 3024
Ala Ser Glu Glu His Val Asn Leu Leu Lys Leu Leu Ala Val Cys Met
        995                 1000                1005

tcc tca caa atg atg cta atc acg caa ctg atg ccg ctt ggc tgc 3069
Ser Ser Gln Met Met Leu Ile Thr Gln Leu Met Pro Leu Gly Cys
    1010                1015                1020

ctg ttg gac tat gtg cga aat aac cgg gac aag atc ggc tct aag 3114
Leu Leu Asp Tyr Val Arg Asn Asn Arg Asp Lys Ile Gly Ser Lys
    1025                1030                1035

gct ctg ctc aac tgg agc acg caa atc gcc aag ggc atg tcg tat 3159
Ala Leu Leu Asn Trp Ser Thr Gln Ile Ala Lys Gly Met Ser Tyr
    1040                1045                1050

ctg gag gag aag cga ctg gtc cac aga gac ttg gct gcc cgc aat 3204
Leu Glu Glu Lys Arg Leu Val His Arg Asp Leu Ala Ala Arg Asn
    1055                1060                1065

gtc ctg gtg cag act ccc tcg ctg gtg aag atc acc gac ttt ggg 3249
Val Leu Val Gln Thr Pro Ser Leu Val Lys Ile Thr Asp Phe Gly
    1070                1075                1080

ctg gcc aag ttg ctg agc agc gat tcc aat gag tac aag gct gct 3294
Leu Ala Lys Leu Leu Ser Ser Asp Ser Asn Glu Tyr Lys Ala Ala
    1085                1090                1095

ggc ggc aag atg ccc atc aag tgg ttg gca ctg gag tgc atc cgc 3339
Gly Gly Lys Met Pro Ile Lys Trp Leu Ala Leu Glu Cys Ile Arg
    1100                1105                1110

aat cgt gta ttc acc agc aag tcc gat gtc tgg gcc ttt ggt gtg 3384
Asn Arg Val Phe Thr Ser Lys Ser Asp Val Trp Ala Phe Gly Val
    1115                1120                1125

acc att tgg gaa ctg ctg acc ttt ggc cag cgt cca cac gag aac 3429
Thr Ile Trp Glu Leu Leu Thr Phe Gly Gln Arg Pro His Glu Asn
    1130                1135                1140

atc cca gct aag gat att ccc gat ctt att gaa gtc ggt ctg aag 3474
Ile Pro Ala Lys Asp Ile Pro Asp Leu Ile Glu Val Gly Leu Lys
    1145                1150                1155

ctg gag cag ccg gag att tgt tcg ctg gac att tac tgt aca ctg 3519
Leu Glu Gln Pro Glu Ile Cys Ser Leu Asp Ile Tyr Cys Thr Leu
    1160                1165                1170

ctc tcg tgc tgg cac ttg gat gcc gcc atg cgt cca acc ttc aag 3564
Leu Ser Cys Trp His Leu Asp Ala Ala Met Arg Pro Thr Phe Lys
    1175                1180                1185

cag ctg act acg gtc ttt gct gag ttc gcc aga gat ccg ggt cgc 3609
Gln Leu Thr Thr Val Phe Ala Glu Phe Ala Arg Asp Pro Gly Arg
    1190                1195                1200

tat ctg gcc att ccc ggg gataagttca cccggctgcc ggcttacacg 3657
Tyr Leu Ala Ile Pro Gly
    1205

agtcaggatg agaaggatct catccgaaaa ttggctccca ccaccgatgg gtccgaagcc 3717

attgcgaaac ccgatgacta cctgcaaccc aaggcagcac ctggtcctag tcacagaacc 3777

gactgcacgg atgagatgcc caagctgaac cgctactgca aggatcctag taacaagaat 3837

tcgagtaccg gagacgatga gagggattcg agtgcccggg aagtgggcgt gggtaatctg 3897

cgcctcgatc taccagtcga tgaggatgat tatctgatgc ccacttgcca accgggtccc 3957

aacaacaaca acaacatgaa taatcccaat caaaacaata tggcagctgt gggcgtggct 4017

gccggctaca tggatctcat cggagtgccc gttagtgtgg acaatccgga gtatctgcta 4077

aacgcgcaga cactgggagt tggggagtcg ccgataccca cccagaccat cgggataccg 4137

gtgatgggag gcccgggcac catggaggtc aaggtgccaa tgccaggcag tgagccaacc 4197

agctccgatc acgagtacta caatgatacc caacgggagt tgcagccact gcatcgaaac 4257

cgcaacacgg agacgagggt gtag 4281

<210>  25

<211>  1209

<212>  PRT

<213>  ´óÊó(Rattus norvegicus)

<400>  25

Met Leu Leu Arg Arg Arg Asn Gly Pro Cys Pro Phe Pro Leu Leu Leu
1               5                   10                  15

Leu Leu Leu Ala His Cys Ile Cys Ile Trp Pro Ala Ser Ala Ala Arg
            20                  25                  30

Asp Arg Tyr Ala Arg Gln Asn Asn Arg Gln Arg His Gln Asp Ile Asp
        35                  40                  45

Arg Asp Arg Asp Arg Asp Arg Phe Leu Tyr Arg Ser Ser Ser Ala Gln
    50                  55                  60

Asn Arg Gln Arg Gly Gly Ala Asn Phe Ala Leu Gly Leu Gly Ala Asn
65                  70                  75                  80

Gly Val Thr Ile Pro Thr Ser Leu Glu Asp Lys Asn Lys Asn Glu Phe
                85                  90                  95

Val Lys Gly Lys Ile Cys Ile Gly Thr Lys Ser Arg Leu Ser Val Pro
            100                 105                 110

Ser Asn Lys Glu His His Tyr Arg Asn Leu Arg Asp Arg Tyr Thr Asn
        115                 120                 125

Cys Thr Tyr Val Asp Gly Asn Leu Lys Leu Thr Trp Leu Pro Asn Glu
    130                 135                 140

Asn Leu Asp Leu Ser Phe Leu Asp Asn Ile Arg Glu Val Thr Gly Tyr
145                 150                 155                 160

Ile Leu Ile Ser His Val Asp Val Lys Lys Val Val Phe Pro Lys Leu
                165                 170                 175

Gln Ile Ile Arg Gly Arg Thr Leu Phe Ser Leu Ser Val Glu Glu Glu
            180                 185                 190

Lys Tyr Ala Leu Phe Val Thr Tyr Ser Lys Met Tyr Thr Leu Glu Ile
        195                 200                 205

Pro Asp Leu Arg Asp Val Leu Asn Gly Gln Val Gly Phe His Asn Asn
    210                 215                 220

Tyr Asn Leu Cys His Met Arg Thr Ile Gln Trp Ser Glu Ile Val Ser
225                 230                 235                 240

Asn Gly Thr Asp Ala Tyr Tyr Asn Tyr Asp Phe Thr Ala Pro Glu Arg
                245                 250                 255

Glu Cys Pro Lys Cys His Glu Ser Cys Thr His Gly Cys Trp Gly Glu
            260                 265                 270

Gly Pro Lys Asn Cys Gln Lys Phe Ser Lys Leu Thr Cys Ser Pro Gln
        275                 280                 285

Cys Ala Gly Gly Arg Cys Tyr Gly Pro Lys Pro Arg Glu Cys Cys His
    290                 295                 300

Leu Phe Cys Ala Gly Gly Cys Thr Gly Pro Thr Gln Lys Asp Cys Ile
305                 310                 315                 320

Ala Cys Lys Asn Phe Phe Asp Glu Ala Val Ser Lys Glu Glu Cys Pro
                325                 330                 335

Pro Met Arg Lys Tyr Asn Pro Thr Thr Tyr Val Leu Glu Thr Asn Pro
            340                 345                 350

Glu Gly Lys Tyr Ala Tyr Gly Ala Thr Cys Val Lys Glu Cys Pro Gly
        355                 360                 365

His Leu Leu Arg Asp Asn Gly Ala Cys Val Arg Ser Cys Pro Gln Asp
    370                 375                 380

Lys Met Asp Lys Gly Gly Glu Cys Val Pro Cys Asn Gly Pro Cys Pro
385                 390                 395                 400

Lys Thr Cys Pro Gly Val Thr Val Leu His Ala Gly Asn Ile Asp Ser
                405                 410                 415

Phe Arg Asn Cys Thr Val Ile Asp Gly Asn Ile Arg Ile Leu Asp Gln
            420                 425                 430

Thr Phe Ser Gly Phe Gln Asp Val Tyr Ala Asn Tyr Thr Met Gly Pro
        435                 440                 445

Arg Tyr Ile Pro Leu Asp Pro Glu Arg Arg Glu Val Phe Ser Thr Val
    450                 455                 460

Lys Glu Ile Thr Gly Tyr Leu Asn Ile Glu Gly Thr His Pro Gln Phe
465                 470                 475                 480

Arg Asn Leu Ser Tyr Phe Arg Asn Leu Glu Thr Ile His Gly Arg Gln
                485                 490                 495

Leu Met Glu Ser Met Phe Ala Ala Leu Ala Ile Val Lys Ser Ser Leu
            500                 505                 510

Tyr Ser Leu Glu Met Arg Asn Leu Lys Gln Ile Ser Ser Gly Ser Val
        515                 520                 525

Val Ile Gln His Asn Arg Asp Leu Cys Tyr Val Ser Asn Ile Arg Trp
    530                 535                 540

Pro Ala Ile Gln Lys Glu Pro Glu Gln Lys Val Trp Val Asn Glu Asn
545                 550                 555                 560

Leu Arg Ala Asp Leu Cys Glu Lys Asn Gly Thr Ile Cys Ser Asp Gln
                565                 570                 575

Cys Asn Glu Asp Gly Cys Trp Gly Ala Gly Thr Asp Gln Cys Leu Thr
            580                 585                 590

Cys Lys Asn Phe Asn Phe Asn Gly Thr Cys Ile Ala Asp Cys Gly Tyr
        595                 600                 605

Ile Ser Asn Ala Tyr Lys Phe Asp Asn Arg Thr Cys Lys Ile Cys His
    610                 615                 620

Pro Glu Cys Arg Thr Cys Asn Gly Ala Gly Ala Asp His Cys Gln Glu
625                 630                 635                 640

Cys Val His Val Arg Asp Gly Gln His Cys Val Ser Glu Cys Pro Lys
                645                 650                 655

Asn Lys Tyr Asn Asp Arg Gly Val Cys Arg Glu Cys His Ala Thr Cys
            660                 665                 670

Asp Gly Cys Thr Gly Pro Lys Asp Thr Ile Gly Ile Gly Ala Cys Thr
        675                 680                 685

Thr Cys Asn Leu Ala Ile Ile Asn Asn Asp Ala Thr Val Lys Arg Cys
    690                 695                 700

Leu Leu Lys Asp Asp Lys Cys Pro Asp Gly Tyr Phe Trp Glu Tyr Val
705                 710                 715                 720

His Pro Gln Glu Gln Gly Ser Leu Lys Pro Leu Ala Gly Arg Ala Val
                725                 730                 735

Cys Arg Lys Cys His Pro Leu Cys Glu Leu Cys Thr Asn Tyr Gly Tyr
            740                 745                 750

His Glu Gln Val Cys Ser Lys Cys Thr His Tyr Lys Arg Arg Glu Gln
        755                 760                 765

Cys Glu Thr Glu Cys Pro Ala Asp His Tyr Thr Asp Glu Glu Gln Arg

770                    775                    780

Glu Cys Phe Gln Arg His Pro Glu Cys Asn Gly Cys Thr Gly Pro Gly
785                 790                 795                 800

Ala Asp Asp Cys Lys Ser Cys Arg Asn Phe Lys Leu Phe Asp Ala Asn
                805                 810                 815

Glu Thr Gly Pro Tyr Val Asn Ser Thr Met Phe Asn Cys Thr Ser Lys
            820                 825                 830

Cys Pro Leu Glu Met Arg His Val Asn Tyr Gln Tyr Thr Ala Ile Gly
        835                 840                 845

Pro Tyr Cys Ala Ala Ser Pro Pro Arg Ser Ser Lys Ile Thr Ala Asn
    850                 855                 860

Leu Asp Val Asn Met Ile Phe Ile Ile Thr Gly Ala Val Leu Val Pro
865                 870                 875                 880

Thr Ile Cys Ile Leu Cys Val Val Thr Tyr Ile Cys Arg Gln Lys Gln
                885                 890                 895

Lys Ala Lys Lys Glu Thr Val Lys Met Thr Met Ala Leu Ser Gly Cys
            900                 905                 910

Glu Asp Ser Glu Pro Leu Arg Pro Ser Asn Ile Gly Ala Asn Leu Cys
        915                 920                 925

Lys Leu Arg Ile Val Lys Asp Ala Glu Leu Arg Lys Gly Gly Val Leu
    930                 935                 940

Gly Met Gly Ala Phe Gly Arg Val Tyr Lys Gly Val Trp Val Pro Glu
945                 950                 955                 960

Gly Glu Asn Val Lys Ile Pro Val Ala Ile Lys Glu Leu Leu Lys Ser
                965                 970                 975

Thr Gly Ala Glu Ser Ser Glu Glu Phe Leu Arg Glu Ala Tyr Ile Met
            980                 985                 990

Ala Ser Glu Glu His Val Asn Leu  Leu Lys Leu Leu Ala  Val Cys Met
        995                 1000                 1005

Ser Ser  Gln Met Met Leu Ile  Thr Gln Leu Met Pro  Leu Gly Cys
    1010                 1015                 1020

Leu Leu  Asp Tyr Val Arg Asn  Asn Arg Asp Lys Ile  Gly Ser Lys
    1025                 1030                 1035

Ala Leu  Leu Asn Trp Ser Thr  Gln Ile Ala Lys Gly  Met Ser Tyr
    1040                 1045                 1050

Leu Glu Glu Lys Arg Leu Val His Arg Asp Leu Ala Ala Arg Asn
    1055                1060                1065

Val Leu Val Gln Thr Pro Ser Leu Val Lys Ile Thr Asp Phe Gly
    1070                1075                1080

Leu Ala Lys Leu Leu Ser Ser Asp Ser Asn Glu Tyr Lys Ala Ala
    1085                1090                1095

Gly Gly Lys Met Pro Ile Lys Trp Leu Ala Leu Glu Cys Ile Arg
    1100                1105                1110

Asn Arg Val Phe Thr Ser Lys Ser Asp Val Trp Ala Phe Gly Val
    1115                1120                1125

Thr Ile Trp Glu Leu Leu Thr Phe Gly Gln Arg Pro His Glu Asn
    1130                1135                1140

Ile Pro Ala Lys Asp Ile Pro Asp Leu Ile Glu Val Gly Leu Lys
    1145                1150                1155

Leu Glu Gln Pro Glu Ile Cys Ser Leu Asp Ile Tyr Cys Thr Leu
    1160                1165                1170

Leu Ser Cys Trp His Leu Asp Ala Ala Met Arg Pro Thr Phe Lys
    1175                1180                1185

Gln Leu Thr Thr Val Phe Ala Glu Phe Ala Arg Asp Pro Gly Arg
    1190                1195                1200

Tyr Leu Ala Ile Pro Gly
    1205

<210>  26

<211>  3576

<212>  DNA

<213>  °ßÂíÓã(Danio rerio)


<220>

<221>  CDS

<222>  (1)..(3573)

<223>


<400>  26
atg gca gga cca act gaa atc gga ttg ttt ttt acc tta ttg ctc tcc        48
Met Ala Gly Pro Thr Glu Ile Gly Leu Phe Phe Thr Leu Leu Leu Ser

1                   5                   10                  15

ggg agc ttc tgc gcg acg ccg gaa aag aaa gtg tgt cag gga gca aac          96
Gly Ser Phe Cys Ala Thr Pro Glu Lys Lys Val Cys Gln Gly Ala Asn
            20                  25                  30

aac aaa ctg act ctt ctg gga acg gtg gaa gac cat tat cag gtt ctg          144
Asn Lys Leu Thr Leu Leu Gly Thr Val Glu Asp His Tyr Gln Val Leu
        35                  40                  45

ctc aga atg tac aga aac tgc act gtg gtt ctg gag aac ctg gaa att          192
Leu Arg Met Tyr Arg Asn Cys Thr Val Val Leu Glu Asn Leu Glu Ile
    50                  55                  60

aca cat ata aca gag aaa tat gac ctg tcc ttc ctc aag agc atc cag          240
Thr His Ile Thr Glu Lys Tyr Asp Leu Ser Phe Leu Lys Ser Ile Gln
65                  70                  75                  80

gaa gtt ggt ggc tat gtt ctt atc gcg gtc aat acg gtt tcc aaa atc          288
Glu Val Gly Gly Tyr Val Leu Ile Ala Val Asn Thr Val Ser Lys Ile
                85                  90                  95

cct ctg gag aac ctg cgc atc att cgc gga cac tca ctt tat gaa gac          336
Pro Leu Glu Asn Leu Arg Ile Ile Arg Gly His Ser Leu Tyr Glu Asp
            100                 105                 110

aaa ttt gcc ttg gcc gtc ctg gtg aac ttc aac aac agc atc gaa caa          384
Lys Phe Ala Leu Ala Val Leu Val Asn Phe Asn Asn Ser Ile Glu Gln
        115                 120                 125

ggc gta aaa gag ttg ccg ctg act agt tta act gaa ata ctt aag ggt          432
Gly Val Lys Glu Leu Pro Leu Thr Ser Leu Thr Glu Ile Leu Lys Gly
    130                 135                 140

gga gtc aag ttt tgc agg aac gat tat tta tgt aat gtg ggg acc atc          480
Gly Val Lys Phe Cys Arg Asn Asp Tyr Leu Cys Asn Val Gly Thr Ile
145                 150                 155                 160

gag tgg gcc gac atc ctg aac atg aag agc ctg cct aca atc gtg agc          528
Glu Trp Ala Asp Ile Leu Asn Met Lys Ser Leu Pro Thr Ile Val Ser
                165                 170                 175

cat aat ata agc tat gga aaa aac tgt gga aag tgc gat cca agc tgt          576
His Asn Ile Ser Tyr Gly Lys Asn Cys Gly Lys Cys Asp Pro Ser Cys
            180                 185                 190

ttc aat ggc tcc tgc tgg ggc acc gga ccc gac aag tgc cag aga atg          624
Phe Asn Gly Ser Cys Trp Gly Thr Gly Pro Asp Lys Cys Gln Arg Met
        195                 200                 205

acg aaa gtg atc tgt gcg gag cag tgt tca ggg agg tgt aaa gga ccc          672
Thr Lys Val Ile Cys Ala Glu Gln Cys Ser Gly Arg Cys Lys Gly Pro
    210                 215                 220

aga ccc att gac tgc tgt aat gaa cac tgt gct gct gga tgc act gga          720
Arg Pro Ile Asp Cys Cys Asn Glu His Cys Ala Ala Gly Cys Thr Gly
225                 230                 235                 240

ccc aga cct aca gac tgt ctg gcc tgt aag gac ttc cag gat gaa ggg          768
Pro Arg Pro Thr Asp Cys Leu Ala Cys Lys Asp Phe Gln Asp Glu Gly
                245                 250                 255

aca tgt aag gac gca tgt ccg cgg ctc atg ctc tac gac cca aac aca          816
Thr Cys Lys Asp Ala Cys Pro Arg Leu Met Leu Tyr Asp Pro Asn Thr
            260                 265                 270

cac cag ctc gcg cca aac cca tat ggg aag tac agc ttt ggg gca acg          864
His Gln Leu Ala Pro Asn Pro Tyr Gly Lys Tyr Ser Phe Gly Ala Thr
        275                 280                 285

tgc atc aag aca tgc cca cac aac tat gtg gtg acg gat cac ggg gcc 912
Cys Ile Lys Thr Cys Pro His Asn Tyr Val Val Thr Asp His Gly Ala
    290                 295                 300

tgt gtg aga aca tgc agc cct ggc acc tat gaa gtg gat gag ggt gga 960
Cys Val Arg Thr Cys Ser Pro Gly Thr Tyr Glu Val Asp Glu Gly Gly
305                 310                 315                 320

gtt cgc aaa tgt aag agg tgt gaa ggc ctg tgt cca aaa gtg tgc aat 1008
Val Arg Lys Cys Lys Arg Cys Glu Gly Leu Cys Pro Lys Val Cys Asn
                325                 330                 335

ggg ttg gga atg ggg cct tta gcc aat gtc ctg tca atc aat gcc acc 1056
Gly Leu Gly Met Gly Pro Leu Ala Asn Val Leu Ser Ile Asn Ala Thr
            340                 345                 350

aac atc gac tcc ttt gag aac tgc act aaa atc agc ggc aat gtt gcc 1104
Asn Ile Asp Ser Phe Glu Asn Cys Thr Lys Ile Ser Gly Asn Val Ala
        355                 360                 365

atc ctc agc acc aca ttc aga ggt gac cca cat act aac act tca gga 1152
Ile Leu Ser Thr Thr Phe Arg Gly Asp Pro His Thr Asn Thr Ser Gly
    370                 375                 380

ctg gat cca gca aag ctc agt gta ttg agt act gtc aaa gaa atc act 1200
Leu Asp Pro Ala Lys Leu Ser Val Leu Ser Thr Val Lys Glu Ile Thr
385                 390                 395                 400

ggt tac ctg atg att cag ctg tgg ccg gag agc atg cag tcc ctt agt 1248
Gly Tyr Leu Met Ile Gln Leu Trp Pro Glu Ser Met Gln Ser Leu Ser
                405                 410                 415

gcc ttc gaa aac ctt gag gtc atc cga gga cgg aca aaa aca caa gga 1296
Ala Phe Glu Asn Leu Glu Val Ile Arg Gly Arg Thr Lys Thr Gln Gly
            420                 425                 430

acg tac agc ttt gct gtc acc aag acg gcc atc act cat tta ggc atg 1344
Thr Tyr Ser Phe Ala Val Thr Lys Thr Ala Ile Thr His Leu Gly Met
        435                 440                 445

cgt tct ctg agg gag atc agt gac ggg gac gtg tcc atc gtt aag aat 1392
Arg Ser Leu Arg Glu Ile Ser Asp Gly Asp Val Ser Ile Val Lys Asn
    450                 455                 460

aag aat ctc tgc tac agc agc cct gaa cac tgg aaa cgc ctc ttc aag 1440
Lys Asn Leu Cys Tyr Ser Ser Pro Glu His Trp Lys Arg Leu Phe Lys
465                 470                 475                 480

tcc aaa caa cag tcg gtc aaa atg att gaa aat atg gat gct gcc acc 1488
Ser Lys Gln Gln Ser Val Lys Met Ile Glu Asn Met Asp Ala Ala Thr
                485                 490                 495

tgc gcc aat cag aac agc aca tgt aat gag atg tgc acg gct gac ggc 1536
Cys Ala Asn Gln Asn Ser Thr Cys Asn Glu Met Cys Thr Ala Asp Gly
            500                 505                 510

tgc tgg ggt ccc ggc ccc acc atg tgc ttc ggc tgt gag cat tac agc 1584
Cys Trp Gly Pro Gly Pro Thr Met Cys Phe Gly Cys Glu His Tyr Ser
        515                 520                 525

cgc gga aaa cac tgc gtg gct tcc tgc aac ctg ctg aat ggt gag ccg 1632
Arg Gly Lys His Cys Val Ala Ser Cys Asn Leu Leu Asn Gly Glu Pro
    530                 535                 540

cgt gaa tat gag gtc aat aaa aca tgc atg gaa tgc gat cct gaa tgt 1680
Arg Glu Tyr Glu Val Asn Lys Thr Cys Met Glu Cys Asp Pro Glu Cys
545                 550                 555                 560

ctg ctc atg aat gaa acc cag acc tgc aac ggc cct gga ccc gac aaa 1728
Leu Leu Met Asn Glu Thr Gln Thr Cys Asn Gly Pro Gly Pro Asp Lys
                565                 570                 575

tgt aca gtg tgt gca aac tat aaa gac gga ccg cac tgt gtg cat cgc 1776
Cys Thr Val Cys Ala Asn Tyr Lys Asp Gly Pro His Cys Val His Arg
            580                 585                 590

tgc ccg caa ggt gta cca gga gag aaa gac aca ctc atc tgg aaa tac 1824
Cys Pro Gln Gly Val Pro Gly Glu Lys Asp Thr Leu Ile Trp Lys Tyr
        595                 600                 605

gct gac gtg aca cac gtt tgc cag ccc tgt cat gaa aac tgc acc cag 1872
Ala Asp Val Thr His Val Cys Gln Pro Cys His Glu Asn Cys Thr Gln
    610                 615                 620

gga tgt acg ggg cct gat cta aag gac tgc aaa gat ttc aaa agc tct 1920
Gly Cys Thr Gly Pro Asp Leu Lys Asp Cys Lys Asp Phe Lys Ser Ser
625                 630                 635                 640

ggt ttg ccg atg atc gct gct ggc gtt gtc gga ggt cta ctg gcg ttt 1968
Gly Leu Pro Met Ile Ala Ala Gly Val Val Gly Gly Leu Leu Ala Phe
                645                 650                 655

gtt att ctg gct ctt gga gtg gcc gtt ctc ctg cgc aga cgc cac atc 2016
Val Ile Leu Ala Leu Gly Val Ala Val Leu Leu Arg Arg Arg His Ile
            660                 665                 670

cgg agg aag agg act ctg aga cga ctc ctg caa gag aga gag ctt gtg 2064
Arg Arg Lys Arg Thr Leu Arg Arg Leu Leu Gln Glu Arg Glu Leu Val
        675                 680                 685

gag cct ctg acc ccc agc ggc gaa gcc ccc aat cag gcc tta ctg cgc 2112
Glu Pro Leu Thr Pro Ser Gly Glu Ala Pro Asn Gln Ala Leu Leu Arg
    690                 695                 700

atc ctc aaa gag acg gag ttc aag aag atc aaa gtg ctg ggc tcc ggg 2160
Ile Leu Lys Glu Thr Glu Phe Lys Lys Ile Lys Val Leu Gly Ser Gly
705                 710                 715                 720

gct ttc ggc act gtg cac aag ggc ctt tgg gtt cca gaa gga gag aat 2208
Ala Phe Gly Thr Val His Lys Gly Leu Trp Val Pro Glu Gly Glu Asn
                725                 730                 735

gtg aag atc cct gtc gcc atc aag gtc tta aga gaa gcc acg tct ccc 2256
Val Lys Ile Pro Val Ala Ile Lys Val Leu Arg Glu Ala Thr Ser Pro
            740                 745                 750

aaa gct aac aag gag ata atg gat gag gcg tac gtc atg gcc agt gtt 2304
Lys Ala Asn Lys Glu Ile Met Asp Glu Ala Tyr Val Met Ala Ser Val
        755                 760                 765

gag cat ccc cat gtg tgt cgt ctg ctg ggc atc tgc ttg acc tcc aca 2352
Glu His Pro His Val Cys Arg Leu Leu Gly Ile Cys Leu Thr Ser Thr
    770                 775                 780

gtg cag ctc atc act cag ctg atg ccc tac ggc tgc ttg ctg gac tat 2400
Val Gln Leu Ile Thr Gln Leu Met Pro Tyr Gly Cys Leu Leu Asp Tyr
785                 790                 795                 800

gtc aga gaa aac aag gac cgc atc ggc tcc cag cac ctg ctc aac tgg 2448
Val Arg Glu Asn Lys Asp Arg Ile Gly Ser Gln His Leu Leu Asn Trp
                805                 810                 815

tgc gtg cag atc gct aaa ggt atg aat tat cta gaa gag cgc cat ctt 2496
Cys Val Gln Ile Ala Lys Gly Met Asn Tyr Leu Glu Glu Arg His Leu
            820                 825                 830

gtg cac cga gac ctg gca gca cgt aat gtg ttg gta aag acg cct cag 2544

Val His Arg Asp Leu Ala Ala Arg Asn Val Leu Val Lys Thr Pro Gln
        835                 840                 845

cat gtc aag att acc gat ttc ggc ctc gcc aag ctg tta aac gcg gac 2592
His Val Lys Ile Thr Asp Phe Gly Leu Ala Lys Leu Leu Asn Ala Asp
    850                 855                 860

gag aag gag tat cac gca gat gga gga aag gtt cca att aaa tgg atg 2640
Glu Lys Glu Tyr His Ala Asp Gly Gly Lys Val Pro Ile Lys Trp Met
865 870 875 . 880

gcg ctg gag tcc atc cag cac agg act tac acc cac cag agt gac gtc 2688
Ala Leu Glu Ser Ile Gln His Arg Thr Tyr Thr His Gln Ser Asp Val
885 890 895

tgg agc tac ggt gtg acc gtc tgg gag ttg atg acg ttt ggg acg aaa 2736
Trp Ser Tyr Gly Val Thr Val Trp Glu Leu Met Thr Phe Gly Thr Lys
900 905 910

cct tat gat ggg att cct gcc agt gaa atc gcc gga gtt ctg gaa aaa 2784
Pro Tyr Asp Gly Ile Pro Ala Ser Glu Ile Ala Gly Val Leu Glu Lys
915 920 925

gga gaa aga ctt cct caa ccc ccc atc tgc acc att gat gtg tac atg 2832
Gly Glu Arg Leu Pro Gln Pro Pro Ile Cys Thr Ile Asp Val Tyr Met
930 935 940

atc atg gtc aaa tgt tgg atg att gat gct gag agc aga ccc cgc ttc 2880
Ile Met Val Lys Cys Trp Met Ile Asp Ala Glu Ser Arg Pro Arg Phe
945 950 955 960

agg gag ctc atc gca gag ttc act aaa atg gct cgt gac ccg tcc cgc 2928
Arg Glu Leu Ile Ala Glu Phe Thr Lys Met Ala Arg Asp Pro Ser Arg
965 970 975

tat ctg gtc att cag ggg gac gac cgc atg cat tta ccc agt cct tct 2976
Tyr Leu Val Ile Gln Gly Asp Asp Arg Met His Leu Pro Ser Pro Ser
980 985 990

gac tcc aag ttc tac cgc agc ctg atg agc gga gag ctg gac gag gcc 3024
Asp Ser Lys Phe Tyr Arg Ser Leu Met Ser Gly Glu Leu Asp Glu Ala
995 1000 1005

gtg gac gca gac gag tat tta gtg ccc aat cac agc ttc ttc agc 3069
Val Asp Ala Asp Glu Tyr Leu Val Pro Asn His Ser Phe Phe Ser
1010 1015 1020

agc ccg agc acg tcc cgc aca caa ctg ctg cac tct gtg agc ctg 3114
Ser Pro Ser Thr Ser Arg Thr Gln Leu Leu His Ser Val Ser Leu
1025 1030 1035

aac agc agc ttt gga aac tgt aat agt aga aac ggg aat ggt tat 3159
Asn Ser Ser Phe Gly Asn Cys Asn Ser Arg Asn Gly Asn Gly Tyr
1040 1045 1050

cca gtg agg gag aac agc atg gtc ctg cgc tac atc cca gac ccc 3204
Pro Val Arg Glu Asn Ser Met Val Leu Arg Tyr Ile Pro Asp Pro
1055 1060 1065

aca gag cgc ttt cag gag gga gac ttt cag cct gcg ccg ggt tat 3249
Thr Glu Arg Phe Gln Glu Gly Asp Phe Gln Pro Ala Pro Gly Tyr
1070 1075 1080

aac gaa tat atg aac cag aat gag tcc agc atg atc aac cca gtg 3294
Asn Glu Tyr Met Asn Gln Asn Glu Ser Ser Met Ile Asn Pro Val
1085 1090 1095

tac cag cag ccc cac gga ccc ccg cgg acc ctc ctc cac tcc tcc 3339
Tyr Gln Gln Pro His Gly Pro Pro Arg Thr Leu Leu His Ser Ser

1100                 1105                 1110

cca gcg  ctg gac gag acg gaa  gag gag tat ctg aac  tgc ttc aag        3384
Pro Ala  Leu Asp Glu Thr Glu  Glu Glu Tyr Leu Asn  Cys Phe Lys
    1115                 1120                 1125

agc ccg  gct ccg gct tca gtg  gtg gag tat ctg aac  acg tcc cac        3429
Ser Pro  Ala Pro Ala Ser Val  Val Glu Tyr Leu Asn  Thr Ser His
    1130                 1135                 1140

aca cag  ctg ctc tcc aca aag  ccc ttc ttc agc atg  gac aac ccc        3474
Thr Gln  Leu Leu Ser Thr Lys  Pro Phe Phe Ser Met  Asp Asn Pro
    1145                 1150                 1155

gac tac  cag cag gac ttc tgc  ccg ctg gag ctc aaa  aca cac acc        3519
Asp Tyr  Gln Gln Asp Phe Cys  Pro Leu Glu Leu Lys  Thr His Thr
    1160                 1165                 1170

aac ggg  cac ctg ccg gcc gcg  cag aac cag gag tac  atg ggc ctg        3564
Asn Gly  His Leu Pro Ala Ala  Gln Asn Gln Glu Tyr  Met Gly Leu
    1175                 1180                 1185

gag gtg  cac tag                                                      3576
Glu Val  His
    1190

<210>  27

<211>  1191

<212>  PRT

<213>  °ßÂíÓã(Danio rerio)


<400>  27

Met Ala Gly Pro Thr Glu Ile Gly Leu Phe Phe Thr Leu Leu Leu Ser
1               5                   10                  15

Gly Ser Phe Cys Ala Thr Pro Glu Lys Lys Val Cys Gln Gly Ala Asn
            20                  25                  30

Asn Lys Leu Thr Leu Leu Gly Thr Val Glu Asp His Tyr Gln Val Leu
        35                  40                  45

Leu Arg Met Tyr Arg Asn Cys Thr Val Val Leu Glu Asn Leu Glu Ile
    50                  55                  60

Thr His Ile Thr Glu Lys Tyr Asp Leu Ser Phe Leu Lys Ser Ile Gln
65                  70                  75                  80

Glu Val Gly Gly Tyr Val Leu Ile Ala Val Asn Thr Val Ser Lys Ile
                85                  90                  95

Pro Leu Glu Asn Leu Arg Ile Ile Arg Gly His Ser Leu Tyr Glu Asp
            100                 105                 110

Lys Phe Ala Leu Ala Val Leu Val Asn Phe Asn Asn Ser Ile Glu Gln
        115                 120                 125

Gly Val Lys Glu Leu Pro Leu Thr Ser Leu Thr Glu Ile Leu Lys Gly
    130                 135                 140

Gly Val Lys Phe Cys Arg Asn Asp Tyr Leu Cys Asn Val Gly Thr Ile
145                 150                 155                 160

Glu Trp Ala Asp Ile Leu Asn Met Lys Ser Leu Pro Thr Ile Val Ser
                165                 170                 175

His Asn Ile Ser Tyr Gly Lys Asn Cys Gly Lys Cys Asp Pro Ser Cys
            180                 185                 190

Phe Asn Gly Ser Cys Trp Gly Thr Gly Pro Asp Lys Cys Gln Arg Met
        195                 200                 205

Thr Lys Val Ile Cys Ala Glu Gln Cys Ser Gly Arg Cys Lys Gly Pro
    210                 215                 220

Arg Pro Ile Asp Cys Cys Asn Glu His Cys Ala Ala Gly Cys Thr Gly
225                 230                 235                 240

Pro Arg Pro Thr Asp Cys Leu Ala Cys Lys Asp Phe Gln Asp Glu Gly
                245                 250                 255

Thr Cys Lys Asp Ala Cys Pro Arg Leu Met Leu Tyr Asp Pro Asn Thr
            260                 265                 270

His Gln Leu Ala Pro Asn Pro Tyr Gly Lys Tyr Ser Phe Gly Ala Thr
        275                 280                 285

Cys Ile Lys Thr Cys Pro His Asn Tyr Val Val Thr Asp His Gly Ala
    290                 295                 300

Cys Val Arg Thr Cys Ser Pro Gly Thr Tyr Glu Val Asp Glu Gly Gly
305                 310                 315                 320

Val Arg Lys Cys Lys Arg Cys Glu Gly Leu Cys Pro Lys Val Cys Asn
                325                 330                 335

Gly Leu Gly Met Gly Pro Leu Ala Asn Val Leu Ser Ile Asn Ala Thr
            340                 345                 350

Asn Ile Asp Ser Phe Glu Asn Cys Thr Lys Ile Ser Gly Asn Val Ala
        355                 360                 365

Ile Leu Ser Thr Thr Phe Arg Gly Asp Pro His Thr Asn Thr Ser Gly
    370                 375                 380

Leu Asp Pro Ala Lys Leu Ser Val Leu Ser Thr Val Lys Glu Ile Thr
385                 390                 395                 400

Gly Tyr Leu Met Ile Gln Leu Trp Pro Glu Ser Met Gln Ser Leu Ser
                405                 410                 415

Ala Phe Glu Asn Leu Glu Val Ile Arg Gly Arg Thr Lys Thr Gln Gly
            420                 425                 430

Thr Tyr Ser Phe Ala Val Thr Lys Thr Ala Ile Thr His Leu Gly Met
        435                 440                 445

Arg Ser Leu Arg Glu Ile Ser Asp Gly Asp Val Ser Ile Val Lys Asn
    450                 455                 460

Lys Asn Leu Cys Tyr Ser Ser Pro Glu His Trp Lys Arg Leu Phe Lys
465                 470                 475                 480

Ser Lys Gln Gln Ser Val Lys Met Ile Glu Asn Met Asp Ala Ala Thr
                485                 490                 495

Cys Ala Asn Gln Asn Ser Thr Cys Asn Glu Met Cys Thr Ala Asp Gly
            500                 505                 510

Cys Trp Gly Pro Gly Pro Thr Met Cys Phe Gly Cys Glu His Tyr Ser
        515                 520                 525

Arg Gly Lys His Cys Val Ala Ser Cys Asn Leu Leu Asn Gly Glu Pro
    530                 535                 540

Arg Glu Tyr Glu Val Asn Lys Thr Cys Met Glu Cys Asp Pro Glu Cys
545                 550                 555                 560

Leu Leu Met Asn Glu Thr Gln Thr Cys Asn Gly Pro Gly Pro Asp Lys
                565                 570                 575

Cys Thr Val Cys Ala Asn Tyr Lys Asp Gly Pro His Cys Val His Arg
            580                 585                 590

Cys Pro Gln Gly Val Pro Gly Glu Lys Asp Thr Leu Ile Trp Lys Tyr
        595                 600                 605

Ala Asp Val Thr His Val Cys Gln Pro Cys His Glu Asn Cys Thr Gln
    610                 615                 620

Gly Cys Thr Gly Pro Asp Leu Lys Asp Cys Lys Asp Phe Lys Ser Ser
625                 630                 635                 640

Gly Leu Pro Met Ile Ala Ala Gly Val Val Gly Gly Leu Leu Ala Phe
                645                 650                 655

Val Ile Leu Ala Leu Gly Val Ala Val Leu Leu Arg Arg Arg His Ile
            660                 665                 670

Arg Arg Lys Arg Thr Leu Arg Arg Leu Leu Gln Glu Arg Glu Leu Val
        675                 680                 685

Glu Pro Leu Thr Pro Ser Gly Glu Ala Pro Asn Gln Ala Leu Leu Arg
    690                 695                 700

Ile Leu Lys Glu Thr Glu Phe Lys Lys Ile Lys Val Leu Gly Ser Gly
705                 710                 715                 720

Ala Phe Gly Thr Val His Lys Gly Leu Trp Val Pro Glu Gly Glu Asn
                725                 730                 735

Val Lys Ile Pro Val Ala Ile Lys Val Leu Arg Glu Ala Thr Ser Pro
            740                 745                 750

Lys Ala Asn Lys Glu Ile Met Asp Glu Ala Tyr Val Met Ala Ser Val
        755                 760                 765

Glu His Pro His Val Cys Arg Leu Leu Gly Ile Cys Leu Thr Ser Thr
    770                 775                 780

Val Gln Leu Ile Thr Gln Leu Met Pro Tyr Gly Cys Leu Leu Asp Tyr
785                 790                 795                 800

Val Arg Glu Asn Lys Asp Arg Ile Gly Ser Gln His Leu Leu Asn Trp
                805                 810                 815

Cys Val Gln Ile Ala Lys Gly Met Asn Tyr Leu Glu Glu Arg His Leu
            820                 825                 830

Val His Arg Asp Leu Ala Ala Arg Asn Val Leu Val Lys Thr Pro Gln
        835                 840                 845

His Val Lys Ile Thr Asp Phe Gly Leu Ala Lys Leu Leu Asn Ala Asp
    850                 855                 860

Glu Lys Glu Tyr His Ala Asp Gly Gly Lys Val Pro Ile Lys Trp Met
865                 870                 875                 880

Ala Leu Glu Ser Ile Gln His Arg Thr Tyr Thr His Gln Ser Asp Val
                885                 890                 895

Trp Ser Tyr Gly Val Thr Val Trp Glu Leu Met Thr Phe Gly Thr Lys
            900                 905                 910

Pro Tyr Asp Gly Ile Pro Ala Ser Glu Ile Ala Gly Val Leu Glu Lys
        915                 920                 925

Gly Glu Arg Leu Pro Gln Pro Pro Ile Cys Thr Ile Asp Val Tyr Met
    930                 935                 940

Ile Met Val Lys Cys Trp Met Ile Asp Ala Glu Ser Arg Pro Arg Phe

945                 950                 955                 960

Arg Glu Leu Ile Ala Glu Phe Thr Lys Met Ala Arg Asp Pro Ser Arg
                965                 970                 975

Tyr Leu Val Ile Gln Gly Asp Asp Arg Met His Leu Pro Ser Pro Ser
            980                 985                 990

Asp Ser Lys Phe Tyr Arg Ser Leu  Met Ser Gly Glu Leu  Asp Glu Ala
        995                 1000                1005

Val Asp  Ala Asp Glu Tyr Leu  Val Pro Asn His Ser  Phe Phe Ser
    1010                 1015                 1020

Ser Pro  Ser Thr Ser Arg Thr  Gln Leu Leu His Ser  Val Ser Leu
    1025                 1030                 1035

Asn Ser  Ser Phe Gly Asn Cys  Asn Ser Arg Asn Gly  Asn Gly Tyr
    1040                 1045                 1050

Pro Val  Arg Glu Asn Ser Met  Val Leu Arg Tyr Ile  Pro Asp Pro
    1055                 1060                 1065

Thr Glu  Arg Phe Gln Glu Gly  Asp Phe Gln Pro Ala  Pro Gly Tyr
    1070                 1075                 1080

Asn Glu  Tyr Met Asn Gln Asn  Glu Ser Ser Met Ile  Asn Pro Val
    1085                 1090                 1095

Tyr Gln  Gln Pro His Gly Pro  Pro Arg Thr Leu Leu  His Ser Ser
    1100                 1105                 1110

Pro Ala  Leu Asp Glu Thr Glu  Glu Glu Tyr Leu Asn  Cys Phe Lys
    1115                 1120                 1125

Ser Pro  Ala Pro Ala Ser Val  Val Glu Tyr Leu Asn  Thr Ser His
    1130                 1135                 1140

Thr Gln  Leu Leu Ser Thr Lys  Pro Phe Phe Ser Met  Asp Asn Pro
    1145                 1150                 1155

Asp Tyr  Gln Gln Asp Phe Cys  Pro Leu Glu Leu Lys  Thr His Thr
    1160                 1165                 1170

Asn Gly  His Leu Pro Ala Ala  Gln Asn Gln Glu Tyr  Met Gly Leu
    1175                 1180                 1185

Glu Val  His
    1190

## Description

### FIELD OF THE INVENTION

The present invention is in the bio-technology field, more particularly, it relates to a method of preparing vaccines and its prepared bio-vaccines, especially to a novel anti-tumor vaccine, including anti-EFGR molecular vaccine. Molecular vaccines are in the field of new biotechnological drug, and encompass many aspects of biotechnology, such as PCR, Molecular Cloning, Gene Expression, Modem vaccine technology, and biotechnological medicine and pharmacology.

### BACKGROUND OF THE INVENTION

Vaccine is a material that is capable of stimulating the organism immune system to initiate an immune response against specific target substances such as virus, bacteria and the like. Classical concept of vaccine originates from immunity against infection which mainly use treated microbial pathogen (such as virus, bacteria etc.) or its derivatives to immunize the organism so as to produce humoral immune response to prevent infectious diseases. For example, inactivated vaccine is derived by inactivating the active component of the infectious pathogen through chemical reaction. Attenuated vaccine is produced by mutating live virus or bacteria so that it cannot reproduce itself in biological organism. These two classes of vaccines achieve immunization through the surface protein (antigen) reacting with B and T gonorrhea cells. When a human is infected by pathogen, B and T gonorrhea cells can rapidly make response and eradicate it before the pathogeny organism produces destruction. However, these two vaccines have potential danger because they can be polluted by infectious pathogen. For example, there is always a faction of children being infected infantile paralysis because of being vaccinated against infantile paralysis. Therefore, one of development direction is subunit vaccine, also known as gene engineering vaccine or DNA recombinant vaccine, which is produced by DNA recombination technique and only consists of surface protein and initiates an immune response. It can be seen in Gene Engineering Principle, Edited by Ma Jiangang, published by Xi'an Jiaotong University Press, page 240, Nov. 2001. In addition to the aforesaid pathogen itself, its ingredient having immunogenicity and gene expression product, "naked" DNA is also studied to be used as gene vaccine of vaccine. "Naked" DNA consists of an antigen coding gene originated from pathogen and the plasmid DNA as its carrier. After the gene vaccine is introduced into human through injection or particle bombardment, or the like method, the gene fragment can synthesize antigen protein, and to initiate immune response. At present, the Expression-Library Immunization technique is the most systemic and objective means of finding immune active gene. It can be seen in the Fourth Tide: Bioeconomic, edited by Feng Zhanqi and Yang Tongwei, published by Economic Management Press, pages 169 to 171, 2000.

The main function of a vaccine consists in preventing disease. Now vaccine is also used to treat disease. Therefore, for normal human and animals, the function of vaccine is to enhance the ability of disease prevention and resistance of organism so as to prevent from diseases. For human and animal having certain disease or disorder, the function of vaccine is to induce organism to produce reaction aiming at specific pathopoiesia factor in order to eliminate focus and treat disease or disorder. It can be considered that vaccine is a general term of substance that can prevent, or treat, or prevent and treat disease or disorder by affecting the immune system of organism.

The immortalized cell resulted from out of control of cell division adjust in animal is known as tumor cell. The tumor having metastasis ability is known as malignancy, and the malignancy of epithelial tissue is known as cancer. It can be seen in Cell Biology, edited by Zhai Zhonghe, etc., published by Higher Education Press, pages 423 to 427, 2000 (2001 reprint). There have been a great number of fundamental research and clinical application researches about the origination of tumor and the response to tumor of host. About origination, it is shown that a great number of environmental factors have carcinogenicity and mutagenicity to animals. In fact, some tumors relate to exposure to certain specific substances (for exapmle mesothelioma of asbestos and shipyard worker). Known virus can also induce animal tumors, for example, hepatitis B virus relates to liver cancer. In the many cases, host produces immune response so as to resist tumor, while in some other cases, it may be protective.

The tumor cell and normal cell can be distinguished depending on the differentia of quality and quantity of their antigen. The antigen of the tumor cell, especially the tumor specific antigen being peculiar to tumor cell, becomes the research emphasis of tumor prevention and treatment.

Cancer vaccines for tumor treatment are of particular importance. This type of therapeutic vaccine is different from traditional preventive vaccine, in that, it is mainly used to treat patients who already suffer from cancer. The purpose of using cancer vaccine is to stimulate the patient's specific immune response against the tumor so as to achieve effective rejection eventually. The research and development of cancer vaccine has become a focus of tumor immunity treatment intemationally in recent years, and it mainly includes tumor cell vaccine, gene-modified vaccine, polypeptide vaccine, gene/DNA vaccine etc.

Tumor cell vaccines are intact, dead cells produced by treating tumor cells of a patient or sick animal with physical or chemical methods, such that after treatment, those cells will possess therapeutic or auxiliary therapeutic effect. The methods used to treat the tumor cells include X-ray radiation, or treating with organic solvent, etc. After introducing this vaccine to the organism by injection, or other methods, it can stimulate or enhance the patient's immunity against its tumor. Gene-modified vaccine, polypeptide vaccine and gene/DNA vaccines are all vaccines having therapeutic or auxiliary therapeutic effect on tumor, and made by using the tumor antigen or its fragments, or polynucleotides coding for tumor antigen or its fragments, and carriers or cells containing these polynucleotides.

As described above, vaccine plays an important role in disease prevention and treatment. With the aggravation of environmental pollution, the occurring frequency of tumor, as a serious abnormal growth of animal somatic cell, enhances. Therefore, it is necessary to develop new vaccine, especially cancer vaccine. The present invention provides a new method for making vaccine, and a new cancer vaccine-EGFR molecular vaccine, which meets the above requirements.

### SUMMARY OF THE INVENTION

For the first aspect, the present invention provides a preparation method for vaccine. The method comprises:
1) analyzing specific antigen of particular pathogen;
2) obtaining a polynucleotide sequence encoding the specific antigen;
3) obtaining a polynucleotide sequence having a sufficient difference from the polynucleotide sequence; and
4) preparing the vaccine using the polynucleotide sequence obtained in step 3).

For the second aspect, the present invention provides a preparation method for cancer vaccine. The method comprises:
1) analyzing specific antigen of tumor cell in an animal;
2) obtaining a polynucleotide sequence encoding the specific antigen;
3) obtaining a polynucleotide sequence having a sufficient difference from the polynucleotide sequence; and
4) preparing the vaccine using the polynucleotide sequence obtained in step 3).

For the third aspect, the present invention provides a preparation method for EGFR vaccine. The method comprises:
1) analyzing EGFR antigen of tumor cell in an animal;
2) obtaining a polynucleotide sequence encoding the specific antigen;
3) obtaining a polynucleotide sequence having a sufficient difference from the polynucleotide sequence; and
4) preparing the vaccine using the polynucleotide sequence obtained in step 3).

For the forth aspect, the present invention provides a nucleic acid vaccine, which comprises a polynucleotide sequence that has certain differences with the polynucleotide sequence that encodes one specific antigen in a pathogen, and its homology needs to reach certain value. The homology between the polynucleotide sequence contained in the vaccine and the polypeptide encoded by the polynucleotide sequence of the specific antigen is 30-95%.

For the fifth aspect, the present invention provides a nucleic acid vaccine, which comprises two or more polynucleotide sequences that have certain differences with the polynucleotide sequence thate encode two or more specific antigens in a pathogen, and their homologies need to reach certain values. The homologies between the polynucleotide sequences contained in the vaccine and the polypeptides encoded by the polynucleotide sequences of the corresponding specific antigens are 30-95%.

For the sixth aspect, the present invention provides a protein or polypeptide vaccine, which comprises one protein or polypeptide molecule that has certain differences with the polynucleotide sequence of the protein portion of one specific antigen in a pathogen, and its homology is 30-95%.

For the seventh aspect, the present invention provides a protein or polypeptide vaccine, which comprises two or more protein or polypeptide molecules that have certain differences with the polynucleotide sequences of the protein portions of two or more specific antigens in a pathogen, and their homologies are 30-95%.

For the eighth aspect, the present invention provides an epidermal growth factor receptor (EGFR) nucleic acid vaccine, which comprises one polynucleotide sequence that has certain differences with the polynucleotide sequence that encodes an EGFR molecule gene in an organism, and its homology needs to reach certain value. The homology between the epidermal growth factor receptor encoded by polynucleotide sequence contained in the vaccine and the amino acid sequence in the epidermal growth factor receptor of the organism is 30-95%.

For the ninth aspect, the present invention provides an epidermal growth factor receptor (EGFR) nucleic acid vaccine, which comprises two or more polynucleotide sequences that have certain differences with the polynucleotide sequence that encode an EGFR molecule gene in an organism, and their homologies need to reach certain values. The homologies between the epidermal growth factor receptors encoded by the polynucleotide sequences contained in the vaccine and the amino acid sequence in the epidermal growth factor receptor of the organism are 30-95%. The two or more polynucleotide sequences contained in the vaccine may have different sources, and they may have different homologies with the polynucleotide sequence in the EGFR molecule gene of the organism.

For the tenth aspect, the present invention provides an epidermal growth factor receptor (EGFR) protein or polypeptide vaccine, which comprises one variant of EGFR molecule in an organism. The homology between the amino acid sequence of epidermal growth factor receptor contained in the vaccine and the amino acid sequence in the epidermal growth factor receptor of the organism is 30-95%.

For the tenth aspect, the present invention provides an epidermal growth factor receptor (EGFR) protein or polypeptide vaccine, which comprises two or more variants that are different with the EGFR molecule in an organism. The homology between the amino acid sequence of epidermal growth factor receptor contained in the vaccine and the amino acid sequence in the epidermal growth factor receptor of the organism is 30-95%.

For the eleventh aspect, the present invention provides a use of the epidermal growth factor receptor nucleic acid vaccine and the epidermal growth factor receptor protein or polypeptide vaccine in preventing and treating cancer.

### DETAIL DESCRIPTION OF THE INVENTION

As used herein, "pathogen" refers to a foreign organism that invades an organism, including bacteria, virus, fungi, protozoon, helminth etc, which can invade other organisms and result in certain diseases or physiological conditions.

As used herein, "cancer cell" refers to a cell that proliferates and divides incontrollably, disengaging from the normal path of fade and death. The "cancer cell" population consists of a cancer organization. In the present invention, the term "cancer" is generalized, including any types of abnormal proliferation of a cell, and also involving cancer organization or cancer cell. It is required to inhibit the proliferation, or inhibit or remove these cells and organizations.

As used herein, "organism" refers to an animal, especially to "human". The method and vaccine of the present invention can be used in an animal with immune system.

The basic research results in the art or well-known methods can be used to confirm the specific antigen in the pathogen or cancer cell as the target of the vaccine of the invention. Since the abnormal cells in the specific pathogen or in vivo have specific label molecules, such as their specific antigen, the vaccine can be designed by taking these molecules as the target, to induce an immune response in the organism to the harmful substances, organisms or cells, so as to achieve a object for preventing and treating diseases.

According to public databases, such as GenBank, or many websites, the gene sequences or amino acid sequences of corresponding useful antigens can be found. According to central dogma, the sequences deduced from the amino acid sequences or gene sequences can also be used in the present invention. Useful websites includes, but not limited to,
http://www.sanger.ac.uk/Projects/Microbes.
http://bioweb.pasteur.fr/Genoist/TubercuList.

In the present invention, the nucleic acid sequences or amino acid sequences of polypeptide that are against the target antigen are different from the nucleic acid sequences or amino acid sequences of the protein or polypeptide portions that encode the target antigen. Their homologies are 30-95% of the coding products.

The homology is generally determined by sequence analysis software. The term "homology' in the context for two polypeptide sequences refers to two or more sequences that are the same or have a specified percentage of amino acid residues or nucleotides that are the same when compared and aligned for maximum correspondence over a comparison window or designated region. This is just as measured using any number of sequence comparison algorithms or by manual alignment and visual inspection.

For sequence comparison, typically one sequence acts as a reference sequence, to which test sequences are compared. When using a sequence comparison algorithm, test and reference sequences are entered into a computer, and sequence algorithm program parameters are designated. The sequence comparison algorithm then calculates the percent sequence identities for the test sequences relative to the reference sequence, based on the program parameters.

In the present invention, certain amino acid sequence in the antigen is chosen as the reference sequence, and the coding sequences of nucleotide sequences in the vaccine, or the amino acid sequences of proteins or polypeptides in the vaccine are testing sequences. The homology of the testing sequence and reference sequence is one aspect of the present invention.

If the nucleic acid sequence of target antigen or the amino acid sequence of polypeptide is taken as "parent sequence or reference sequence", the nucleic acid sequence or the amino acid sequence of polypeptide used in the present invention can be taken as "daughter sequence or derivative sequence".

The source of "parent sequence or reference sequence" can be the prior art, such as the databases mentioned above, websites, disclosed inventions and papers. They can also derived from the researches on specific antigen, either microbian antigen or cancer antigen in vivo. The parent sequence or reference sequence or its sequence information can be obtained by studying and analyzing the genome, chromosome, protein or polypeptide, mRNA, and DNA molecules.

The "daughter sequence or derivative sequence" is a sequence derived from the "parent sequence or reference sequence". It has some differences with the "parent sequence or reference sequence", but not completely different. The "daughter sequence or derivative sequence" can be existing sequence in nature, which has some structural correlation with the "parent sequence or reference sequence", or can be sequence obtained by artificial process. The former refers to the sequence that encoding homologic protein existed in different organisms or in individual organisms. For example, the coding sequence of human epidermal growth factor receptor, and the coding sequence of mice epidermal growth factor receptor, can be taken as the "parent sequence" and the "daughter sequence" as mentioned in the invention, respectively, or as the "reference sequence" and the "derivative sequence", respectively. These designates can be converted. For example, the coding sequence of mice epidermal growth factor receptor can be taken as the "parent sequence", while the coding sequence of human epidermal growth factor receptor can be taken as the "daughter sequence"; or the coding sequence of human epidermal growth factor receptor can be taken as the "parent sequence", and the coding sequence of mice epidermal growth factor receptor can be taken as the "daughter sequence". The "parent sequence or reference sequence" can be the initial substance, from which the "daughter sequence or derivative sequence" can be obtained by mutation or modification. The "daughter sequence or derivative sequence" that has some difference with the "parent sequence or reference sequence", can also be obtained by artificial synthesis processes. The nucleic acids, proteins and the fragmental substances thereof having "daughter sequence or derivative sequence", are obtained by searching corresponding databases, performing corresponding program on the computer to obtain the "daughter sequence or derivative sequence" of the "parent sequence or reference sequence", and then artificial synthesis. The "daughter sequence or derivative sequence" of the invention can also be obtained by screening corresponding molecule libraries. The nucleic acids, nucleic acid fragments, oligonucleotides, polynucleotides having "daughter sequence or derivative sequence", and the proteins, polypeptides, and fragments thereof having "daughter sequence or derivative sequence" can be used in the embodiments of the invention. In the embodiments of the invention, various modifications can be carried out on the nucleic acids, nucleic acid fragments, oligonucleotides, polynucleotides having "daughter sequence or derivative sequence", and the proteins, polypeptides, and fragments thereof having "daughter sequence or derivative sequence". It is also possible to include them in suitable vectors or cells or viruses, to achieve the object of the invention. The above-mentioned various substances used in the embodiments of the invention can be isolated, purified; or they can be mixed together, and even mixed with certain substances, such as adjuvant.

As used herein, the phrases "nucleic acid" or "nucleic acid sequence" refer to an oligonucleotide, nucleotide, polynucleotide, or to a fragment of any of these, to DNA or RNA of genomic or synthetic origin, to peptide nucleic acid (PNA), or to any DNA-like or RNA-like material, nature or synthetic origin.

The "coding sequence of' or "nucleotide sequence encoding" a particular polypeptide or protein", is a nucleic acid sequence which is transcribed and translated into a polypeptide or protein when placed under the control of appropriate regulatory sequences.

The term "gene" means the segment of DNA involved in producing a polypeptide chain. It includes regions preceding and following the coding region (leader and trailer) as well as, where applicable, intervening sequences (introns) between individual coding segments (exons).

"Amino acid" or "amino acid sequence" as used herein refer to an oligopeptide, peptide, polypeptide, or protein sequence, or to a fragment, portion, or subunit of any of these, and to naturally occurring or synthetic molecules.

The term "polypeptide" as used herein, refers to amino acids joined to each other by peptide bonds or modified peptide bonds, i.e., peptide isosteres, and may contain modified amino acids other than the 20 gene-encoded amino acids. The polypeptides may be modified by either natural processing, such as posttranslational processing, or by chemical modification techniques which are well known in the art. Modification can occur anywhere in the polypeptide, including the peptide backbone, the amino acid side-chains and the amino or carboxyl termini. It will be appreciated that the same type of modification may be present in the same or varying degrees at several sites in a given polypeptide. Also a given polypeptide may have many types of modifications. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of a phosphytidylinositol, cross-linking cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristolyation, oxidation, pergylation, proteolytic processing, phosphorylation, prenylation, racemization, selenoylation, sulfation, and transfer-RNA mediated addition of amino acids to protein such as arginylation. (See Creighton, T. E., Proteins - Structure and Molecular Properties, 2nd Ed., W. H. Freeman and Company, New York (1993); Posttranslational Covalent Modification of Proteins, B. C. Johnson, Ed., Academic Press, New York, pp. 1-12 (1983)).

As used herein, the term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

As used herein, the term "purified" does not require absolute purity; rather, it is intended as a relative definition.

As used herein, the term "recombinant" means that the nucleic acid is adjacent to a "backbone" nucleic acid to which it is not adjacent in its natural environment. Backbone molecules according to the invention include nucleic acids such as expression vectors, self-replicating nucleic acids, viruses, integrating nucleic acids, and other vectors or nucleic acids used to maintain or manipulate a nucleic acid insert of interest.

"Recombinant" polypeptides or proteins refer to polypeptides or proteins produced by recombinant DNA techniques; i.e., produced from cells transformed by an exogenous DNA construct encoding the desired polypeptide or protein. "Synthetic" polypeptides or protein are those prepared by chemical synthesis. Solid-phase chemical peptide synthesis methods can also by used to synthesize the polypeptide or fragments of the invention. Such methods have been known in the art since the early 1960's (Merrifield, R. B., J. Am. Chem. Soc., 85: 2149-2154, 1963) (See also Stewart, J. W. and Young, J. D., Solid Phase Peptide Synthesis, 2nd Ed., Pierce Chemical Co., Rockford, I11, pp. 11-12) and have recently been employed in commercially available laboratory peptide design and synthesis kits (Cambridge Research Biochemicals). Such commercially available laboratory kits have generally utilized the teachings of H. M. Geysen et al, Proc. Natl. Acad. Sci., USA, 81:3998 (1984) and provide for synthesizing peptides upon the tips of a multitude of "rods" or "pins" all of which are connected to a single plate. When such a system is utilized, a plate of rods or pins is inverted and inserted into a second plate of corresponding wells or reservoirs, which contain solutions for attaching or anchoring an appropriate amino acid to the pin's or rod's tips. By repeating such a process step, i.e., inverting and inserting the rod's and pin's tips into appropriate solutions, amino acids are built into desired peptides. In addition, a number of available FMOC peptide synthesis systems are available. For example, assembly of a polypeptide or fragment can be carried out on a solid support using an Applied Biosystems, Inc. Model 431A automated peptide synthesizer. Such equipment provides ready access to the peptides of the invention, either by direct synthesis or by synthesis of a series of fragments that can be coupled using other known techniques.

A promoter sequence is "operably linked to" a coding sequence when RNA polymerase which initiates transcription at the promoter will transcribe the coding sequence into mRNA.

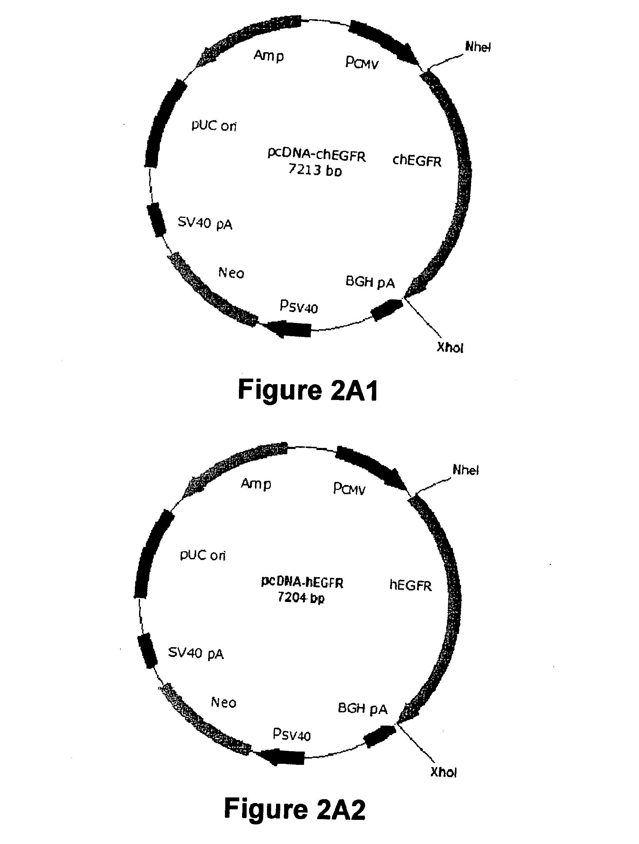

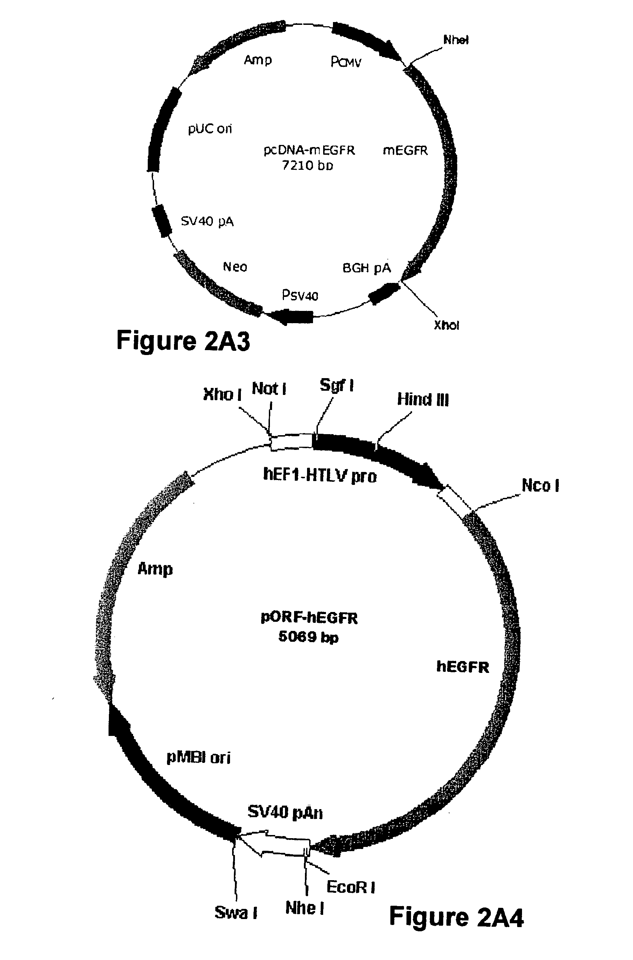

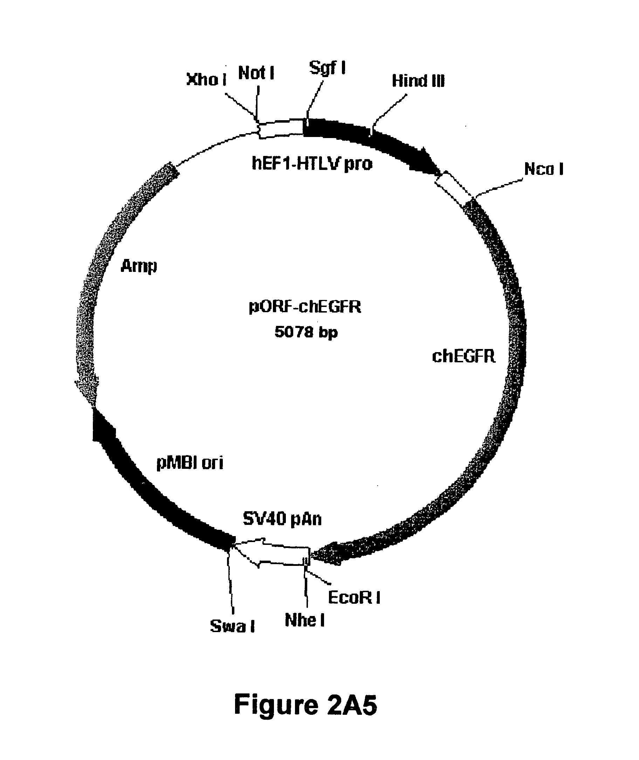

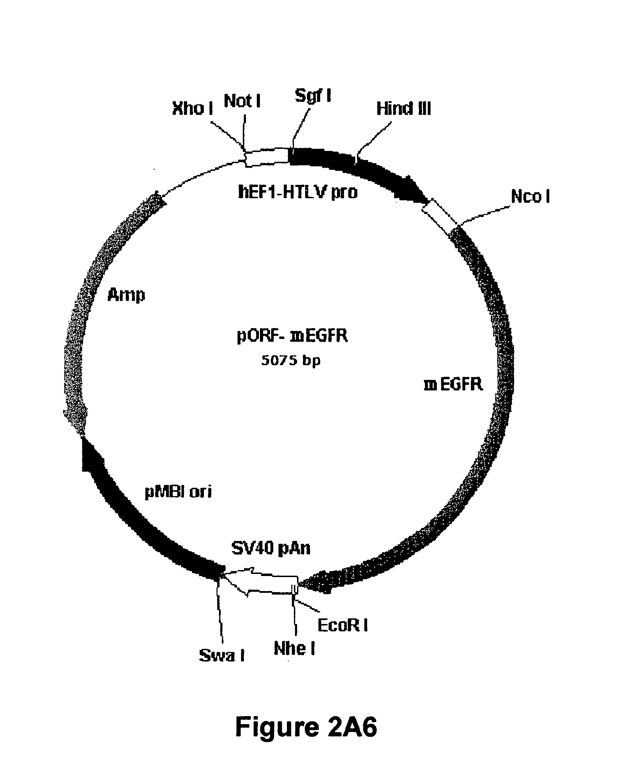

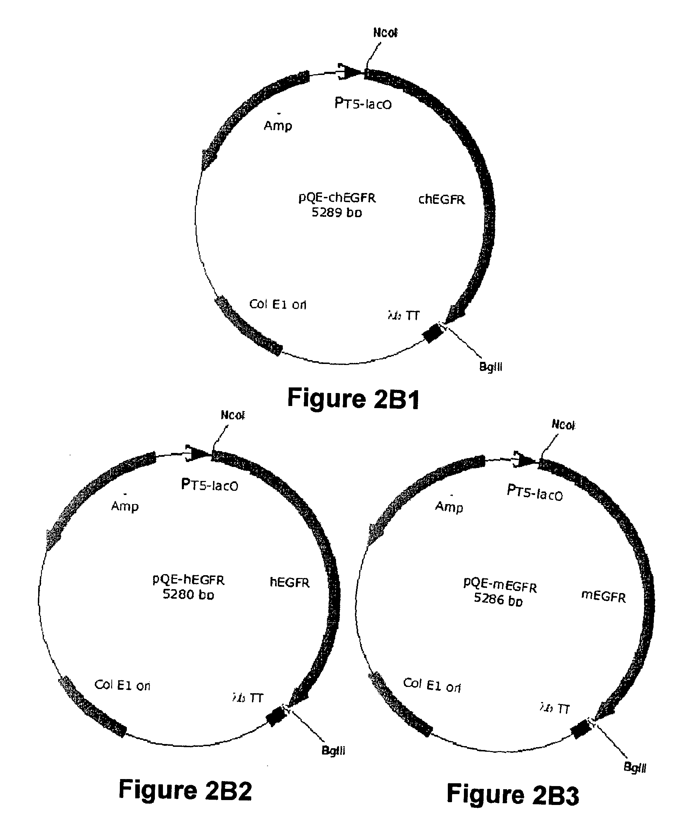

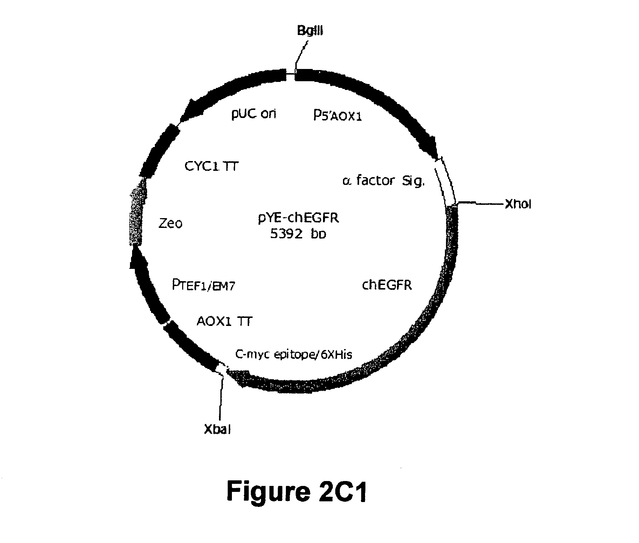

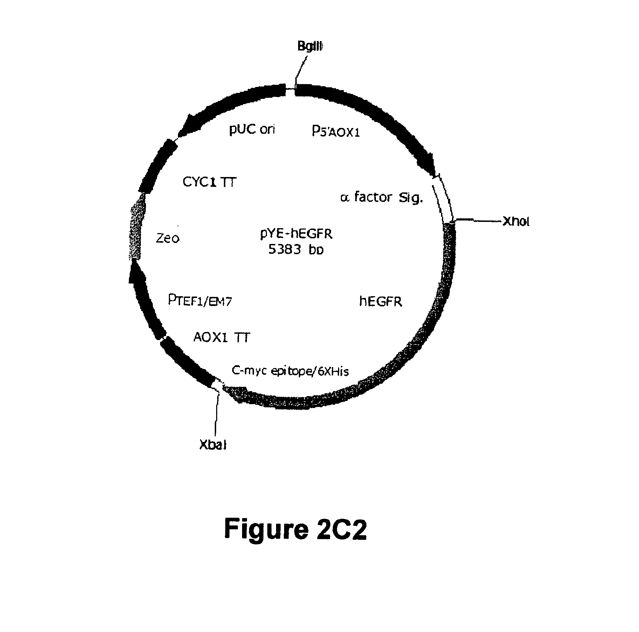

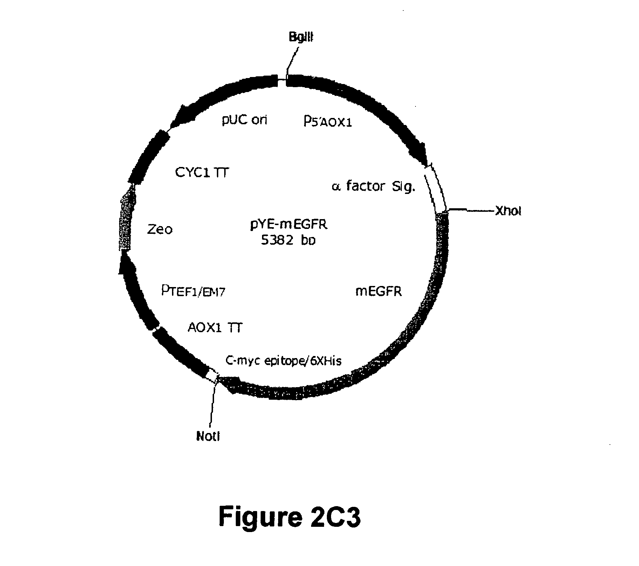

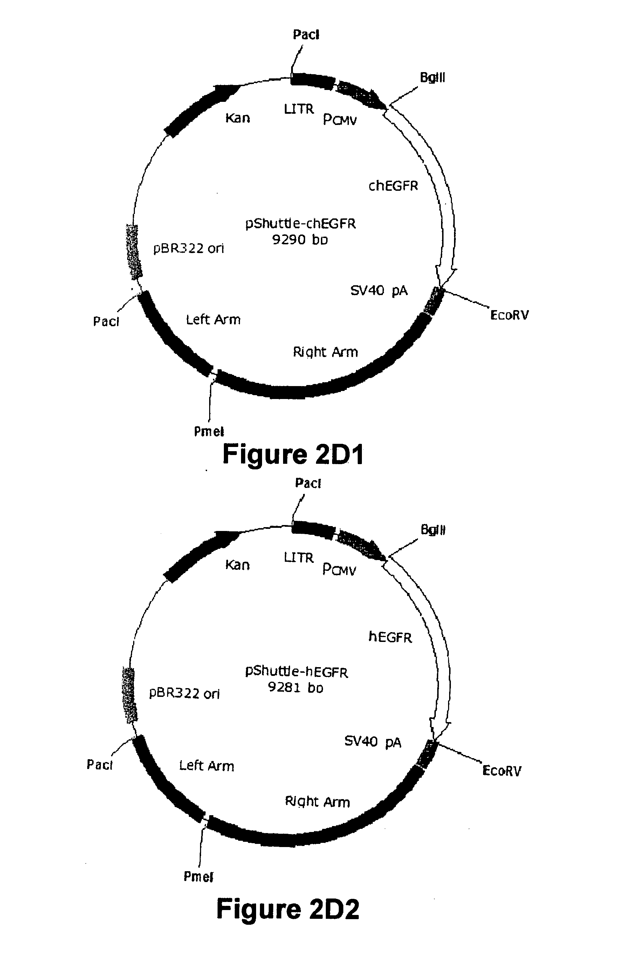

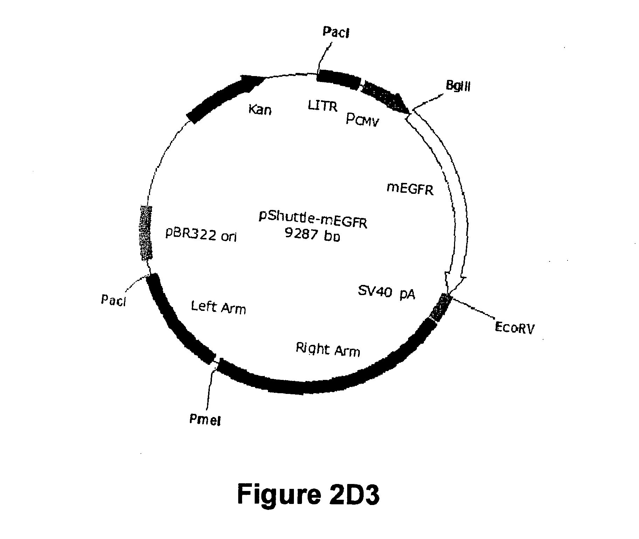

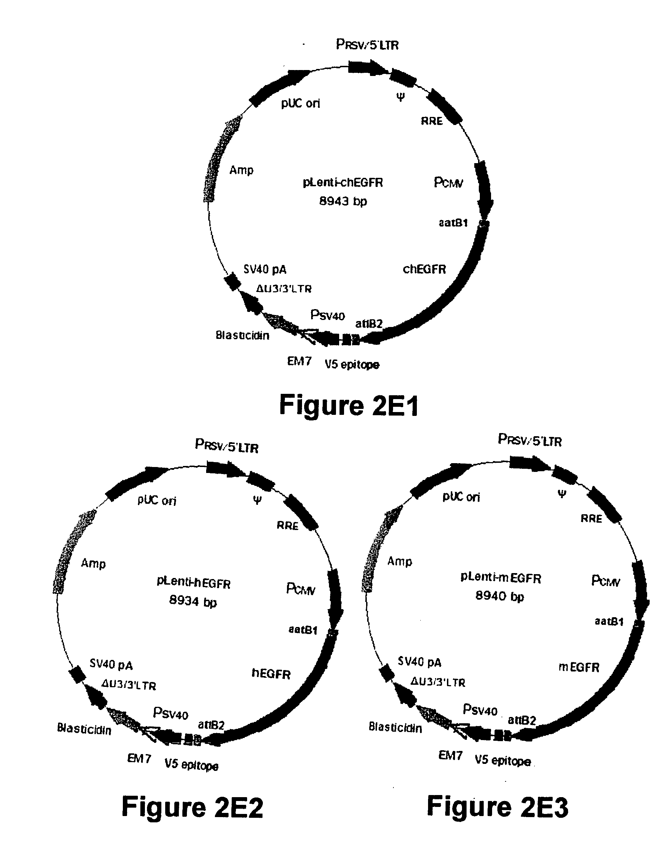

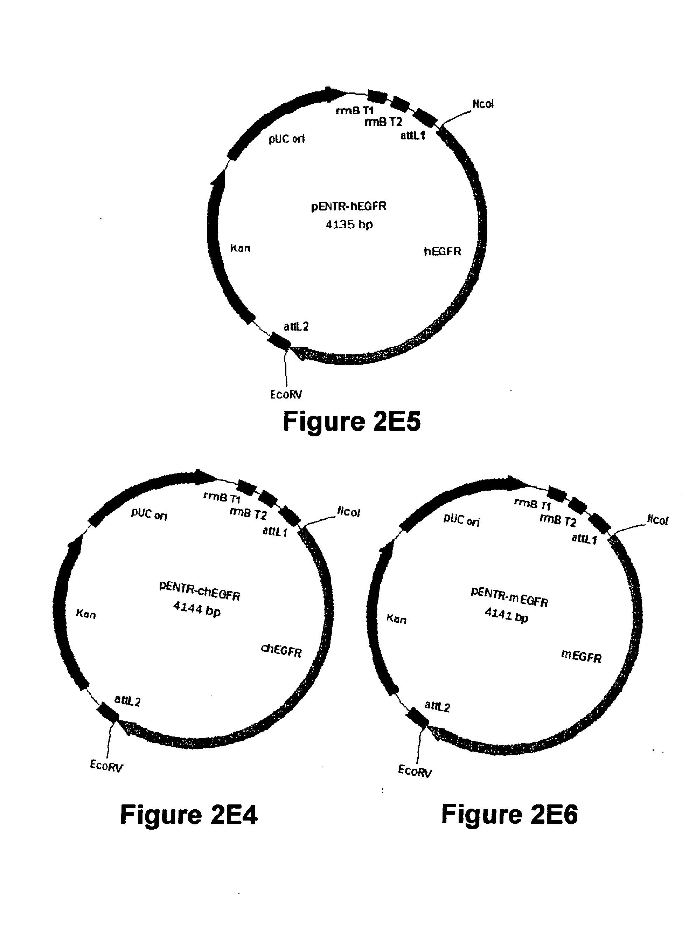

"Plasmids" are designated by a lower case "p" preceded and/or follow by capital letters and/or numbers. The starting plasmids herein are either commercially available, publicly available on an unrestricted basis, or can be constructed from available plasmids in accord with published procedures. In addition, equivalent plasmids to those described herein are known in the art and will be apparent to the ordinarily skilled artisan. In the present invention, some typical plasmids are constructed, as shown in Figure 2. These plasmids are one form of embodiments of the invention.

"Oligonucleotide" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Fragments" as used herein are a portion of a naturally occurring protein which can exist in at least two different conformations. Fragments can have the same or substantially the same amino acid sequence as the naturally occurring protein. "Substantially the same" means that an amino acid sequence is largely, but not entirely the same.

The term "variant" refers to "parent sequence or reference sequence" of the invention modified at one or more base pairs, codons, introns, exons or amino acid residues (respectively). Variants can be produced by any number of means including methods, for example, error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, GSSM, and any combination thereof.

For another aspect of the invention, said "daughter sequences or derivative sequences" can be taken as the "variants" of "parent sequences or reference sequences". Because they have some structural similarities with "parent sequences or reference sequences" in antigen, but also have some differences. Therefore, a material containing "daughter sequence or derivative sequence" may induce an immune response of the organism to the antigen, in particularly, may destroy the tolerance of the organism to the antigen that have already produced. Actually, it is indeed because that the material used in said vaccine of the invention has some similarity with the structure of the antigen, the immune response against the material in the vaccine of the invention, which is performed by the organism, may cross-react on the antigen. At the same time, since the material used in the vaccine of the invention has some differences with the structure of the antigen, the vaccine of the present invention is particularly useful in inducing a response of the organism against the antigen that has produced tolerance.

The method of the present invention is described by using EGFR molecular vaccine as an example. Actually, the present invention is not limited to EGFR molecular vaccine, and according to the present invention, a skilled person in the art can conveniently prepare other vaccines by using the method of the invention. The more important value of the invention is that it discloses a method for producing vaccines, especially a method that destroys the tolerance of an organism; especially it has important value in treatment of cancer.

The EGFR molecular vaccines are a variety of new biotechnological vaccines constructed by using homologous EGFR molecules derived from human or other heterogeneous biology that were reconstructed and evolved through modem biotechnological engineering or formed in biological evolution process as antigen, including recombined protein vaccine, recombined gene vaccine, recombined virus vaccine, genetically modified vaccine, and stably inverted symbiotic bacteria. These are cancer vaccines with novel concept.

A homologous EGFR molecule refers to the total designation of EGDR molecules from other species origins (such as, mice, rat, chicken, mackerel, drosphia, etc., see SEQ ID NO 1-14), which have some extent of similarities with human EGFR molecules at amino acid or nucleic acid level.

Human epidermal growth factor receptor (EGFR) is a trans-membrane, single chain, glycosylated protein with a molecular weight of 170KD. It consists of 1186 amino acid residues, and possesses Tyrosine Protein Kinase (PTK) activity. EGFR consists of 5 portions, including extracellular ligand-binding domain, transmembrane hydrophobic domain, juxtamembrane domain, PTK active site and receptor C-terminal peptide. EGFRs have various types of transcript means (See SEQ IN NO 1-5), and have various mutants, wherein the major mutant is type III EGFRvIII (also called ΔEGFR or de2-7EGFR, see SEQ ID NO 6), which is specific on the membrane of malignant tumor cell with highly tumor specificity. Human EGFR molecules are a type of gene family; other family members except for EGFR (also called c-erbB1/HER1) include c-erbB2/HER2, c-erbB3/HER3 and c-erbB4/HER4, which encoding membrane proteins p185erbB2, p160erbB3, p180erbB4 respectively. Although these members have highly homologous amino acid sequences and similar structural characteristics, their ligands are different. The target molecules of the cancer vaccine involved in the invention are EGFR (c-erbB1/HER1), excluding c-erbB2/HER2, c-erbB3/HER3 and c-erbB4/HER4.

The ligands of EGFR molecules, EGF or TGF, can act on EFGR through either autocrine pathway, or paracrine pathway, by activating PTK, and through a series of signal transduction, causing cells to divide and proliferate.

EFGR can be widely found on the surface of normal mammalian epithelial cell surface. On average, there are about 50-100 thousand receptor molecules per cell. Many cancer cells, such as lung cancer, breast cancer, ovary cancer, colon cancer, prostate cancer, bladder cancer, head and neck squamocarcinoma and glioma, all have over-expressed EGFR, up to 1-3×10⁶ molecules/cell. Take, as an example, in the case of lung cancer, the overexpression of EGFR is closely related to the cancer's infiltration, metastasis and prognosis. Logically, EGFR has been hailed as one of the ideal target molecules in cancer therapy. So far, however, the major application of using EGFR as a therapy target has been with monoclonal antibody and small molecular synthetic compounds. There has been some positive development in areas, such as using EGFRvIII molecule in tumor peptide vaccine, and anti-sense RNA gene therapy. Nonetheless, there have been few reports in literature regarding using EGFR as a target molecule in making anti-tumor molecular vaccine.

A tumor cell will produce one or more tumor antigens in its malignant convertion, proliferation process. However, in the most circumstances, the immunogenicity of the tumor cell is weak, and cannot induce an active immune response of the organism. From the viewpoint of immunology, a tumor cell is a "self' tissue cell in a host that can continuously express "normal" antigen (gene overexpression) and/or "abnormal" antigen (gene modification, mutation or deletion). Thus, the tumor antigen can be taken as a self-antigen. Under the normal conditions, the organism does not produce immune response to self-antigen, i.e., it presents an immune tolerance. In fact, self-antigen is the most relevant and abundant antigen, which needs to be tolerated by immune system of the host. The induction and maintenance tolerated by self-antigen is directed by various mechanisms, which can prevent the improper destruction to normal tissue. However, when organism sequestered antigen release occurs, or the change of self-antigen occurs due to biological, physical or chemical factors, it results in a self immune response, which leads to pathological damage and functional disturbance of the cells, tissues or organs where target antigens are located. Thus, if tumor cell sequestered self-antigen is released, or it performs certain changes, it is possible to induce a specific self immune response of the organism to the tumor cell; thus, the tumor is reduced or even extinct. As describe above, EGFR molecule overexpresses in various tumors, therefore, EGFR molecule can be taken as a tumor antigen and a self-antigen. Accordingly, various biotechnical modifications can be carried out on human EGFR antigen molecules (see SEQ ID NO 1-6), producing molecular vaccine, to induce anti-tumor immune response, and achieve an object of antitumorigenesis.

In a biological evolution process, there are large quantities of different extents of homological molecules between different species. For example, EGFR molecules are broadly present in biology, such as human, mouse, fruit flies, fishes, avians, and there are different extents of homologies between them. The homologies of human EGFR molecules and mice, chicken and drosphia EGFR molecules on amino acid level, are 90%, 64% and 32%, respectively. The slight difference formed by xenogeneic homological molecules in evolution process can be used to destroy immune tolerance, increase immunogenicity, induce self immune response of tumor cell, so as to achieve an object of antitumorigenesis. This is most likely that although the neutral mutation of homological gene in evolution process does not lead to a loss or change of its function, it may effect or change its means of immune response. When a xenogeneic homologous molecule is introduced into the body of a bearing subject, and expresses a corresponding xenogeneic homologous protein, the subject will recognize it as a foreign antigen, and will produce a corresponding antibody or cytotoxic lymphocyte to remove it. At the same time, since the expressed xenogeneic homologous protein has some extents of differences with corresponding protein molecule in the body of subject, it will produce non-specific cross-reaction to induce self-immune response, and destroy the immune tolerance of the organism to this protein.

As described above, the EGFR of human has different homologies with mice, chickens and fruit flies. The human EGFR has homology of 90% with mouse EGFR, 64% with chicken EGFR, and 32% with fruit flies EGFR analyzed by BLAST at the amino acid level. We use the immune cross-reactions produced by xenogeneic homologous EGFR moleculars to illicit self-immune reaction to break the tolerance of self-tumor cells. The result antibody competitively inhibits the combination of EGFR and its ligand, EGF or TGF- can inhibit the growth of tumor cells. In addition, the result can be obtained by the induced CTL.

Recent development in modem biotechnology provides a variety of techniques, such as error-prone PCR technology, DNA Shuffling, Phage Display technique, etc. to support EFGR molecular vaccine made by gene-directed evolution technique.

The invention relates to an anti-tumor EGFR molecular vaccine, including autologous EGFR vaccine, xenogeneic EGFR vaccine and directed evolution EGFR vaccine.

The invention relates mainly to a novel tumor vaccine---EGFR molecular vaccine with the xenogeneic homologous EGFR molecule origined from human or other animals as antigen, which is made either with modem genetic engineering, or derived from the natural evolutionary process. The vaccines of the present invention include protein vaccine, gene vaccine, virus vaccine, modified-gene vaccine and stable commensal bacteria. The EGFR molecular vaccine has effect to all the solid tumors wherein EGFR is over-expressed. The mechanism of anti-tumor effects is that EGFR molecule is an immune cross-reactive antigen, which can break the body's tolerance for self-EGFR molecule, and induce self cross-reactive immune response against the EGFR molecule. The anti-tumor EGFR molecular vaccine is also the nanoparticle agent which is made by the nanotechnology.

### 1. Origin of EGFR molecule, its selection and improvement

The term EGFR molecule as used herein includes autologous EGFR molecule, xenogeneic EGFR molecule, and gene-directed evolution EGFR molecule.

A key feature of the present invention is demonstrated in the use of xenogeneic homologous EGFR molecule as molecular antigen in anti-tumor immunotherapy. The EGFR molecular vaccine is derived through a natural evolutionary process, or made with modern genetic engineering.

EGFR is widely found in nature. It expresses in a variety of organisms ranging from mammals, such as human, mouse, to avian organism such birds and chickens, to lower organism such as nematode, and drosphia. The EGFR molecules from these different organisms have certain differences among them, and the homology among each other is from 30% to 100%. The homology between the xenogeneic EGFR molecules as disclosed in the present invention is from 30% to 95%. The exemplary species referred to are human, mice, chicken, and drosophila, and at the amino acid level, the homology of EGFR molecular of human with mice, chiken, and drosophila is, respectively, 90%, 64%, AND 32%. The human EGFR, mouse EGFR, chicken EGFR and drosophila EGFR molecules represent, respectively, the monitor points of homology related to the invention.

The EGFR molecule is immuno-tolerant by the host body, and thus its immunogenicity is very weak, if at all. By using a modem biotechnological engineering, autologous EGFR molecules can be modified with the procedure of gene directed evolution, and thus improved, thereby enhancing the immunogenicity of the EGFR antigen. The general technical protocol is using an error-prone PCR, random primer extension technique, and DNA shuffling technique to cause artificial mutation in autologous EGFR molecule and establish a gene mutation library. And then, a process of selection is conducted by using phage display technique, and ribosome display technique, to obtain EGFR molecules with stronger immunogenicity.

In addition, we can utilize the differences between the expression modification systems of bacteria, virus and other organisms, or further modify and improve the autologous EGFR molecules at the protein level to increase the immunogenicity of autologous EGFR.

### 2. EGFR Recombinant Gene Vaccine

Gene vaccine, also known as DNA vaccine, is a new type of vaccine based on nucleic acids made with modem molecular technology.

One of the EGFR molecular vaccines of the present invention is DNA vaccine. We search for sequences of EGFR disclosed in publicly available databanks, such as GenBank (including gene sequences, cDNA sequences, mRNA sequences and amino acid sequences). Using these sequences, we designed primers or probes, and using techniques such PCR, RT-PCR, molecular hybridization. From various commercial gene library, cDNA library, cell lines, tissue cultures, we cloned and isolated the intracellular domains of the EGFR molecules from a variety of different species. We have found that the extracellular domain of EGFR is the preferred active region causing immunogenicity. We can further use the gene directed evolution technique to select EGFR molecules with stronger immunogenicity. After confirming the sequences of the extracellular domain of the EGFR cDNAs using sequencing, we constructed Eukaryotic plasmid expression systems containing these intracellular domains of the EGFR using molecular cloning technique. These constructs are then transfected to CHO cell lines, and they are observed and tested for its EGFR expression and the level of expression. The recombinant EGFR constructs of these eukaryotic plasmid expression systems can be analyzed and confirmed using restriction enzyme digestion, SDS-PAGE and Western Blot. We used alkaline extraction to obtain confirmed recombinant EGFR expression plasmids, and then used ultracentrifuge, ultra-filtration methods to eliminate E.Coli endotoxins. After that, we got pure recombinant plasmid DNA. The plasmid DNA molecules can be used as DNA vaccine for immunization.

Fig. 2 shows some representative plasmids used to make the EGFR DNA molecular vaccine. The detailed process for constructing these plasmids is described briefly as follows: public database, such as GenBank, is used to obtain the cDNA sequences of EGFR molecules of human, mouse, chicken (which correspond respectively to SEQ ID NO 1-5, 7-9, 19). We designed the following primers based upon the cDNA sequences:

Human Primers: 5' GACCATGGAGGAAAAGAAAGTTTGC3',
5' ACGAATTCTTAGGACGGGATCTTAGGCCCA 3';

Mouse Primers: 5' GACCATGGAGGAAAAGAAAGTCTGC3',
5' ACGAATTCTTAATAGATGGTATCTTTGGC 3';

Chicken Primers: 5' GACCATGGAGGAGAAGAAAGTTTGTC3',
5' ACGAATTCTTAAGATGGAGTTTTGGAGCC 3'.

The total RNA from human lung cancer cell line A431, mouse lung cancer cell line LL2, and chick embryo are usedm, resoectively, to undertake RT-PCR amplification, and then collect and purify the amplified EGFR fragment (average 1.9 kb in length) using electrophoresis, and then subclone the PCR products. After confirming the sequences of the PCR subclones through sequencing, we digested them using Ncol and EcoRl, collected the 1.9 kb fragments and purifed them, and then inserted the fragments into the pORF-MCS vector (from the InvivoGen Corporation) which had been digested with Ncol and EcoRI, selecting recombiant plasmids. Candidate recombinant plasmids are confirmed by both restriction enzyme digestion analysis and PCR amplification. They are named pORF-hEGFR, pORF-mEGFR and pORF-chEGFR. For the construction of pcDNA-hEGFR, pcDNA-mEGFR and pcDNA-chEGFR, the process is similar to the above described process. The pcDNA3.1 (+) vector is made by InvitroGen, but the sequences of the PCR primers are varied somewhat from those primers as described in the above paragraph: They are, respectively:

Human Primers:5' GAGCTAGCATGGAGGAAAAGAAAGTTTGC3',
5' CACTCGAGTTAGGACGGGATCTTAGGCCCA 3';

Mouse Primers: 5' GAGCTAGCATGGAGGAAAAGAAAGTCTGC3',
5' CACTCGAGTTAATAGATGGTATCTTTGGC 3';

Chicken Primers: 5' GAGCTAGCATGGAGGAGAAGAAAGTTTGTC3',
5' CACTCGAGTTAAGATGGAGTTTTGGAGCC 3'.

Human EGFR gene's extracellular domain was used to construct and prepare the DNA vaccine for immunization of Lewis lung cancer model mouse. It was discovered that 8 weeks after immunization the survival rate of the mice injected with this EGFR DNA vaccine was 78%, significantly higher those mice injected with mouse EGFR DNA vaccine (with a survival rate of 25%), and much higher than comparision experienmental animals (with a survival rate of 10-15%). At the same time, we did not find any pathological changes to the lungs, liver, heart, and kidney of the experimental model mice. Further research was conducted and demonstrated that the induced self-autoimmune response in mice was mainly dependent upon CD4⁺ T lymphocytes. The CTL activity test did not find any target cells-specific cytocidal effect. Immunohistochemistry results showed deposition of autoantibodies in tumor tissues, but no such deposition in tumor tissues such as lung, liver, etc. The autoantibody in this case is mainly IgG.

The anti-sense RNA and RNAi molecules designed with EGFR molecule as template can be viewed as specific examples of the EGFR recombinant gene vaccine. This was not primarily by increasing the immunogenicity of EGFR molecule to induce anti-EGFR antibody and specific CTL reaction to achieve the goal of blocking the EGFR signal pathway, thereby further inducing tumor cell apoptosis, and inhibiting tumor cells growth and metastasis, but rather, it directly restricts and prohibits the expression of EGFR molecules at the DNA and RNA level.

### 3. EGFR Recombinant Protein Vaccine

Protein vaccine is a relatively traditional vaccine, but the protein possesses very good immunogenicity. One of the vaccines of the present invention is a recombinant protein vaccine, including those constructed with the recombinant proteins expressed by various expression systems, such as E.coli recombinant expression vectors, yeast recombinant expression vectors, baculovirus recombinant expression vectors.

Using molecular cloning technique, such as PCR, RT-PCR, molecular hybridization, we cloned and isolated the intracellular domains of the EGFR molecules of varieties of different species from various commercially available gene libraries, cDNA library, cell lines, and tissues, and further used the gene directed evolution technique to select EGFR molecules with strong immunogenicity. Then, the molecular cloning technique was used to construct prokaryotic plasmid expression systems, and transform suitable E.coli host with the result expression systems. Their EGFR expression levels were observed and examined. The recombinant EGFR constructs (plasmids) were analyzed and confirmed using restriction enzyme digestion, SDS-PAGE, and Western Blot. After confirmation, the recombinant EGFR molecules were used to transform E.coli, and the transformed E.coli cells were grown in a large quantity in order to produce recombinant proteins. Low temperature ultra centrifuge was used to collest the transformed cells, and then the cells were resuspended in PBS solution, and lysed using ultrasound techniques. The recombinant EGFR proteins were isolated and purified using ion-exchange chromatography and affinity chromatography. The isolated recombinant EGFR proteins can be used as protein vaccine for immunization.

Representative plasmids for recombinant EGFR protein vaccine expressed in E.coli are shown in Fig. 2B. The detailed process to construct these plasmids is described briefly as follows: the following PCR primers were designed in accordance with the cDNA sequences of EGFR molecules of human, mouse, and chicken described in the public databases, such GenBank, that correspond to SEQ ID NO 1-5,7-9,19, respectively.

Human Primers:5' GACCATGGAGGAAAAGAAAGTTTGC 3',
5' ACAGATCTAGG ACGGGATCTTAGGCCCA 3';

Mouse Primers: 5' GACCATGGAGGAAAAGAAAGTCTGC3',
5' ACAGATCTATAGATGGTATCTTTGGC 3';

Chicken Primers: 5' GACCATGGAGGAGAAGAAAGTTTGTC 3',
5' ACAGATCTAGATGGAGTTTTG GAGCC 3'

Respective pORF-hEGFR, pORF-mEGFR and pORF-chEGFR were used as templates for PCR amplification. Then, the amplified EGFR fragments (average 1.9kb in length) were collected and purified using electrophoresis, and then the PCR products were subcloned. After confirming the sequences of the PCR subclones, they were digested using Ncol and BglII to collect the 1.9 kb fragments, and purified, and then the fragments were inserted into the pQE60 vector (from the Qiagen Corporation) which had been digested with two restriction enzymes Ncol and BglII, and the recombiant plasmids were selected. Candidate recombinant plasmids were confirmed by both restriction enzyme digestion analysis and PCR amplification. They are named as pQE-hEGFR, pQE-mEGFR and pQE-chEGFR.

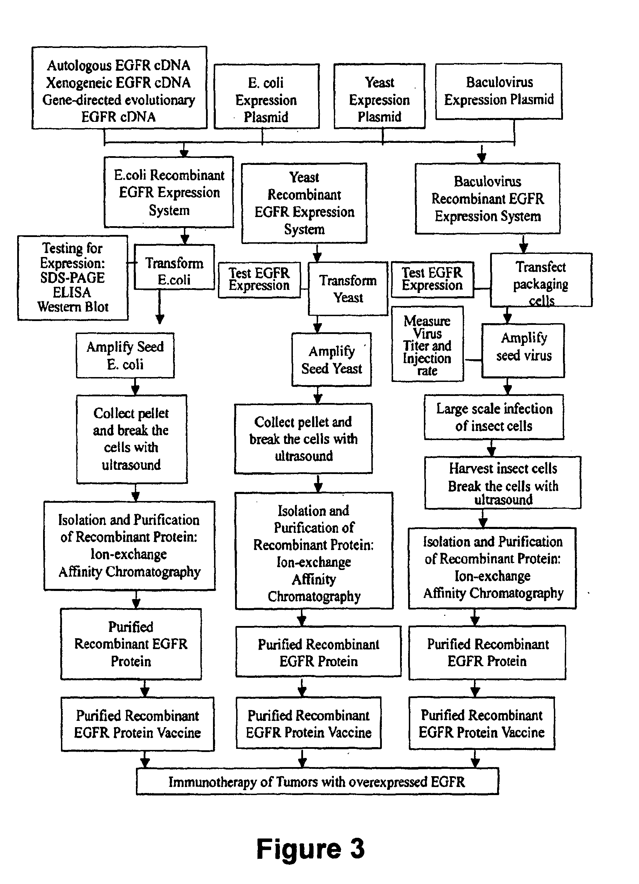

Recombinant EGFR proteins can be produced by methods other than the E.coli recombinant expression systems as described above, such as, using the yeast recombinant expression system, baculovirus recombinant expression system.
See Figure 3 for the protocol of the construction process of the EGFR recombinant protein vaccine.

Respresentative maps for plasmid expressing EGFR recombinant protein vaccine made from yeast expression system are shown in Fig. 2C. The detailed procedure is described as follows: the following PCR primers were desiged in accordance with the cDNA sequences of EGFR molecules of human, mouse, and chicken stated in the public databases, such GenBank, corresponding to SEQ ID NO 1-5, 7-9, 19, respectively.

Human Primers: 5' GACCATGGAGGAAAAGAAAGTTTGC 3',
5' ACAGATCTAGG ACGGGATCTTAGGCCCA 3';

Mouse Primers: 5' GACCATGGAGGAAAAGAAAGTCTGC3',
5' ACAGATCTATAGATGGTATCTTTGGC 3';

Chicken Primers: 5' GACCATGGAGGAGAAGAAAGTTTGTC 3',
5' ACAGATCTAGATGGAGTTTTG GAGCC 3'.

Respective pORF-hEGFR, pORF-mEGFR and pORF-chEGFR were used as templates for PCR amplification, and the amplified EGFR fragments (average 1.9kb in length) were collected and purifed using electrophoresis. And then the PCR products were subcloned. After confirming the sequences of the PCR subclones through sequencing, they were digested using two restriction enzymes Xhol and Xbal (in the case of plasmids containing mouse EGFR, Xhol and NotI double enzyme digestion were used), and the 1.9 kb fragments were collected and purified, and then inserted into pPICZ A vector (from Invitrogen Corporation) which had been digested with two restriction enzymes Xhol and Xbal (in the case of plasmids containing mouse EGFR, using Xhol and NotI double enzyme digestion). E.coli was transformed with these plasmid preparations, and the recombiant plasmids were selected. Candidate recombinant plasmids were confirmed by both restriction enzyme digestion analysis and PCR amplification. They are named as yeast expression plasmids: pYE-hEGFR, pYE-mEGFR and pYE-chEGFR. After digesting these yeast expression plasmids with Pmel to linerize them, an electric perforation method was used to transform yeast cell lines X33, or GS115. Zeocin resistance was used to select stable transformants. MMH (Minimal Methanol with histidine, MMH) and MDH (Minimal Dextrose with histidine, MDH) agar plate were used to determine and selecte Mut⁺ transformants. 6-10 Mut⁺ transformants were selected for small-scale expression, and then the methods of SDS-PAGE, Western Blot, ELISA, etc. were used to confirm the expressed recombinant protein. The Mut⁺ transformants with the highest expression efficiency were used as a large scale expression so as to establish yeast expressions seed libraries. Large flasks were used to culture or ferment the recombinant yeast cell lines, and the yeast pellets were collected using low temperature centrifuge. After the pellets were resuspended in PBS solution, ultrasound was used to break the cells. Then, ion exchange chromatography and affinity chromatography were used to isolate and purify the recombinant EGFR protein. The recombinant EGFR protein derived as such can be used as protein vaccine for immunization of subjects.

Similarly, yeast recombinant expression plasmids made with EGFR can be produced with other yeast expression systems.

Recombinant protein vaccine has a stronger effect than DNA vaccine in terms of the ability to illicit immune cross-reaction. It can stimulate production of high-titer anti-EGFR antibody and specific CTL activity to inhibit the growth and metastasis of tumor cells.

### 4. EGFR Recombinant Viral Vaccine

Recombinant viruses are also a good choice as a system for producing molecular vaccine. Molecular vaccines made with these recombinant viral expression systems include recombinant adenovirus vaccine, Lentivirus vaccine, adenovirus-related viral vaccine, retroviral viral vaccine, vaccinia virus vaccine and herpes simplex virus vaccine.

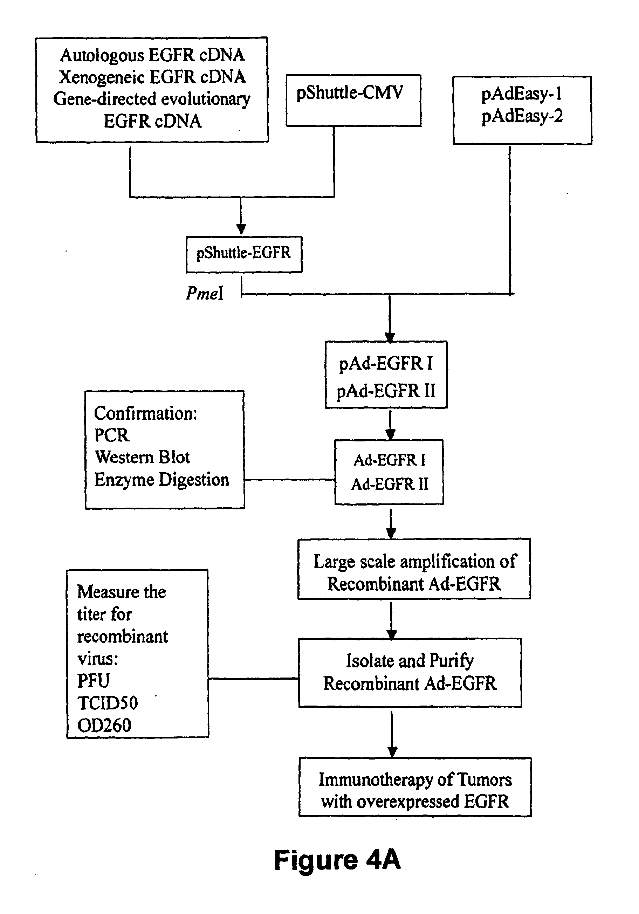

Currently, adenovirus vector is one of the most effective vectors in tumor gene therapy. It has the advantage of having high titer, safe, and can infect dividing, or non-dividing cells, and it does not integrate into the chromosomes of the host. In addition, adenovirus has relatively strong immunogenicity, which perhaps is a downside in gene therapy, but a big advantage in gene immunotherapy. As disclosed in the present invention, the recombinant adenovirus-derived EGFR recombinant viral vaccine is one of the most important embodiments. As described in the procedure above, we first cloned various kinds of autologous, xenogeneic or gene directed evolutionary EGFR cDNA, and then used these cDNAs to construct recombinant adenoviral expression vectors by molecular biological techniques. We then transfected 293 cells, and harvested the resulting recombinant adenovirus. The recombinant adenovirus was further confirmed using PCR, Western Blot, etc. We took the EGFR recombinant adenoviral vaccine made in large quantity using 293 cells, isolated and purified the recombinant virus using ultracentrifuge and ultra-filtration methods. The EGFR recombinant viral vaccine purified above can be used as vaccine to immunize subjects. See Figure 4A for the flow chart showing the construction of EGFR recombinant adenoviral vaccine.

We obtained the cDNA sequences of EGFR from human, mouse, and chicken stated in public databases, such GenBank, corresponding to SEQ ID NO 1-5,7-9,19, respectively. Based upon these sequences, we designed the following primers:

Human Primers: 5' GACCATGGAGGAAAAGAAAGTTTGC 3',
5' ACAGATCTAGG ACGGGATCTTAGGCCCA 3';

Mouse Primers: 5' GACCATGGAGGAAAAGAAAGTCTGC3',
5' ACAGATCTATAGATGGTATCTTTGGC 3';

Chicken Primers: 5' GACCATGGAGGAGAAGAAAGTTTGTC 3',
5' ACAGATCTAGATGGAGTTTTG GAGCC 3'.

Respective pORF-hEGFR, pORF-mEGFR and pORF-chEGFR were used as templates for PCR amplification, and the amplified EGFR fragments (average 1.9kb in length) were collected and purified using electrophoresis. And then the PCR products were subcloned. After confirming the sequences of the PCR subclones through sequencing, we digested them using two restriction enzymes BglII and EcoRV double enzyme digestion, collected the 1.9 kb fragments and purified them, and then inserted the fragments into pShuttle-CMV vector (made by Quantum Biotechnologies) which was pre-digested with BglII and EcoRV. We selected recombiant plasmids. Resulting candidate recombinant plasmids were confirmed by both restriction enzyme digestion analysis and PCR amplification. They are named as Adenovirus shuttle expression plasmids: pShuttle-hEGFR, pShuttle-mEGFR and pShuttle-chEGFR. See Fig. 2D for plasmid maps of these Adenovirus shuttle expression plasmids. We then took the various adenovirus shuttle expression plasmids as described before, digested them with Pmel enzyme, and co-transformed E.coli BJ5183 cells with backbone vector containing the adenovirus genome pAdEasy-1 or pAdEast-2. The resulting recombinant adenovirus vector plasmids are named as pAd-hEGFR, pAd-mEGFR and pAd-chEGFR. These adenovirus vector plasmids were digested with Pacl enzyme, and then using the Calcium-phosphate-DNA coprecipitation method, they were transfected into the adenovirus packaging cell line 293 cells. The resulting recombinant Adenoviruses are called Ad-hEGFR, Ad-mEGFR and Ad-chEGFR. PCR, Western blot and other methods were used to confirm that the EGFR gene had been indeed integrated into the adenovirus vector and efficiently expressed in Eukaryotic cells.

Depending upon the difference of the adenovirus genome, the recombinant adenovirus vaccine can be classified into two groups: the first group is called the generation I of EGFR recombinant adenovirus, resulting from the recombination between adenovirus shuttle expression plasmid pShuttle-EGFR and AdEasy-1 recombinant, thus named Ad-hEGFR I, Ad-mEGFR I and Ad-chEGFR I, and the second group is called the generation II of EGFR recombinant adenovirus, resulting from the recombination between adenovirus shuttle expression plasmid pShuttle-EGFR and AdEasy-2 recombinant, thus named Ad-hEGFR II, Ad-mEGFR II and Ad-chEGFR II.

In addition, the EGFR recombinant virus vaccine can be modified specifically to increase its targeting ability with the compound Mannan.

Because adenovirus can introduce genes effectively, EGFR recombinant viral vaccine made with adenovirus can effectively induce the anti-tumor immune response in the subject, thereby inhibiting the growth of tumors with over-expressed EGFR.

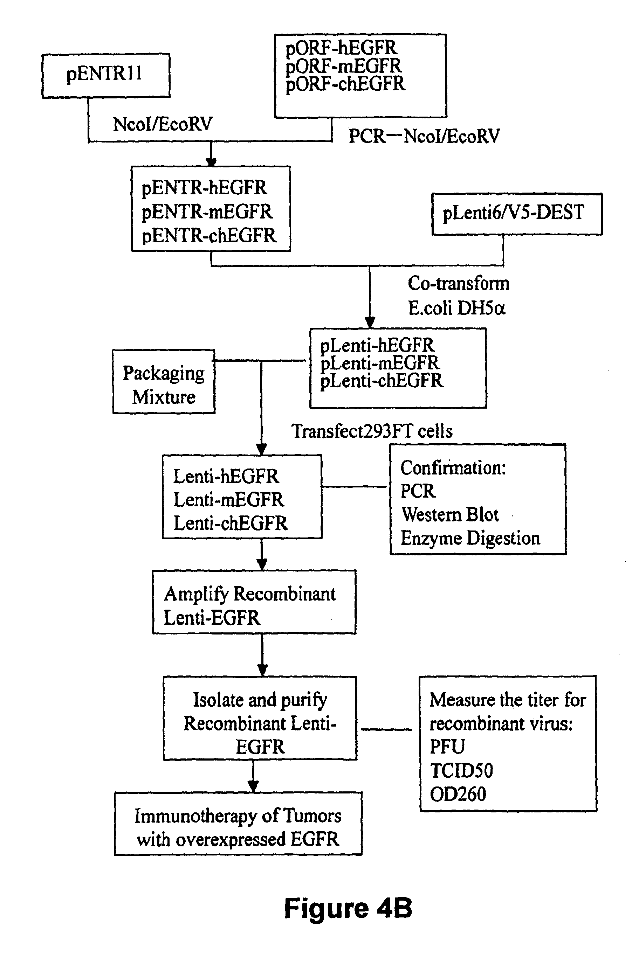

Lentivirus vector is a new generation gene therapy vector. It is originated from lentivirus which has a HIV-1 replication deficiency. It is different from the traditional reverse transcriptase virus vector originated from the Moloney Leukemia Virus (MoMLV). The difference is that Lentivirus vector can effectively transfect both dividing and non-dividing mammalian cells, and it has good biological safety. In addition, Lentivirus vector differs from Adenovirus vector in that Lentivirus vector can integrate into the host cell's chromosomes, and thus enable the introduced exogenous gene's stable and long-term expression. Recombinant Lentivirus vector vaccine is also an important variation of EGFR recombinant virus vaccines. The process to make EGFR Recombinant Lentivirus Vaccine is similar to what is described with respect to adenovirus. That is, we first cloned various autologous, xenogeneic, or gene-directed evolution EGFR cDNA, and then using molecular biology technique, we constructed its recombinant Lentivirus expression vector, then transfected 293FT cells, resulting in recombinant Lentiviruses. The recombinant Lentiviruses were confirmed using PCR, Western Blot, etc. Further, after confirmation, we used 293FT cells to amplify large quantity of the EGFR Recombinant Lentivirus vaccine, and further isolated and purified the recombinant Lentivirus using ultracentrifuge and ultrafiltration. The purified EGFR Recombinant Lentivirus can be used as vaccine to immunized subjects. See Fig. 4B for a flow chart of the construction of Recombinant Lentivirus vaccine.

We used the ViraPower™ Lentiviral Gateway® Expression Kit made by Invitrogen Corporation to produce EGFR recombinant Lentivirus. The detailed process is as follows: we first searched public databases, such as GenBank, to obatin cDNA sequences of EGFR molecule of human, mouse and chicken. Their sequences are listed as SEQ ID NO 1-5, 7-9, 19, respectively. We then designed the following PCR primers:

Human Primers: 5' GACCATGGAGGAAAAGAAAGTTTGC 3',
5' ACAGATCTAGG ACGGGATCTTAGGCCCA 3';

Mouse Primers: 5' GACCATGGAGGAAAAGAAAGTCTGC3',
5' ACAGATCTATAGATGGTATCTTTGGC 3';

Chicken Primers: 5' GACCATGGAGGAGAAGAAAGTTTGTC 3',
5' ACAGATCTAGATGGAGTTTTG GAGCC 3'.

We used pORF-hEGFR, pORF-mEGFR and pORF-chEGFR as templates for PCR amplification, then collected and purified the amplified EGFR fragments (average 1.9kb in length) using electrophoresis, and then subcloned the PCR products. After confirming the sequences of the PCR subclones through sequencing, we digested them using two restriction enzymes Ncol and EcoRV double enzyme digestion, collected the 1.9 kb fragments and purify them, and then inserted the fragments into pENTR11 vector (made by Invitrogen) which was pre-digested with Ncol and EcoRV. We selected recombiant plasmids. The resulting candidate recombinant plasmids were confirmed by both restriction enzyme digestion analysis and PCR amplification. They are named as pENTR-hEGFR, pENTR-mEGFR and pENTR-chEGFR. See Fig. 2E for plasmid maps of these Lentivirus expression plasmids. We then took the various Lentivirus expression plasmids as described before, transformed E.coli DH5 cells together with backbone vector containing the Lentivirus genome pLenti6/V5-DEST. The resulting recombinant Lentivirus vector plasmids are named as pLenti-hEGFR, pLenti-mEGFR and pLenti-chEGFR. See also Fig. 2E. We then mixed these Recombinant Lentivirus vector plasmids with packaging mixture (the ViraPower™ Packaging Mix), and then using the Calcium-phosphate-DNA coprecipitation method, the Recombinant Lentivirus vector plasmids were transfected into the Lentivirus packaging cell line 293FT cells. The resulting recombinant Lentivirus is called Lenti-hEGFR, Lenti-mEGFR and Lenti-chEGFR. PCR, Western blot and other methods were used to confirm that the EGFR gene had been indeed integrated into the Lentivirus vector, and that EGFR had been correctly, and efficiently expressed in Eukaryotic cells.

### 5. Modification and Improvement of EGFR Molecular Vaccine

What is described above is the basic procedure of preparing recombinant vaccine (including recombinant gene vaccine, recombinant protein vaccine and recombinant virus vaccine), and in the preferred embodiment, recombinant EGFR molecular vaccine. We now describe more embodiments hereinafter which include modifications and improvements that can enhance the efficency and specificity of the EGFR molecular vaccine.

### 1) Choice of adjuvant for the EGFR molecular vaccine

Suitable adjuvant is good carriers for vaccines that can increase the effective immune response of the vaccine. Different vaccines have different adjuvants. In the present invention, we used Freund's adjuvant and liposome for the EGFR DNA vaccine. For the EGFR recombinant protein vaccine, we mainly used aluminum adjuvant, and for the EGFR recombinant viral vaccine, generally, no adjuvant was used.

### 2) EGFR Gene-Modified Vaccine

Gene-modified vaccine refers to cell vaccine derived from human, other xenogeneic organism's cell lines transfected with EGFR molecule. These cell vaccines include vaccine made using various stably transformed tumor cell lines, vascular endothelial cells and dendritic cells. They also include vaccines made by using tumor cells or tissues infected by recombinant viruses. EGFR gene-modified vaccine is a particular example of EGFR molecular vaccine.

As discussed above in the present invention, EGFR is highly expressed in a variety of tumors, such as lung cancer, breast cancer, ovarian cancer, colorectal cancer, prostate cancer, stomach cancer, bladder cancer, head and neck squamocarcinoma, and glioma, etc. However, EGFR is tolerated in all these tumors, and thus cannot elicit immune response. Nonetheless, by using gene-modified vaccine, we can break the immune tolerance in these cells, and thus induce anti-EGFR immune response. We used Eukaryotic plasmids containing recombinant EGFR from a variety of different sources to transfect various kinds of tumor cell lines, such as lung cancer line A431, breast cancer cell line MCF7 etc., or vascular endothelial cells. After transfecting, we selected stable transfectants containing stably expressed EGFR molecules. We harvested the transfectants, and generated cellular vaccines using paraformaldehyde fixation method. In addition, we can also use various EGFR recombinant viral vaccines from different sources to infect tumor cell lines, tumor tissue, and tumor vascular endothelial cells, etc. We then can generate cellular vaccine using the same paraformaldehyde fixation method.

### 3) Stable EGFR transformants of commensal bacteria

EGFR molecular vaccines also include live vaccine, which is the stable EGFR transformants of commensal bacteria.

To produce these live vaccines, we used prokaryotic recombinant expression plasmids constructed with EGFR from various different sources, such as autologous, xenogeneic, and gene-directed evolutionary EGFR. We used these plasmids to transform intestinal commensal bacteria, such as Bifidobacterium. We then selected stable transformants. These stable EGFR transformants of commensal bacteria can continuously secrete exogenous EGFR molecules, thereby inducing the body's immune response. Thus, they are categorized as live vaccine. It is a particular example of the EGFR molecular vaccine.

### 4) Targeted Nano particles for EGFR molecular vaccine

The pure recombinant EGFR molecular vaccine (including EGFR recombinant gene vaccine, recombinant protein vaccine and recombinant viral vaccine) still should improve to enhance its specificity and immunogenicity. The present invention discloses the use of nanotechnology to enhance and modify the EGFR molecular vaccine, by using targeted nanoparticles in making the EGFR molecular vaccine. Using EGFR molecules, either its DNA or protein form, as target antigen molecule, biological molecules such as liposomes, degradable polymer biological materials, such as poly DL-lactide-co-glycolide polymer (PLGA) as nano materials, MIP-3 and Flt3-L as modifying genes, dendritic cells as target cells, we can construct a new type of targeted EGFR vaccine nanoparticles.

The EGFR molecular vaccine targeted with nanoparticles in the present invention come in a variety of forms, including: (1) mannan-modified nanoparticle, including mannan-modified recombinant adenoviral EGFR vaccine and protein vaccine, mannan-modified liposome recombinant EGFR gene vaccine and protein vaccine, (2) gene-targeted nanoparticle, including gene-targeted nanoliposome recombinant EGFR vaccine, gene-targeted nano-PLGA recombinant EGFR vaacine, and gene-targeted recombinant adenoviral EGFR vaccine, which express EGFR and MIP-3 simultaneously, (3) recombinant adenoviral EGFR vaccine targeting cancer vescular endothelial cell, including RGD-modified recombinant adenoviral EGFR vaccine).

As disclosed in the present invention, the diameter of the nanoparticles is generally less than 500nm, and they can be classified into three sizes: 200-500nm, 100-200nm, and 50-100nm. It is preferable to use nanoparticles with diameter ranging from 50nm to 100nm, and the nano peak value at around 80nm.

The following procedure applies to the preparation of Mannan-modified Recombinant Adenovirus EGFR vaccine: we first used the conventional method to obtain and amplify EGFR Recombinant Adenovirus (either Generation I or II), and then used chromatography or ultracentrifuge to purify the recombinant adenovirus. Next, we dissolved 70mg Mannan from Sigma into 5ml 0.1M phosphate buffer (pH6.0) to reach a final concentration of 14mg/ml, and then added 45ml 0.01 M Sodium Periodate solution, and mixed and oxidized it at 4□ for 60 minutes. After that, we added 10µl glycol, and incubated for 30 minutes at 4□, resulting in Oxidative Mannan (Ox-M) mixture. We then loaded the Ox-M mixture onto Sephadex-G25 columns previously balanced with bicarbonate buffer (pH6.0-9.0) and performed chromatography, with Ox-M being eluted into 2ml sized empty vessel. After that, we mixed the purified Ox-M with 1×10¹⁴ recombinant adenovirus particles at room temperature overnight, obtaining the needed Ox-M-Adenovirus. We then added 1mg/ml Sodium Borohydride to the Ox-M-Adenovirus, left it at the room temperature for 3 hours, forming Reductive Mannan Adenovirus (Red-M-Adenovirus). Both Ox-M-Adenovirus and Red-M-Adenovirus are desalted by ultrafiltration, and condensed, filtering out bacteria. They were then stored in small test tubes, and preserved at -800. The Mannan-modified Recombinant EGFR Adenovirus can be used as vaccine to immunize a subject.

As the size of the adenovirus is around 80nm, it is considered a natural nanoparticle. Adenovirus particles can be modified so that the adenoviral fiber protein can express the tri-peptide: RGD. The RGD tripeptide has specific ability to target tumor vascular endothelial cells. Recombinant Adenovirus EGFR vaccine modified with RGD can be viewed as a natural targeted EGFR molecular vaccine nanoparticle.

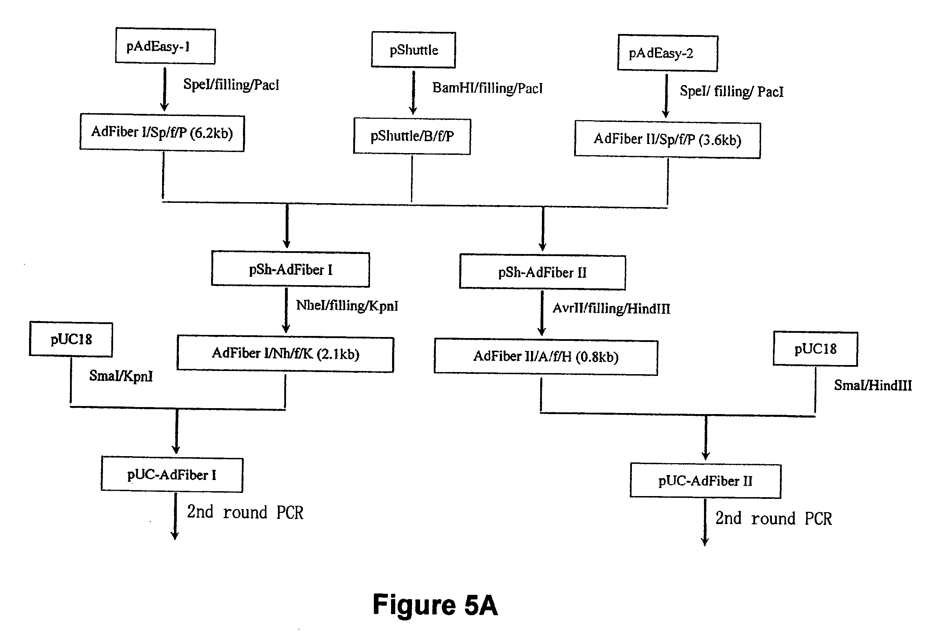

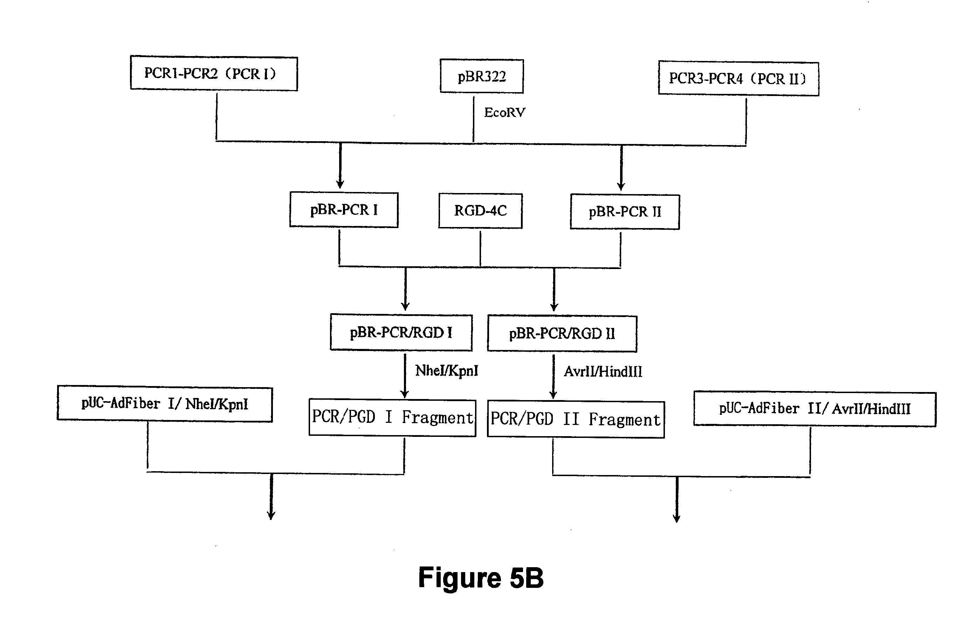

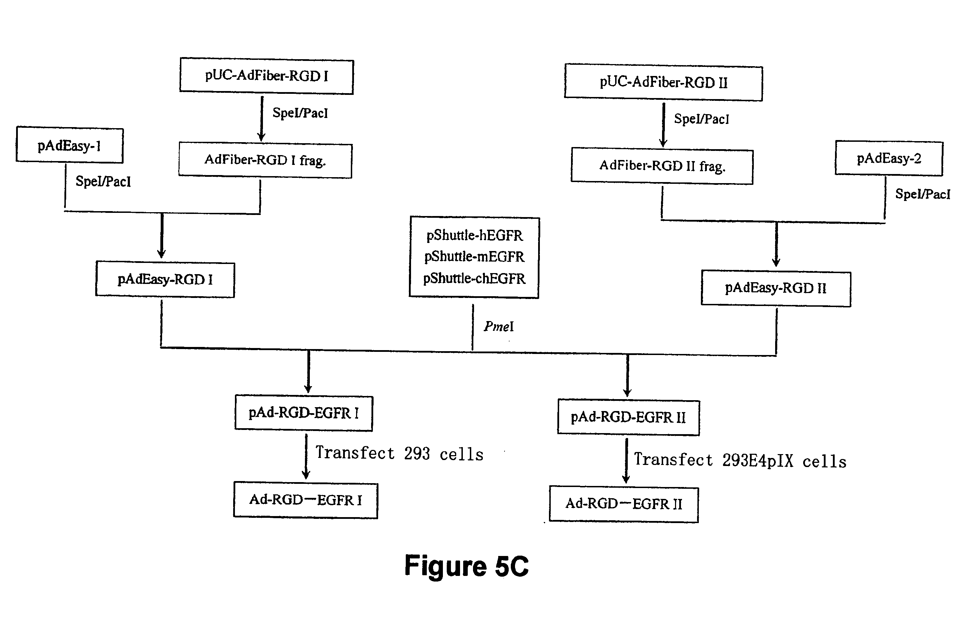

In the present invention, the AdEasy system is utilized to construct the RDG modified Adenovirus Recombinant EGFR vaccine. The detailed process is shown step by step in Figure 5. The detailed process is as follows: we digested the Adenovirus genome backbone plasmid pAdEasy-1 and pAdEasy-2 with restriction enzyme Spel (Sp), and then used T4 DNA Polymerase to fill in the ends (filling, f) so as to make them blunt, and then digested the filled-in product with PacI (P), and recovered the 6211bp and 3579bp fragments using electrophoresis, and named them as AdFiber I/Sp/f/P and AdFiber II/Sp/f/P, respectively. These fragments contain the intact Adenovirus fiber protein gene. Separately, the pSuttle vector was prepared by digesting it with BamHI first, and then filling in with T4 DNA Polymerase, and then digested with PacI. After such BamHI/filling /PacI-digestion treatment, the vector is ready to be inserted with the AdFiber I/Sp/f/P and AdFiber II/Sp/f/P fragments. The resulting recombinant plasmids are named pSh-AdFiber I and pSh-AdFiber II, respectively. We then digested pSh-AdFiber I with Nhel enzyme, filled in with T4 DNA polymerase, and digested them again with Kpnl enzyme (NheI/filling/KpnI), recovered, using electrophoresis, the 2090 bp fragment called AdFiber I/Nh/f/K; inserted this fragment into a pUC18 vector which had been pre-digested with Smal and Kpnl double enzyme digestion, resulting in the recombinant plasmid named pUC-AdFiber I. On the other hand, pSh-AdFiber II was digested with AvrII enzyme, then filled in with T4 DNA polyerase, and then digested with HindIII (AvrII/filling/HindIII), using electrophoresis, to recover the 838 bp fragment, called AdFiber I/A/f/H. This fragment was inserted into a pUC18 vector which had been previously digested with Smal and HindIII double enzyme digestion, resulting in new plasmids named pUC-AdFiber II. Then, we designed a series of PCR primers so as to use pUC-AdFiber I and pUC-AdFiber II as templates to amplify the Adenovirus knob, (Ad-knob) gene sequences. The primers used are, respectively:

F1 (5'GAAAGCTAGC CCTGCAAACATCA 3'),

R1 (5'ACTCCCGGGAGTTGTGTCTCCTGTTTCCTG3'),

F2 (5'ACTCCCGGGAGTGC ATACTCTATGTCA 3'),

R2 (5'TATGGTAC CGGGAGGTGGTGA 3'),

F3 (5'AACCTAGGGAGGTTAACCTAAGCACTG 3'), and

R3 (5'CTCAAGCTTTTTGG AATTGTTTGA 3').

Using primers F1-R1, F2-R2, F3-R1 and F2-R3, respectively, for the first round PCR, we obtained products PCR1, PCR2, PCR3 and PCR4. Again, using F1-R2 and F3-R3 as primers, and using the amplified products from the first round, PCR1 and PCR2, PCR3 and PCR4 as templates, we undertook the second round of PCR amplification, resulting in PCR products PCR1-PCR2 (PCR I), PCR3-PCR4 (PCR II). We took the PCR I and PCR II from the amplification in the second round, inserted them into pBR322 vector that had been previously cut with EcoRV. The resulting recombinant plasmids are named pBR-PCR I and pBR-PCR II. The sequence of the RGD-4C duplex is as follows:

5’TGTGACTGCCGCGGAGACTGTTTCTGC 3’

3'ACACTGACGGCGCCTCTGACAAAGACG 5'

We inserted the RGD-4C into the pBR-PCR I and pBR-PCR II vectors, where the vectors were previously digested with Smal. The resulting recombinant plasmids are named pBR-PCR/RGD I and pBR-PCR/RGD II. The sequences of the recombinant plasmids are confirmed using sequencing. Cut pBR-PCR/RGD I with NheI/KpnI double digestion , using electrophoresis, recovered the PCR/RGD I fragment, and inserted the fragment into the pUC-AdFiber I vector which had been double digested using NheI/KpnI. The resulting recombinant plasmids are called pUC-AdFiber-RGD I. Similarly, we used AvrII/HindIII to double digest pBR-PCR/RGD II, and using electrophoresis, recovered the PCR/RGD II fragment, and again inserted the fragment into pUC-AdFiber II vectors previously digested with double enzume AvrII/HindIII. The resulting plasmid is named pUC-AdFiber-RGD II. Afterwards, we used Spel/Pacl double enzyme to digest pUC-AdFiber-RGD I and pUC-AdFiber -RGD II vector, used electrophoresis to recover the AdFiber-RGD I and AdFiber-RGD II fragments, and then inserted the fragment into pAdEasy-1, pAdEasy-2 vectors both of which were previously digested with SpeI/PacI. The resulting recombinant plasmids are called pAdEasy-RGD I, and pAdEasy-RGD II, respectively. Then, we first took Adenovirus shuttle expression plasmids pShuttle-hEGFR, pShuttle-mEGFR and pShuttle-chEGFR as described above, and linerized them with Pmel. After that, we co-transformed the E.coli BJ5183 cells with these Shuttle-EGFR plasmids together with pAdEasy-RGD I, and pAdEasy-RGD II, respectively. The resulting recombinant plasmids are named Adenovirus plasmids pAd-RGD-EGFR I, and pAd-RGD-EGFR II. We used the Adenovirus plasmid pAd-RGD-EGFR I to transfect 293 cells, resulting in recombinant Adenovirus named Ad-RGD-EGFR I. Similarly, we used the Adenovirus plasmid pAd-RGD-EGFR II to transfect 293E4pIX cells, resulting in recombinant Adenovirus named Ad-RGD-EGFR II. After purification, Ad-RGD-EGFR I and Ad-RGD-EGFR II can be used as vaccine to immunize subjects, and more importantly, these vaccines possess specificity targeting tumor vascular endothelial cells.

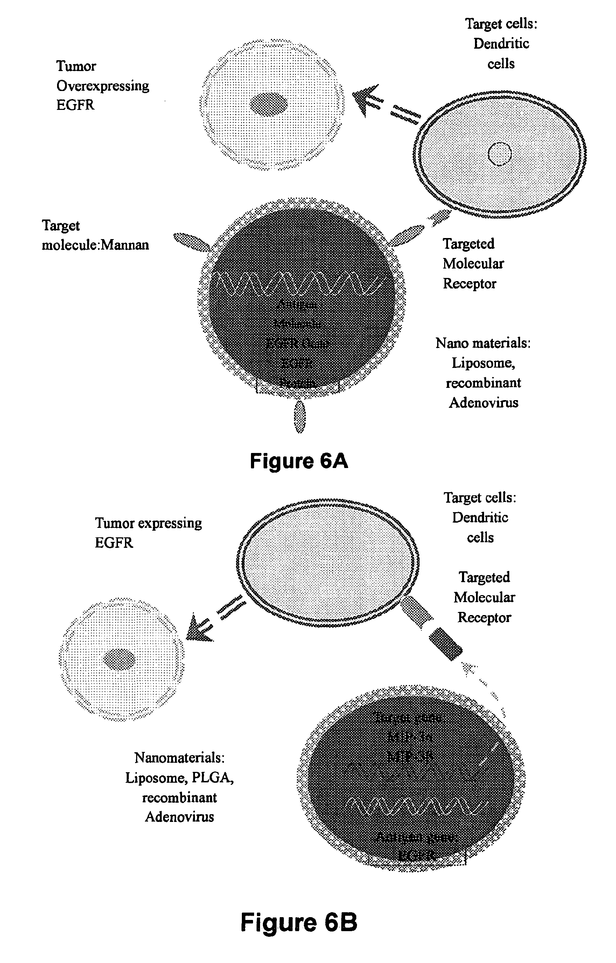

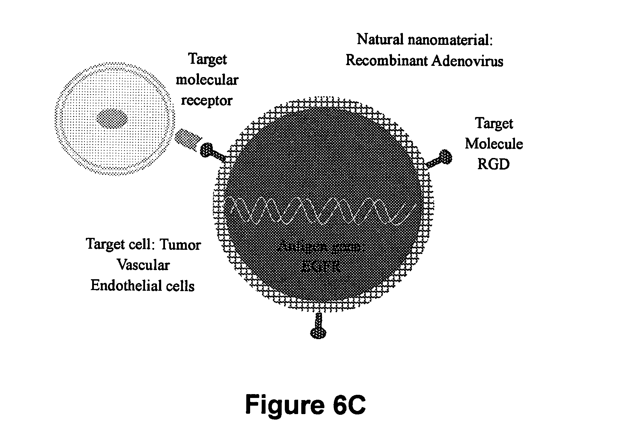

It is found in the present invention that EGFR molecular vaccine targeted with nanoparticles can increase the immunogenicity of EGFR greatly compared to a normal EGFR molecular vaccine, and thus enable EGFR to illicit even stronger anti-tumor immune response. See Fig. 6 for schematic description of the mechanism of action for EGFR molecular vaccine targeted with nanoparticle.

### 5) Combination of EGFR molecular vaccine with other immune response stimulating factors

The anti-tumor effect of EGFR molecular vaccine can be enhanced by the combing effects of other immune response stimulating factors. These factors include cytokines, such as IL-2, TNF, IFN- , and GM-CSF, etc., chemokines, such as MIP3, MIP3, and IP10, etc., stringent factors, such as CEA, HSP70, HSP90, etc., and various immune co-stimulating factors, such as B7, etc. The combinatorial effects of these immune response stimulating factors with EGFR molecular vaccine can realize their effects through gene fusion at the gene level, or fusion protein at the protein level. They can also act through co-transfecting tumor cells, dendritic cells, and vascular endothelia cells at the cellular level.

### 6. The anti-tumor effect of EGFR Molecular Vaccine

As discussed above, the present invention provides a new type of molecular vaccine using tumor associated proteins. As in the specific embodiment of EGFR molecular vaccine, it encompasses protein vaccine, gene vaccine, viral vaccine, and gene-modified vaccine, all made using autologous, xenogeneic, or gene directed evolutionary EGFR molecules. These molecular vaccines possess effective anti-tumor functions, including preventing tumor formation, inhibiting tumor growth, anti-metastasis effect on tumor, and increasing the survival rate of human or animals carrying tumors.

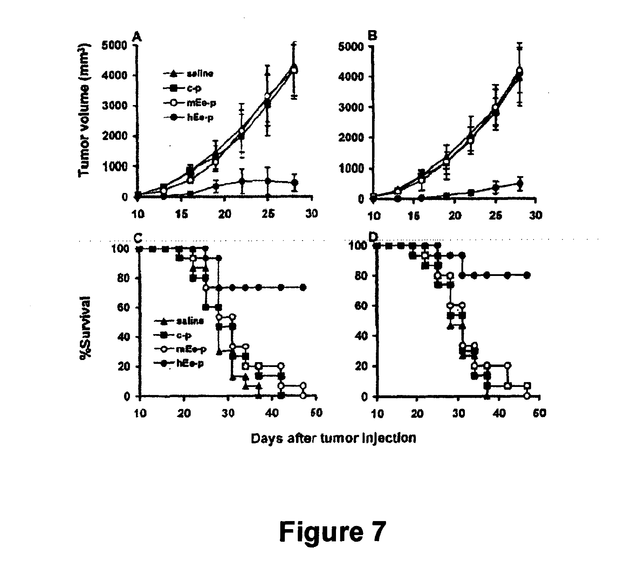

To observe the anti-tumor effect of EGFR recombinant DNA vaccine, mice were randomly divided into groups (15 mice per group), and each group was injected intramuscularly 100µg EGFR recombinant DNA vaccine, hEe-p, mEe-p, c-p (control plasmid), or Saline, respectively. The injection was done once a week for four weeks continuously. One week after the fourth immunization, immunized mice were each implanted with 5×10⁵ of LL/2c Lewis lunger cancer cells (Fig. 7A and C), or MA782/5S mammary cancer cells (Fig. 7B and D), respectively. It can be seen from the figures that tumors kept growing in mice immunized with mEe-p, c-p, or Saline, while mice immunized with hEe-p exhibited significant protective immunity. In addition, the survival rate of hEe-p immunized mice is significantly higher than those of the mice immunized with mEe-p, c-p, or saline. For example, mice immunized with hEe-p lived longer than 5 months. After 150 days of tumor implantation, the mice immunized with LL/2c Lewis lung cancer and with MA782/5S mammary cancer reached survial rate of 60% and 66%, respectively. It is also discovered that the protective immunity is dosage-dependent. That is, the immunity obtained with dosage of 150µg is similar to that of 100µg of the EGFR DNA vaccine, but there is almost no immunity when the dosage is lowered to 5-15µg. In addition, the mice bearing EGFR-negative tumors, such as H22 liver cancer, and MMT-06052 mammary cancer exhibited no protective immunity when immunized with hEe-p.

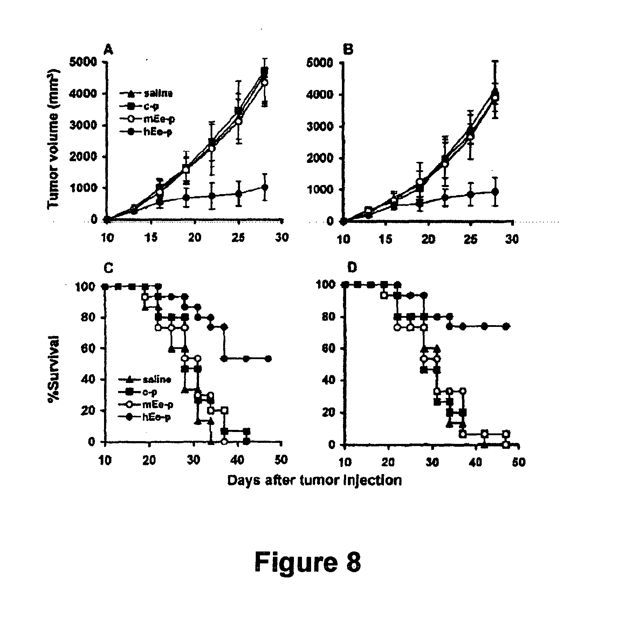

Other than the protective immunity, EGFR recombinant DNA vaccine also possesses therapeutic immunity (Fig. 8). Likewise, mice were randomly divided into groups (15 mice per group). They were each injected subcutaneously once a week for 4 continuous weeks with 1×10⁶ LL/2c Lewis Lung cancer cells (Fig. 8A and C), or MA782/5S mammary cancer cells (Fig. 8B and D). Five days later, they were injected intramuscularly 100µg each hEe-p, mEe-p, c-p, or saline once a week for 4 continuous weeks. As seen from the figures, tumors in the mice immunized with mEe-p, c-p, or saline continued to grow, while tumors in the mice immunized with hEe-p exhibited significant therapeutic effect. In addition, the survival rate of the mice immunized with hEe-p is significantly higher than those immunized with mEe-p, c-p, or saline. The mice immunized with hEe-p lived longer than 5 months. 150 days after tumor implantation, the mice carrying LL/2c Lewis cancer and MA782/5S mammary cancer have a survival rate of 40% and 53%, respectively.

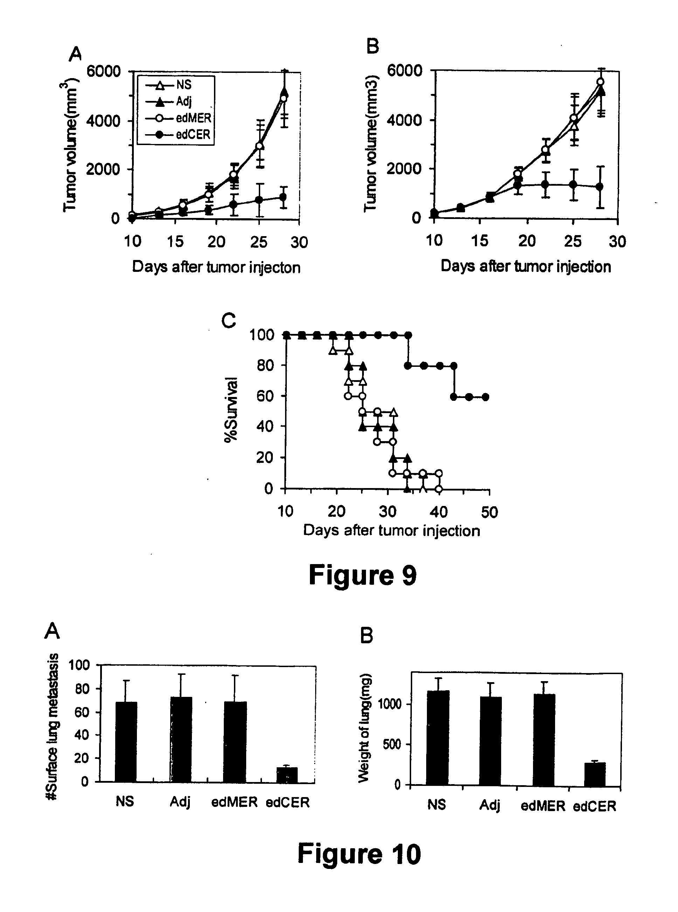

EGFR recombinant protein vaccine has a similar protective and therapeutic immunity. See Fig. 9. As described above, 6-8 weeks old female mice which carried C57BU6 or BALB/c were randomly divided into groups, and tumor models were established for LL/2c Lewis lung cancer, MA782/5S mammary cancer and C26 intestinal cancer in those mice. Tumor-bearing mice were injected subcutaneously EGFR recombinant protein vaccine in the amount of 5-50µg, or adjuvant aluminium hydroxyl gel and saline 100µl, once a week for 4 consecutive weeks. The mice immunized with recombinant chEGFR protein vaccine exhibited significant anti-tumor protective immunity and therapeutic immunity, and the growth of the LL/2c Lewis lung cancer and MA782/5S mammary cancer is inhibited, and the life span of the mice bearing these two tumors is extended, while the EGFR-native tumor C26 is not significantly affected. In addition, it can be seen that tumors kept growing at a fast pace in the control groups where the mice were immunized with recombinant mEGFR protein vaccine, alum adjuvant, or saline, and the life span of these mice were significantly shortened. Comparing to the control group, the tumor volume (t-test) and the length of survival (log-rank test) in the test groups showed significant difference (p<0.05). Fig. 9 shows the anti-tumor effect of EGFR recombinant protein vaccine in the mice bearing MA782/5S mammary cancer.

Metastasis is a common cause for tumor progression and for failure of chemothearpy and radiation therapy. The presence of tumor cells in blood and lymph circulation and the formation of microtransferer is a key to metastasis. It is disclosed in this invention that recombinant EGFR molecular vaccine has anti-metastasis effect on tumor. See Fig. 10. It is discovered during the therapy research of LL/2c Lewis lung cancer metastasis in animal model by injection in the tail vein, tumor-bearing mice immunized with chEGFR protein vaccine has far less metastasis in lung, or to a much lesser degree comparing to control group, whereas tumor-bearing mice in the control groups where they were immunized with recombinant mEGFR protein vaccine, alum adjuvant, or saline showed 100% metastasis, and they showed countless transferer. Figure 10 shows the anti-tumor metastasis effect of EGFR recombinant protein vaccine in mice bearing LL/2c Lewis lung cancer. From the figure, it can be seen that the number of transferer of LL/2c lung cancer and lung wet weight after LL/2c tumor metastasis in the mice immunized with chEGFR protein vaccine are both less comparing to control group.

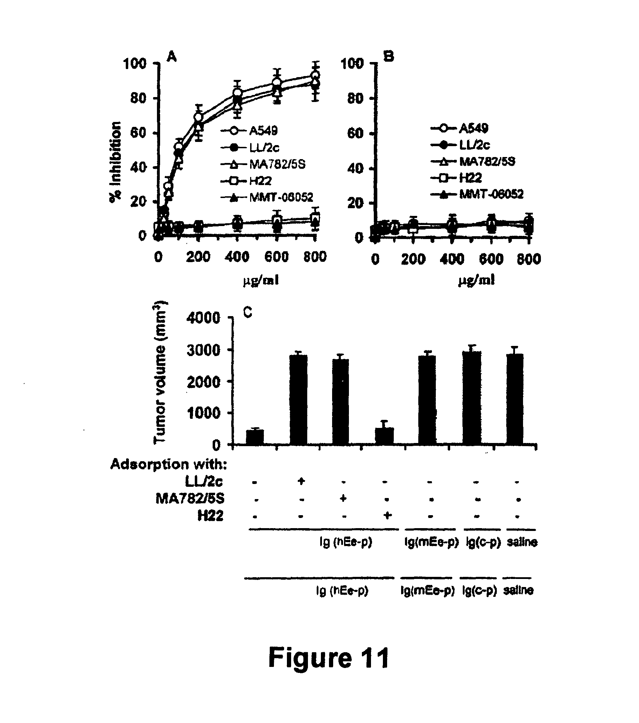

In addition, EGFR molecular vaccine can inhibit growth of tumor cells in vitro, and they possess adoptive anti-tumor immunity in vivo. See Fig. 11. The process is described as follows: mice were immunized with EGFR DNA vaccine, and sera were collected 7 days after the fourth immunization. Serum Immunoglobulin (Ig) was purified using affinity chromatography. Various conentration of Ig (1-1000 mg/ml) were added to 2×10⁵/ml EGFR-positive tumor cells (A549, LL/2c, MA782/5S) and EGFR-negative tumor cells (H22 and MMT-06052), both kinds of tumor cells being in log-growth stage. The cells and the Ig were then co-cultured for 72 hours, and live cells were examined using Tai Pan Blue method, and the rate of growth inhibition was calculated. The results showed that EGFR-positive tumor cells that were treated with Ig purified from the sera of mice immunized with human EGFR recombinant DNA vaccine hEe-p showed significant growth inhibition, whereas EGFR-negative tumor cells were not affected. See Fig. 11A. As a control, Ig derived from non-immunized normal mice was co-cultured with the corresponding tumor cells. It has no effect on either EGFR-positive tumor cells, or EGFR-negative tumor cells. See Fig. 11B.

These purified Ig originated from the immunized mice also possess adoptive immunity. The process to study adoptive immunity is as follows: nude mice were injected subcutanously 1×10⁵-1×10⁶ tumor cells. One day later, purified Igs were intravenously injected in the dosage of 10-300mg/kg twice a week for three consecutive weeks. Results showed that the adoptive transfer of human EGFR recombinant DNA vaccine hEe-p has significant tumor suppression effect. As a comparison, purified Ig were incubated at 4□ with fixed EGFR-positive tumor cells or EGFR-native tumor cells for one hour of shaking and mixing in order for the tumor cells to adsorb the Ig. This process was repeated four times. Results showed that Ig from the mice immunized with human EGFR DNA vaccine hEe-p were pre-adsorbed by the EGFR-positive tumor cells, such as LL/2c and MA782/5S, and thus lost their anti-tumor effect, while the anti-tumor effect of the Ig still remained after incubating with EGFR-negative tumor cells, such as H22 (Fig. 11C).

Besides the lung cancer and mammary gland cancer as described above, the tumors that can be affected include other solid tumors wherein EGFR is over-expressed, including ovarian cancer, colorectal cancer, prostate cancer, stomach cancer, bladder cancer, head and neck squamocarcinoma and glioma. EGFR molecular vaccine has reliable anti-tumor effect on all cancers mentioned above.

### 7. Principle of the effect and biological safety of the EGFR molecular vaccine

It is well known in modern immunology that the mutual recognition between an antigen or antigenic epitope and its corresponding receptor/antibody is not merely specific. In fact, there is a certain degree of plasticity, promiscuity, and degeneracy in this recognition. Thus, it is possible to induce immune cross-reaction against a self-antigen through the mechanism of molecular mimicry in order to break the immuno-tolerance for the self-antigen.

As discussed above, the present invention discloses the discovery that tumor specific protein, such as EGFR molecule, is an immune cross-reactive antigen. Certain variation of the EGFR molecule can break the body's tolerance for self-EGFR molecule, and induce self cross-reactive immune response against the EGFR molecule. These immune response against EGFR include active immune response (such as cellular immune response and humoral immune response), and passive immune response (such as adoptive immune response).

In order to study the principle or mechanism of the anti-tumor function of EGFR molecular vaccine, we first undertook an immune testing for humoral liquid from EGFR immunized mice. The testing used included flow cytometry, Western Blot, immunoprecipitation in order to determine the existence of anti-EGFR autoantibody in mouse and rabbit serum. We used ELISA to test for the titer and type of anti-EGFR antibody in mouse/rabbit immunized sera, and we used immunohistochemistry to test the autoantibody in the tumor tissue of immunized mice. We used tumor cell aggregation test, and serum adoptive immune test for examining the funtion of autoantibody.

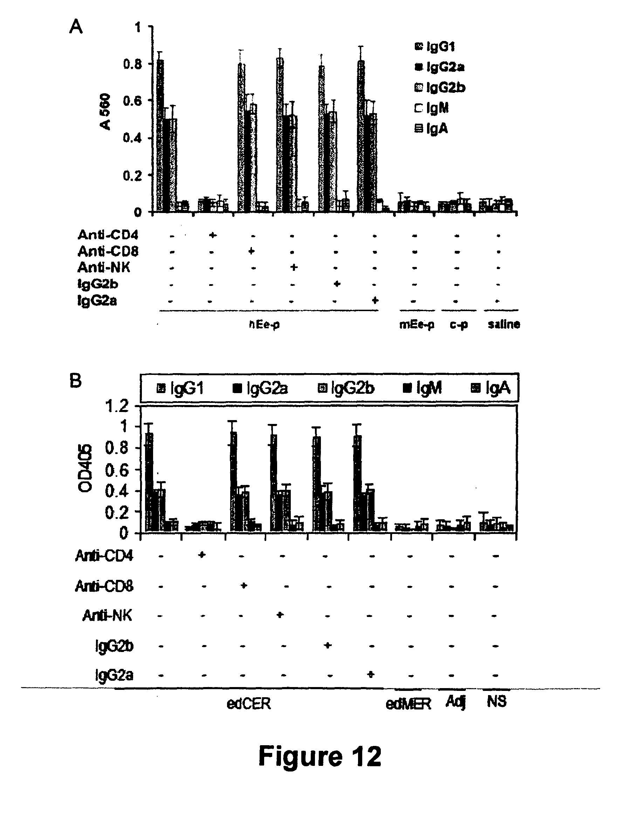

The following experimental protocols were undertaken. Blood was taken from each mouse before and after immunization every week using extraction of blood sample from the mouse tail vein, or after the mouse was sacraficed, sera were collected for use in the next step. Western Blot showed that antibodies induced by recombinant EGFR molecular vaccine could recognize specifically the corresponding antigen (recombinant EGFR protein or EGFR expression in tumor cells), but they could not recognize EGFR-negative cells. At the same time, flow cytometry was used to identify the antibody stereo surface recognition and we found that recombinant EGFR molecular vaccine could stimulate the production of specific antibodies which could in turn recognize the EGFR on the surface of tumor cells. Testing for autoantibodies using ELISA showed that the mice started to produce anti-mouse autoantibody two weeks after being immunized with recombinant EGFR molecular vaccine, reaching a titer of 1:100 to 1:5000. It gradualy increased to 1:10000 to 1:500000 at week 4, and could maintain a titer of 1:500 to 1:1000 at week 8. As a comparison, the corresponding control group had no detectable antibody production. Further testing for the subtype of the resultant antibody showed that antibody produced by EGFR molecular vaccine was mainly IgG. Fig. 12 shows the types of antibodies produced by the mice immunized with EGFR molecular vaccine (both recombinant DNA vaccine and recombinant Protein vaccine). From the figure, it can be seen that recombinant EGFR molecular vaccine can increase significantly the production of IgG1, IgG2a, and IgG2b, but that the production of IgM and IgA is not increased. Moreover, the production of the antibodies stimulated by recombinant EGFR molecular vaccine can be blocked by anti-CD4 antibody, but not by anti-CD8, anti-NK, or control antibodies. As comparison, no specific antibodies were detected in the control groups of protein vaccine, adjuvant, or saline.

EGFR molecular vaccine can also induce cellular immunity. The tests used to examine cellular immunity in the mice immunized with EGFR molecular vaccine include: using ⁵¹Cr release method to measure CTL activities; using ELISPOT to test cytokine level (mainly for testing serum concentration of IFN-γ, and IL-4); using depletion of immune cell subsets tests to determine T cell types (CD4⁺ T lymphocyte, CD8⁺ T lymphocyte, or NK cells, etc).

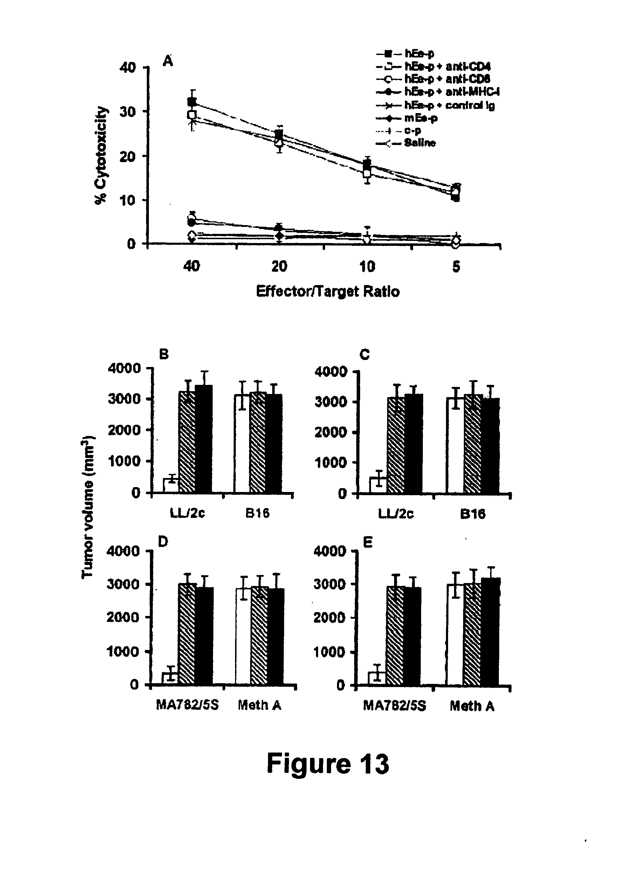

The protocols are carried out as follows: mice were immunized with recombinant chEGFR vaccine, and then spleen monolymphocyte were taken for ELISPOT test, which showed a large quantity of antigen specific B cells in the monolymphocyte. Testing in the mice immunized with recombinant mEGFR showed the existence of a small amount of antigen specific B cells. However, no statistically significant amount of antigen specific B cells was found in control groups including adjuvant group or blank vector group. In another experiment, spleen T cells were taken from the mice immunized with recombinant EGFR molecular vaccine for three weeks. Then, we used monolymphocyte from health spleen as antigen presenting cells to activate T cells in vitro in the presence of antigen. Results showed that the mice immunized with recombinant chEGFR molecular vaccine contained relatively a larger amount of IFN-γ, and IL-4 producing cells. Spleen T derived from recombinant chEGFR molecular vaccine immunized mice cells also produced many IFN-γ and IL-4 producing cells after being stimulated again in vitro by mEGFR molecular vaccine. Both groups of spleen T cells produced significantly more than T cells that were not stimulated in vitro. Furthermore, standard ⁵¹Cr tests were used to determine the specific cytocidal effect of spleen T cells immunized by recombinant EGFR molecular vaccine against the relevant tumor cells from human and mice. Results showed that after immunized by chEGFR molecular vaccine, at the ratio of 40:1, mouse spleen T cells exhibited specific cytocidal activity against EGFR positive tumor cells. In the example of LL/2c and MA 782/5S, they are 40.27%, 42.83%, respectively. In contrast, mice spleen T cells immunized with mEGFR exhibited no cytocidal effect on LL/2c and MA 782/5S tumor cells, and on EGFR-negative tumor cells, such as C26 cells. At the same time, the cytocidal effect mentioned above can be blocked by corresponding anti-CD8 and anti MHC-I monoclonal antibodies, but was not blocked by anti-CD4 and anti-MHC-II monoclonal antibodies. Spleen T cells from control groups did not show any statistically signifcant cytocidal activity. Figure 13 shows the CTL effect of EGFR recombinant DNA vaccine on immunized mice. It can be seen from this figure that T cells from mice immunized with human EGFR recombinant DNA vaccine hEe-p has higher cytocidal activity against EGFR-positive tumor cells compared to T cells from the control groups. Moreover, this in vitro cytocidal activity can be blocked by anti-CD8 or anti-MHC-I monoclonal antibody, but not by anti-CD4 monoclonal antibody, showing that this cytocidal activity is dependent on MHC-I dependent CD8⁺ T cells. In addition, the activated spleen cells exhibited no increase in NK activity against YAC-1 target cells. Moreover, adoptive transfer of CD4-depleted (CD8⁺), or CD8-depleted (CD4⁺) T cells derived from mice immunized with human EGFR recombinant DNA vaccine hEe-p exhibited anti-tumor effect against EGFR-positive tumor. In contrast, its anti-tumor effect is not significant against EGFR-negative tumor cells. No anti-tumor effect was seen from the control group.

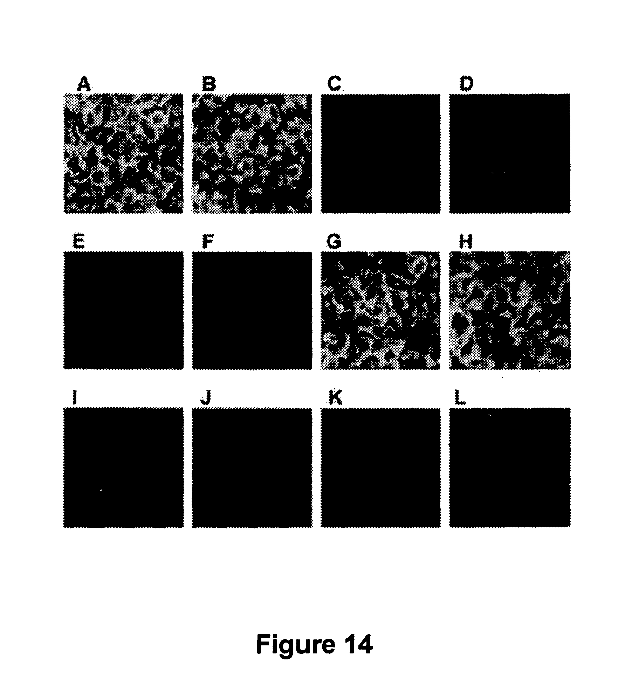

We also observed a long-term potential toxic impact on the mice immunized with EGFR molecular vaccine. No obvious toxic side effects were observed, such as weight loss, skin and hair deteriaration, loss of apetitie, reduced life expectancy. Microscopic examination of the liver, lung, spleen, and brain of immunized mice uncovered no pathological changes. Immunofluorescent staining showed no deposition of autoantibody in major organs of the mice. See Fig. 14.

### BRIEF DESCRIPTION OF THE DRAWINGS

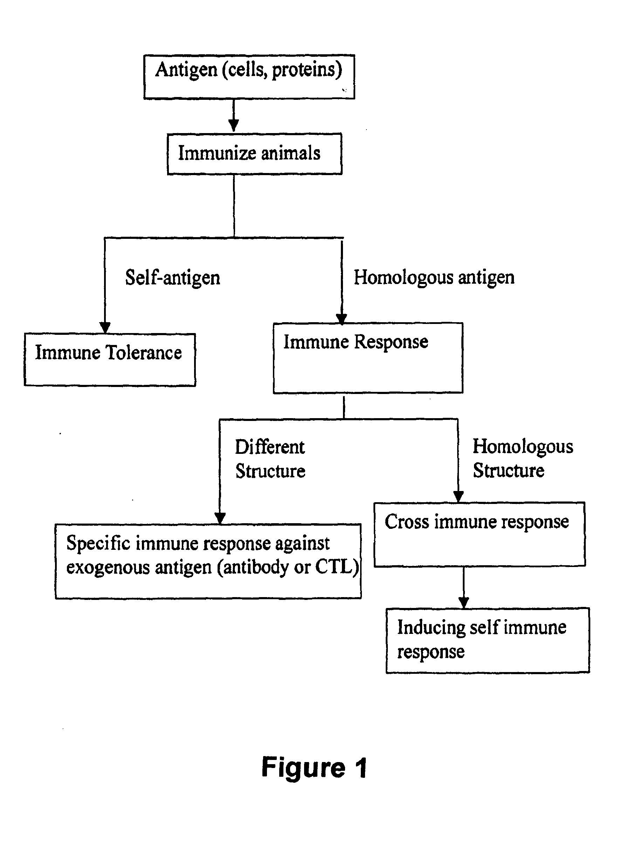

Figure 1 is a schematic diagram of the mechanism of action of homologous molecular vaccine.
Figure 2 shows recombinant EGFR plasmid maps. 2A shows recombinant EGFR eukaryotic expression plasmid maps; 2B shows recombinant EGFR prokaryotic expression plasmid maps; 2C shows recombinant EGFR yeast expression plasmid maps; 2D shows recombinant EGFR Adenovirus shuttle plasmid maps; 2E shows recombinant EGFR Lentivirus precursor plasmid maps.
Figure 3 is a flow chart depicting the construction of EGFR recombinant protein vaccine.
Figure 4 is a flow chart depicting the construction of EGFR recombinant virus vaccine. Figure 4A is a flow chart depicting the construction of EGFR recombinant Adenovirus vaccine; Figure 4B is a flow chart depicting the construction of EGFR recombinant Lentivirus vaccine.
Figure 5 is a flow chart depicting the construction of the RGD-modified EGFR recombinant Adenovirus vaccine.
Figure 6, including Figure 6A, 6B and 6C, is a schematic diagram depicting the mechanism of action for the nanoparticle targeted EGFR molecular vaccine.
Figure 7 is a graph showing the induction of protective anti-tumor immunity of EGFR recombinant DNA vaccine. 7A. Tumor Volume changes of immunized mice bearing LL/2c Lewis lung cancer. 7B. Tumor Volume changes of immunized mice bearing MA782/5S mammary cancer. 7C. Survival rate of immunized mice bearing LL/2c Lewis lung cancer. 7D. Survival rate of immunized mice bearing MA782/5S mammary cancer. hEe-p, human EGFR extracellular DNA vaccine; mEe-p, mouse EGFR extracelluar DNA vaccine; c-p, blank plasmid control; Saline, saline control.
Figure 8 is a graph showing the induction of therapeutic anti-tumor immunity of EGFR recombinant DNA vaccine. 8A. Tumor Volume changes of immunized mice bearing LL/2c Lewis lung cancer. 8B. Tumor Volume changes of immunized mice bearing MA782/5S mammary cancer. 8C. Survival rate of immunized mice bearing LL/2c Lewis lung cancer. 8D. Survival rate of immunized mice bearing MA782/5S mammary cancer, hEe-p, human EGFR extracellular DNA vaccine; mEe-p, mouse EGFR extracelluar DNA vaccine; c-p, blank plasmid control; Saline, saline control.
Figure 9 depicts the induction of anti-tumor immunity of EGFR recombinant protein vaccine. 9A. Protective immunity; 9B. Therapeutic immunity; 9C. Survival curves for mice bearing MA782/5S mammary cancer. edCER, Recombinant protein vaccine containing Chicken EGFR extracellular domain. edMER: Recombinant protein vaccine containing Mouse EGFR extracellular domain. Adj: Adjuvant. NS: Saline.
Figure 10 depicts the anti-tumor metastasis effect of EGFR recombinant protein vaccine. 10A. Number of transferer of LL/2c lung cancer. 10B. Lung wet weight after LL/2c tumor metastasis. edCER: Recombinant protein vaccine containing Chicken EGFR extracellular domain. edMER: Recombinant protein vaccine containing Mouse EGFR extracellular domain. Adj: Adjuvant. NS: Saline.
Figure 11 depicts EGFR molecular vaccine's inhibition of tumor cell growth in vitro, and its adoptive anti-tumor immunity. 11A. The inhibition of growth of EGFR-positive tumor cells (A549, LL/2c, MA782/5S) and EGFR-negative tumor cells (H22 and MMT-06052) by Ig derived from mice immunized with EGFR Recombinant DNA vaccine hEe-p. 11 B. Inhibition of growth of tumor cells by Ig derived from non-immunized normal mice. 11C. In vivo adoptive anti-tumor immunity of Ig derived from mice immunized with EGFR recombinant DNA vaccine hEe-p. hEe-p, human EGFR extracellular DNA vaccine; mEe-p, mouse EGFR extracelluar DNA vaccine; c-p, blank plasmid control; Saline, saline control.
Figure 12 depicts the types of antibodies induced by EGFR molecular vaccine. 12A. Types of antibodies induced by EGFR recombinant DNA vaccine.12B. Types of antibodies induced by EGFR recombinant protein vaccine. hEe-p, human EGFR extracellular DNA vaccine; mEe-p, mouse EGFR extracelluar DNA vaccine; c-p, blank plasmid control; Saline, saline control. edCER. Recombinant protein vaccine containing Chicken EGFR extracellular domain. edMER: Recombinant protein vaccine containing Mouse EGFR extracellular domain. Adj: Adjuvant. NS: Saline.
Fig.13 depecits the induction of CTL-mediated cytotoxicity in vitro with EGFR DNA vaccine and adoptive transfer of T cell subsets. A, T cells derived from the spleens of hEe-p-immunized mice were tested against LL/2c cells at different E:T ratios by a standard ⁵¹Cr release assay. T cells derived from the spleens of hEe-p-immunized mice showed higher cytotoxicity against LL/2c cells than did T cells from mEe-p, c-p, or nonimmunized mice by a standard ⁵¹Cr release assay. hEe-p-induced tumor killing activity can be blocked by anti-CD8 or anti- MHC class I (anti-H-2Kb/H-2Db) mAb. B and C, T cells were isolated from spleens of C57BL/6 mice, immunized with hEe-p (□), mEe-p, and nonimmunized mice (■), and were depleted of CD4⁺ or CD8⁺ lymphocytes. The adoptive transfer of 2×10⁷ CD4-depleted (CD8⁺) (B) or CD8-depleted (CD4⁺) (C) T cells from mice immunized with hEe-p showed the antitumor activity against EGFrpositive LL/2c. D and E, The adoptive transfer of 2×10⁷ CD4-depleted (CD8⁺)(D) or CD8-depleted (CD4⁺) (E) T cells from BALB/c mice immunized with hEe-p was also found to be effective in EGFR-positive MA782/5S mammary cancer model. There was no antitumor activity found in syngeneic EGFr-negative tumor (B16 and Meth A) (B-E). In addition, the transfer of T lymphocyte subsets from mice immunized with mEe-p and nonimmunized mice (■) had no effect (B-E). Data represent day 25 after tumor cell injection.
Fig.14 depicts the identification of auto-antibodies on the tumor cells by fluorescence microscopy. There was the deposition of auto-antibodies on LL/2c tumor cells (A) and MA782/5S tumor cells (B) from hEe-p-immunized mice, but not on the corresponding tumor cells from non-immunized mice (C and D). The mice depleted of CD4⁺T lymphocytes were immunized with hEe-p and did not develop detectable IgG-specific fluorescence on LL/2c tumor cells (E) and MA782/5S tumor cells the tumor cells (F). In contrast, the depletion of CD8⁺ lymphocytes (G and H) or NK cells showed no effect. There was no deposition of auto-antibodies within the tissues of the liver (I) and kidney (J) of mice immunized with hEe-p and the corresponding tissues (K and L) from non-immunized mice.

### DETAILED EMBODIMENTS OF THE INVENTION

The following examples are included for illustrative purposes only and are not intended to limit the scope of the invention.

### Example One: EGFR Recombinant DNA Vaccine

GenBank was searched to obtain the cDNA sequences for human, mouse, Chicken, and they are listed herein as SEQ ID NO 1-5,7-9,19, respectively. We designed the following PCR primers based upon the cDNA sequences. For pORF based plasmids

Human Primer: 5'GACCATGGAGGAAAAGAAAGTTTGC3',
5'ACGAATTCTTAGGACGGGATCTTAGGCCCA 3';

Mouse Primer: 5'GACCATGGAGGAAAAGAAAGTCTGC3',
5'ACGAATTCTTAATAGATGGTATCTTTGGC 3';

Chicken Primer: 5'GACCATGGAGGAGAAGAAAGTTTGTC3',
5'ACGAATTCTTAAGATGGAGTTTTGGAGCC 3'.

We use the total RNA from human lung cancer cell line A431, mouse lung cancer cell line LL2, and Chick Embryo to undertake RT-PCR amplification, then collect and purify the amplified EGFR fragment (average 1.9kb in length) using electrophoresis, and then subclone the PCR products. After confirming the sequences of the PCR subclones through sequencing, we digest them using Ncol and EcoRI, collect the 1.9 kb fragments and purify them, and then insert the fragments into the pORF-MCS vector (from the InvivoGen Corporation) which had been digested with Ncol and EcoRI. We select recombiant plasmids. Candidate recombinant plasmids are confirmed by both restriction enzyme digestion analysis and PCR amplification. They are named pORF-hEGFR, pORF-mEGFR and pORF-chEGFR.

### For pcDNA based plasmids:

For the construction of pcDNA-hEGFR, pcDNA-mEGFR 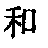 pcDNA-chEGFR, the process is similar to the above described process. In this case, we used pcDNA3.1 (+) vector made by InvitroGen, and vary the sequences of the PCR primers somewhat from those primers described in the above paragraph.

Human Primer: 5'GAGCTAGCATGGAGGAAAAGAAAGTTTGC3',
5'CACTCGAGTTAGGACGGGATCTTAGGCCCA 3';

Mouse Primer: 5'GAGCTAGCATGGAGGAAAAGAAAGTCTGC3',
5'CACTCGAGTTAATAGATGGTATCTTTGGC 3';

Chicken Primer: 5'GAGCTAGCATGGAGGAGAAGAAAGTTTGTC3',
5'CACTCGAGTTAAGATGGAGTTTTGGAGCC 3'.

We selected recombinant expression plasmids, use restriction enzyme analysis to confirm the presence of the EGFR sequence, and then transfect them into CHO, NIH3T3, Vero cell lines, respectively. We monitor the level of EGFR expression using SDS-PAGE, ELISA, Western Blot techniques. We used alkaline extraction to harvest the recombinant EGFR expression plasmids. Then, we use ultracentrifuge and ultra-filtration to eliminate E. coli toxin contamination, and thus obtaining pure plasmid DNA. These plasmid DNAs can be used as DNA vaccine to immunize subjects, in this case, mice.

Similar to the above process, we can design other primers or probes according to the sequences collected in public databases such as GenBank, the sequences including gene, cDNA, mRNA and amino acid sequences. These sequences can be from a variety of species such as human, mouse, rat, chicken, mackeral, drosphia, etc, see SEQ ID Nos: 1-14. Using these sequences, we clone and isolate the extracellular domains of EGFR molecules from different biological sources, using PCR, RT-PCR, molecular hybridization techniques, using commercial gene libraries, cDNA libraries (such as ClonTech, StrateGene's gene libraries and cDNA libraries), and cell lines (such as human lung cancer cell line A431, mouse Lewis lung cancer line LL2, etc.), or tissues (such as lung cancer, breast cancer tissues, drosphia tissue, and chick embryo, etc.). Also, we obtained EGFR molecules possessing strong immunogenicity using selections with the gene-directed evolutionary technique. After confirming the EGFR cDNA extracellular sequences using sequencing, we inserted the EGFR extracellular domain into Eukaryotic expression plasmids such as pcDNA3.1, pORF-mcs, pBLAST-MCS, pSecTag2, etc.

We selected mice aged 6-8 weeks old, and established various mouse tumor models using routine techniques. We injected 100□g of EGFR recombinant expression plasmids, once a week, for 4 weeks continuously. We observed the growth of the tumor-bearing mice, and the progression of the tumor. After 8 weeks, the mice were sacrificed, and sera of the immunized mice and organs were collected. We used flowcytometry, ELISA, and Westem Blot to test for humoral immune response. To examine cellular response, we used Cr⁵¹, ELISpot methods. To determine toxicity and side effects of the experiments, we used immunohistochemistry. Next, we purified the sera of the immunized mice, used routine methods to establish tumor models in nude mice, and undertake adoptive immunotherapy, and further observe the growth of the tumors.

### Example Two: EGFR Recombinant Protein Vaccine (Expressed in E. coli)

As in Example One, we obtain the cDNA sequences of EGFR from human, mouse, and chicken from public databases such GenBank, corresponding to SEQ ID NO 1-5,7-9,19, respectively. Based upon these sequences, we designed the following primers:

Human Primer: 5'GACCATGGAGGAAAAGAAAGTTTGC3',
5'ACAGATCTAGG ACGGGATCTTAGGCCCA 3'.

Mouse Primer: 5'GACCATGGAGGAAAAGAAAGTCTGC3',
5'ACAGATCTATAGATGGTATCTTTGGC 3'

Chicken Primer: 5'GACCATGGAGGAGAAGAAAGTTTGTC3',
5'ACAGATCTAGATGGAGTTTTGGAGCC 3'.

We use pORF-hEGFR, pORF-mEGFR 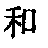 pORF-chEGFR as template for PCR amplification, then collect and purify the amplified EGFR fragment (average 1.9kb in length) using electrophoresis, and then subclone the PCR products. After confirming the sequences of the PCR subclones through sequencing, we digest them using Ncol and BglII, collect the 1.9 kb fragments and purify them, and then insert the fragments into the pQE60 vector (from the Qiagen Corporation) which had been digested with two restriction enzymes Ncol and BgIII. We select recombiant plasmids. Candidate recombinant plasmids are confirmed by both restriction enzyme digestion analysis and PCR amplification. They are named pQE-hEGFR, pQE-mEGFR 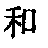 pQE-chEGFR.

Similarly, depending upon different prokaryotic expression vectors such as pET32, pLLp, pSE420, different primers can be designed and used to construct other EGFR recombinant prokaryotic expression plasmids.

Similar to Example One, we cloned the extracellular domain of various EGFR molecules, and then insert the domain into the prokaryotic expression plasmids such as pQE, pET32, pLLp, etc. After selecting recombinant expression plasmids, we confirmed the sequences of the domain using restriction enzyme digestion. We then transformed these recombinant plasmids into E.coli hosts such as E.coli TOP10F', E.coli BL21(DE3)pLys, E.coli M15, etc., and then observed and tested the level of expression of EGFR molecule using techniques such as SDS-PAGE, ELISA, Western Blot, etc. From these testing, we determined the optimal E.coli host for expression the recombinant plasmids. We then used confirmed EGFR expression plasmids to transform the optimal E.coli host cells, and then established stable transformants and various levels of seed bank libraries. We cultured the recombinant bacterial cells in shaking incubators, or alternatively, we fermented the bacteria using standard methods. We harvested the recombinant bacteria using low temperature centrifuge. Then we suspended the bacteria in PBS, and then broke the cells using ultrasound method. We used ionic exchange chromatography and affinity chromatography to isolate and purify the recombinant EGFR proteins. The purified EGFR proteins can be used as protein vaccine to immunize subject mice.

As in Example One, we selected mice aged 6-8 weeks old, and established various mouse tumor models using routine techniques. We injected 5-50□g of EGFR recombinant protein, once a week, for 4 weeks continuously. We observed the growth of the tumor-carrying mice, and the development of the tumor. After 8 weeks, the mice were sacrificed, and sera of the immunized mice and organs were collected. We used flowcytometry, ELISA, and Western Blot to test for humoral immune response. To examine cellular response, we used Cr⁵¹, ELISpot methods. To determine toxicity and side effects of the experiments, we used immunohistochemistry. Next, we purified the sera of the immunized mice, used routine methods to establish tumor models in nude mice, and undertake adoptive immunotherapy, and further observe the growth of the tumors.

### Example Three: EGFR Recombinant Protein Vaccine (Expressed in Yeast Pichia pastoris)

Again, as described in the previous sections, we obtain the cDNA sequences of EGFR from human, mouse, and chicken from public databases such GenBank, corresponding to SEQ ID NO 1-5,7-9,19, respectively. Based upon these sequences, we designed the following primers.

Human Primer:
5'ATACTCGAGAAAAGAGAGCTGGAGGAAAAGAAAG3',

5'GCTCTAGAATGGCACAGGTGGCACA 3';

Mouse Primer:
5'ATGCTCGAGAAAAGAGAGTTGGAGGAAAAGAAAGTC3',
5'AAGCGGCCGCCATAGATGGTATCT TTG 3';

Chicken Primer:
5'ATACTCGAGAAAAGAGAGGTGGAGGAGAAGAAAG3',
5'CGTCTAGAAGATGGAGTTTTGGAG 3'

We use pORF-hEGFR, pORF-mEGFR and pORF-chEGFR as template for PCR amplification, then collect and purify the amplified EGFR fragment (average 1.9kb in length) using electrophoresis, and then subclone the PCR products. After confirming the sequences of the PCR subclones through sequencing, we digest them using two restriction enzymes Xhol and Xbal (in the case of plasmids containing mouse EGFR, we use Xhol and NotI double enzyme digestion), collect the 1.9 kb fragments and purify them, and then insert the fragments into pPICZ A vector (from Invitrogen Corporation) which had been digested with two restriction enzymes Xhol and Xbal (in the case of plasmids containing mouse EGFR, we use Xhol and Notl double enzyme digestion). We transformed E.coli with these plasmid preparations, and select recombiant plasmids. Candidate recombinant plasmids are confirmed by both restriction enzyme digestion analysis and PCR amplification. They are named yeast expression plasmids: pYE-hEGFR, pYE-mEGFR and pYE-chEGFR.

After digesting these yeast expression plasmids with Pmel to linerize them, we use electric perforation method to transform yeast cell lines X33, or GS115. We use Zeocin resistance to select stable transformants. We use MMH (Minimal Methanol with histidine, MMH) and MDH (Minimal Dextrose with histidine, MDH) agar plate) to determine and selecte Mut⁺ transformants. We select 6-10 Mut⁺ transformants for small-scale expression, and then use SDS-PAGE, Western Blot, ELISA, etc. to confirm the expressed recombinant protein. We select the Mut⁺ transformants with the highest expression efficienty, and culture them at a large scale so as to establish yeast expression seed libraries. We use large flasks to culture or ferment the recombinant yeast cell lines, collect the yeast pellets using low temperature centrifuge. After resuspend the pellets n PBS solution, we use ultrasound to break the cells. Then, we used Ion Exchange Chromatography and affinity chromatography to isolate and purify the recombinant EGFR protein. The recombinant EGFR protein derived as such can be used as protein vaccine to immunize subjects. Similarly, yeast recombinant expression plasmids made with EGFR can be produced with other yeast expression systems.

As in Example Two, we selected mice aged 6-8 weeks old, and established various mouse tumor models using routine techniques. We injected 5-50□g EGFR recombinant protein, once a week, for 4 weeks continuously. We observed the growth of the tumor-carrying mice, and the development of the tumor. After 8 weeks, the mice were sacrificed, and sera of the immunized mice and organs were collected. We used flowcytometry, ELISA, and Western Blot to test for humoral immune response. To examine cellular response, we used Cr⁵¹, ELISpot methods. To determine toxicity and side effects of the experiments, we used immunohistochemistry. Next, we purified the sera of the immunized mice, used routine methods to establish tumor models in nude mice, and undertake adoptive immunotherapy, and further observe the growth of the tumors.

### Example Four: EGFR Recombinant Adenovirus Vaccine

As in the previous examples, we obtained the cDNA sequences of EGFR from human, mouse, and chicken from public databases such GenBank, corresponding to SEQ ID NO 1-5,7-9,19, respectively. Based upon these sequences, we designed the following primers:

Human Primer:
5'GAAGATCTATGGAGGAAAAGAAAGTTTGC3',
5'ACGATATCTTAAGGACGGGATCTTAGGCCCA 3';

Mouse Primer:
5'GAAGATCTATGGAGGAAAAGAAAGTCTGC3',
5'ACGATATCTTAATAGATGGTATCTTTGGC 3';

Chicken Primer:
5'GAAGATCTATGGAGGAGAAGAAAGTTTGTC3',
5'ACGATATCTTAAGATGGAGTTTTGGAGCC 3';

We use pORF-hEGFR, pORF-mEGFR and pORF-chEGFR as template for PCR amplification, then collect and purify the amplified EGFR fragment (average 1.9kb in length) using electrophoresis, and then subclone the PCR products. After confirming the sequences of the PCR subclones through sequencing, we digest them using two restriction enzymes BglII and EcoRV double enzyme digestion, collect the 1.9 kb fragments and purify them, and then insert the fragments into pShuttle-CMV vector (made by Quantum Biotechnologies) which was pre-digested with BglII and EcoRV. We select recombiant plasmids. The resulting candidate recombinant plasmids are confirmed by both restriction enzyme digestion analysis and PCR amplification. They are named Adenovirus shuttle expression plasmids: pShuttle-hEGFR, pShuttle-mEGFR and pShuttle-chEGFR. See Fig. 2D for plasmid maps of these Adenovirus shuttle expression plasmids.

We then take the various Adenovirus shuttle expression plasmids as described before, digest them with Pmel enzyme, and co-transform E.coli BJ5183 cells with backbone vector containing the Adenovirus genome pAdEasy-1 or pAdEast-2. The resulting recombinant Adenovirus vector plasmids are named pAd-hEGFR, pAd-mEGFR and pAd-chEGFR. These Adenovirus vector plasmids are digested with PacI enzyme, and then using the Calcium-phosphate-DNA coprecipitation method, they are transfected into the Adenovirus packaging cell line 293 cells. The resulting recombinant Adenoviruses are called Ad-hEGFR, Ad-mEGFR and Ad-chEGFR. PCR, Western blot and other methods are used to confirm that the EGFR gene has been indeed integrated into the Adenovirus vector, and that EGFR has been correctly, and efficiently expressed in Eukaryotic cells.

Depending upon the difference of the Adenovirus Genome, the recombinant Adenovirus vaccine can be classified into two groups: the first group is called Generation I of EGFR Recombinant Adenovirus, resulting from the recombination between Adenovirus Shuttle expression plasmid pShuttle-EGFR and AdEasy-1 recombinant, thus named Ad-hEGFR I, Ad-mEGFR I and Ad-chEGFR I. The second group is called Generation II of EGFR Recombinant Adenovirus, resulting from the recombination between Adenovirus Shuttle expression plasmid pShuttle-EGFR and AdEasy-2 recombinant, thus named Ad-hEGFR II, Ad-mEGFR II and Ad-chEGFR II.

We used ultracentrifuge to collect large quantity of the recombinant adenovirus Ad-EGFR, and then tested the titer (pfu) of each batch of the recombinant virus using upper layer agarose method, and TCID50 method. We then used 293 cells to produce large amount of confirmed EGFR adenovirus vaccine. We further isolated and purified the recombinant virus using ultracentrifuge and ultrafiltration. The purified EGFR recombinant adenovirus can be used a vaccine to immunize subject animals.

Similar to what's described in the previous examples, we selected mice aged 6-8 weeks old, and established various mouse tumor models using routine techniques. We injected 1×10⁹ PFU EGFR recombinant adenovirus in each mouse, once a week, for 4 weeks continuously. We observed the growth of the tumor-carrying mice, and the development of the tumor. After 8 weeks, the mice were sacrificed, and sera of the immunized mice and organs were collected. We used flowcytometry, ELISA, and Western Blot to test for humoral immune response. To examine cellular response, we used Cr⁵¹, ELISpot methods. To determine toxicity and side effects of the experiments, we used immunohistochemistry. Next, we purified the sera of the immunized mice, used routine methods to establish tumor models in nude mice, and undertake adoptive immunotherapy, and further observe the growth of the tumors.

### Example Five: EGFR Recombinant Lentivirus Vaccine

As an illustration of the process of making EGFR Recombinant Lentivirus, we use the ViraPower™ Lentiviral Gateway® Expression Kit made by Invitrogen Corporation. The detailed process is as follows: we first search public database such as GenBank to obatin cDNA sequences of EGFR molecule of human, mouse and chicken. Their sequences are listed as SEQ ID NO 1-5,7-9,19, respectively.

We then designed the following PCR primers:

Human Primer: 5'GACCATGGAGGAAAAGAAAGTTTGC3',
5'ACGATATCAGGACGGGATCTTAGGCCCA 3';

Mouse Primer: 5'GACCATGGAGGAAAAGAAAGTCTGC3',
5'ACGATATCATAGATGGTATCTTTGGC 3';

Chicken Primer: 5'GACCATGGAGGAGAAGAAAGTTTGTC3',

5'ACGATATCAGATGGAGTTTTGGAGCC 3'

We use pORF-hEGFR, pORF-mEGFR and pORF-chEGFR as template for PCR amplification, then collect and purify the amplified EGFR fragment (average 1.9kb in length) using electrophoresis, and then subclone the PCR products. After confirming the sequences of the PCR subclones through sequencing, we digest them using two restriction enzymes Ncol and EcoRV double enzyme digestion, collect the 1.9 kb fragments and purify them, and then insert the fragments into pENTR11 vector (made by Invitrogen) which was pre-digested with Ncol and EcoRV. We select recombiant plasmids. The resulting candidate recombinant plasmids are confirmed by both restriction enzyme digestion analysis and PCR amplification. They are named as pENTR-hEGFR, pENTR-mEGFR and pENTR-chEGFR. See Fig. 2E for plasmid maps of these Lentivirus expression plasmids.

We then take the various Lentivirus expression plasmids as described before, co-transform E.coli DH5 cells together with backbone vector containing the Lentivirus genome pLenti6/V5-DEST. The resulting recombinant Lentivirus vector plasmids are named pLenti-hEGFR, pLenti-mEGFR and pLenti-chEGFR. See also Fig. 2E. We then mix these Recombinant Lentivirus vector plasmids with packaging mix, the ViraPower™ Packaging Mix, and then using the Calcium-phosphate-DNA coprecipitation method, the Recombinant Lentivirus vector plasmids are transfected into the Lentivirus packaging cell line 293FT cells. The resulting recombinant Lentivirus are called Lenti-hEGFR, Lenti-mEGFR and Lenti-chEGFR. PCR, Western blot and other methods are used to confirm that the EGFR gene has been indeed integrated into the Lentivirus vector, and that EGFR has been correctly, and efficiently expressed in Eukaryotic cells.

We used ultracentrifuge to collect large quantity of the recombinant adenovirus Ad-EGFR, and then tested the titer (pfu) of each batch of the recombinant virus using upper layer agarose method, and TCID50 method. We then used 293 cells to produce large amount of confirmed EGFR adenovirus vaccine. We further isolated and purified the recombinant virus using ultracentrifuge and ultrafiltration. The purified EGFR recombinant adenovirus can be used a vaccine to immunize subject animals.

Similar to what's described in the previous examples, we selected mice aged 6-8 weeks old, and established various mouse tumor models using routine techniques. We injected 1×10⁹ PFU EGFR recombinant Lentivirus in each mouse, once a week, for 4 weeks continuously. We observed the growth of the tumor-carrying mice, and the development of the tumor. After 8 weeks, the mice were sacrificed, and sera of the immunized mice and organs were collected. We used flowcytometry, ELISA, and Western Blot to test for humoral immune response. To examine cellular response, we used Cr⁵¹, ELISpot methods. To determine toxicity and side effects of the experiments, we used immunohistochemistry. Next, we purified the sera of the immunized mice, used routine methods to establish tumor models in nude mice, and undertake adoptive immunotherapy, and further observe the growth of the tumors.

### Example Six: Mannan-modified Recombinant Adenovirus EGFR vaccine

The following procedure applies to the preparation of Mannan-modified Recombinant Adenovirus EGFR vaccine: we first use standard protocol to obtain and amplify EGFR Recombinant Adenovirus (either Generation I or II), and then use chromatography or ultracentrifuge to purify the recombinant adenovirus. Next, we dissolve 70mg Mannan from Sigma into 5ml 0.1 M phosphate buffer (pH6.0) to reach a final concentration of 14mg/ml, and then add 45ml 0.01M Sodium Periodate solution, and mix and oxidize at 4° C for 60 minutes. After that, we add 10µl glycol, and incubate for 30 minutes at 4° C, resulting in Oxidative Mannan (Ox-M) mixture.

We then load the Ox-M mixture onto Sephadex-G25 columns previously balanced with bicarbonate buffer (pH6.0-9.0) and perform chromatography, with Ox-M being eluted into 2ml sized empty vessel. After that, we mix the purified Ox-M with 1x10¹⁴ Recombinant Adenovirus particles at room temperature overnight, obtaining the needed Ox-M-Adenovirus. We then add 1mg/ml Sodium Borohydride to the Ox-M-Adenovirus, leave at room temperature for 3 hours, forming Reductive Mannan Adenovirus (Red-M-Adenovirus). Both Ox-M-Adenovirus and Red-M-Adenovirus are desalted by ultrafiltration, and condensed, filtering out bacteria. They are stored in small test tubes, and preserved at -80° C. The Mannan-modified Recombinant EGFR Adenovirus can be used as vaccine to immunize a subject.

Similar to what's described in the previous examples, we selected mice aged 6-8 weeks old, and established various mouse tumor models using routine techniques. We injected 1×10¹⁰ PFU EGFR recombinant Lentivirus in each mouse, once a week, for 4 weeks continuously. We observed the growth of the tumor-carrying mice, and the development of the tumor. After 8 weeks, the mice were sacrificed, and sera of the immunized mice and organs were collected. We used flowcytometry, ELISA, and Western Blot to test for humoral immune response. To examine cellular response, we used Cr⁵¹, ELISpot methods. To determine toxicity and side effects of the experiments, we used immunohistochemistry. Next, we purified the sera of the immunized mice, used routine methods to establish tumor models in nude mice, and undertake adoptive immunotherapy, and further observe the growth of the tumors.

### Example Seven: RGD-modified Recombinant Adenovirus EGFR vaccine

In the present invention, we utilize the AdEasy system to construct the RDG modified Adenovirus Recombinant EGFR vaccine. The detailed process is shown step by step in Figure 5.

The detailed process is as follows: we digest the Adenovirus backbone plasmid pAdEasy-1 and pAdEasy-2 with restriction enzyme Spel (Sp), and then use T4 DNA Polymerase to fill in the ends (filling, f) so as to make them blunt, and then digest the filled-in product with PacI (P), and recover the 6211 bp and 3579bp fragments using electrophoresis, and name them AdFiber I/Sp/f/P and AdFiber II/Sp/f/P , respectively. These fragments contain the intact Adenovirus fiber protein gene. Separately, prepare the psuttle vector by digesting it with BamHI first, and then fill in with T4 DNA Polymerase, and then digest with PacI. After such BamHI/filling /PacI-digestion treatment, the vector is ready to be inserted with the AdFiber I/Sp/f/P 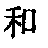 AdFiber II/Sp/f/P fragments. The resulting plasmids are named pSh-AdFiber I and pSh-AdFiber II, respectively.

We then digest pSh-AdFiber I with Nhel enzyme, fill in with T4 DNA polymerase, and digest again with Kpnl enzyme ( NheI/filling/KpnI), recover, using electrophoresis, the 2090 bp fragment called AdFiber I/Nh/f/K; insert this fragment into a pUC18 vector which had been pre-digested with Smal and Kpnl double enzyme digestion, resulting in the recombinant plasmid named pUC-AdFiber I.

On the other hand, pSh-AdFiber II is digested with Avrll enzyme, then filled in with T4 DNA polymerase, and then digested with Hindlll (AvrII/filling/HindIII), using electrophoresis, recover the 838 bp fragment, called AdFiber I/A/f/H. Insert this fragment into a pUC vector which had been previously digested with SmaI and Hindlll double enzyme digestion, resulting in new plasmids named pUC-AdFiber II.

Next, we designed a series of PCR primers so as to use pUC-AdFiber I and pUC-AdFiber II as templates to amplify the Adenovirus knob, (Ad-knob) gene sequences. The primers used are, respectively:

F1（5'GAAAGCTAGC CCTGCAAACATCA 3'），

R1（5'ACTCCCGGGAGTTGTGTCTCCTGTTTCCTG3'），

F2（5'ACTCCCGGGAGTGC ATACTCTATGTCA 3'），

R2（5'TATGGTAC CGGGAGGTGGTGA 3'），

F3（5'AACCTAGGGAGGTTAACCTAAGCACTG 3'）, and

R3（5'CTCAAGCTTTTTGG AATTGTTTGA 3'）.

Using primer F1-R1, F2-R2, F3-R1 and F2-R3, respectively, for the first round PCT, we obtain products PCR1, PCR2, PCR3 and PCR4. Again, using F1-R2 and F3-R3 as primers, and using the amplified products from the first round, PCR1 and PCR2, PCR3 and PCR4 as templates, we undertake the second round of PCR amplification, resulting in PCR products PCR1-PCR2 (PCR I) , PCR3-PCR4 (PCR II) . We take the PCR I and PCR II from the amplification in the second round, insert them into pBR322 vector that had been previously cut with EcoRV. The resulting recombinant plasmids are named pBR-PCR I and pBR-PCR II.

The sequence of the RGD-4C duplex is as follows:

5'TGTGACTGCCGCGGAGACTGTTTCTGC 3'

3'ACACTGACGGCGCCTCTGACAAAGACG 5'

We insert the RGD-4C into the pBR-PCR I and pBR-PCR II vectors, where the vector were previously digested with Smal. The resulting recombinant plasmids are named pBR-PCR/RGD I and pBR-PCR/RGD II. The sequences of the recombinant plasmids are confirmed using sequencing. Cut pBR-PCR/RGD I with NheI/KpnI double digestion, using electrophoresis, rcover the PCR/RGD I fragment. Insert the fragment into the pUC-AdFiber I vector which had been double digested using Nhel/Kpnl. The resulting recombinant plasmids are called pUC-AdFiber-RGD I.

Similarly, we use AvrII/HindIII to double digest pBR-PCR/RGD II, and using electrophoresis, recover the PCR/RGD II fragment. Again, insert the fragment into pUC-AdFiber II vectors previously digested with double enzume AvrII/HindIII, the resulting plasmid is named pUC-AdFiber-RGD II.

Afterwards, we use SpeI/PacI double enzyme to digest pUC-AdFiber-RGD I and pUC-AdFiber -RGD II vector, and using electrophoresis, recover the AdFiber-RGD I, and AdFiber -RGD II fragments. Insert the fragment into pAdEasy-1, pAdEasy-1 both of which were previously digested with SpeI/PacI, the resulting recombinant plasmids are called pAdEasy-RGD I, and pAdEasy-RGD II, respectively.

Next, we first take pShuttle-hEGFR, pShuttle-mEGFR and pShuttle-chEGFR as described above, and linerize them with Pmel. After that, we co-transform the E.coli BJ5183 cells with these Shuttle-EGFR plasmids together with pAdEasy-RGD I, and pAdEasy-RGD II, respectively. The resulting recombinant plasmids are named Adenovirus plasmids pAd-RGD-EGFR I, and pAd-RGD-EGFR II.

We used the Adenovirus plasmid pAd-RGD-EGFR I to transfect 394 cells, resulting in recombinant Adenovirus named Ad-RGD-EGFR I. Similarly, we transfect Adenovirus plasmid pAd-RGD-EGFR II to 293E4pIX cells, resulting in recombinant Adenovirus named Ad-RGD-EGFR II. After purification, Ad-RGD-EGFR I and Ad-RGD-EGFR II can be used as vaccine to immunize subjects, and more importantly, these vaccines have specificity targeting tumor vascular endothelial cells.

### Example Eight: Pharmacological studies of Recombinant EGFR Molecular vaccine

It is important to obtain pharmacological studies relating to the anti-tumor effect of recombinant EGFR molecular vaccine. Studies can be performed for this purpose in the present invention include: ordinary tumor-bearing mice (including protective immunity study, therapeutic immunity study, and metastasis model study), rabbit immunological test, monkey immunological test, nude mice adoptive immunological test, dosage-dependency test, and in vitro cellular test, etc. In this example, we describe the ordinary tumor-bearing mice test, using lung cancer as a model. Other tests can be performed similarly.

### 1. Protective immunity of tumor-bearing mice:

6-8 weeks old C57BU6 mice are randomly divided into groups. They are injected EGFR molecular vaccine in both hind legs intramuscularly, once a week for four weeks continuously. At the 5^{th} week, mice were are challenged with LL/2c tumor cells in the concentration of 1x10⁶. Blood is taken from tail vein or from sacraficing the mice at week 0, 1, 3, 5 after initial immunization. After centrifuge (5000RPM for 3 minutes), sera is collected and stored at -20° C for future use.

### 2. Therapeutic immunity of tumor-bearing mice:

6-8 weeks old C57BL/6 mice are randomly divided into groups. They are challenged with LL/2 cancer cells at a concentration of 1x10⁶. Four days after tumor introduction, they are randomly divided into groups, and immunized with EGFR molecular vaccine by injection intramuscularly in the hind legs, once a week for four weeks continuously. At week 0, 2. 4, and 6 after initial immunization, blood is taken from tail vein or from sacraficing the mice. After centrifuge (5000RPM for 3 minutes), sera is collected and stored at -20° C for future use. Record tumor weight, volume and survial curves.

### 3. Metastasis model test of tumor-bearing mice:

6-8 weeks old C57BL/6 female mice are randomly divided into groups. They are injected EGFR molecular vaccine in both hind legs intramuscularly, once a week for four weeks continuously. After two weeks, LL/2 tumor cells at log growth period are selected, and injected intramuscularly into the hind legs of the mice, at a concentration of 2x10⁵ per mouse. Contiue the injection mentioned above. After four weeks, mice are killed, and their lungs are weighed. After examination for tumor transferer in the lung, the lungs are fixated with 10% neutrally buffered formaldehyde.

It is understood that the examples and embodiments described herein are for illustrative purposes only and that various modifications or changes in light thereof will be suggested to persons skilled in the art and are to be included within the spirit and purview of this application and scope of the appended claims.

## Claims

**1.** A method for making vaccine comprising the steps of:
1) analyzing specific antigen of particular pathogen;
2) obtaining a polynucleotide sequence encoding the specific antigen;
3) obtaining a polynucleotide sequence having a sufficient difference from the polynucleotide sequence; and
4) preparing the vaccine using the polynucleotide sequence obtained in step 3).

**2.** The method according to claim 1, wherein said pathogen is selected from the group of bacterial, virus, fungus and protozoan.

**3.** The method according to claim 1, wherein the homology between the polynucleotide sequence of step 1) and the polynucleotide sequence of step 3), as compared by the polypeptide sequences encoded by them, is 30 to 95%.

**4.** The method according to claim 1, wherein the form of said vaccine is selected from the group consisting of recombined protein vaccine, recombined gene vaccine, recombined virus vaccine, genetically modified vaccine, and stably inverted symbiotic bacteria.

**5.** A method for making cancer vaccine comprising the steps of:
1) analyzing specific antigen of tumor cell in an animal;
2) obtaining a polynucleotide sequence encoding the specific antigen;
3) obtaining a polynucleotide sequence having a sufficient difference from the polynucleotide sequence; and
4) preparing the vaccine using the polynucleotide sequence obtained in step 3).

**6.** The method according to claim 5, wherein said tumor cell occurs in an animal.

**7.** The method according to claim 5, wherein said tumor cell originates from an animal.

**8.** The method according to claim 6 or 7, wherein said animal includes human.

**9.** The method according to claim 5, wherein the homology between the polynucleotide sequence of step 1) and the polynucleotide sequence of step 3), as compared by the polypeptide sequences encoded by them, ranges from 30 to 95%.

**10.** The method according to claim 5, wherein the form of said vaccine is selected from the group consisting of recombined protein vaccine, recombined gene vaccine, recombined virus vaccine, genetically modified vaccine, and stably inverted symbiotic bacteria.

**11.** A method for making EGFR vaccine comprising the steps of:
1) analyzing EGFR antigen of tumor cell in an animal;
2) obtaining a polynucleotide sequence encoding the specific antigen;
3) obtaining a polynucleotide sequence having a sufficient difference from the polynucleotide sequence; and
4) preparing the vaccine using the polynucleotide sequence obtained in step 3).

**12.** The method according to claim 11, wherein said tumor cell occurs in an animal.

**13.** The method according to claim 11, wherein said tumor cell originates from an animal.

**14.** The method according to claim 11, wherein the homology between the polynucleotide sequence of step 1) and the polynucleotide sequence of step 3), as compared by the polypeptide sequences encoded by them, ranges from 30 to 95%.

**15.** The method according to claim 11, wherein the form of said vaccine is selected from the group consisting of recombined protein vaccine, recombined gene vaccine, recombined virus vaccine, genetically modified vaccine, and stably inverted symbiotic bacteria.

**17.** A nucleic acid vaccine, wherein the homology between the polypeptide encoded by the polynucleotide sequence contained in the vaccine and the polypeptide encoded by the polynucleotide sequence of specific antigen in pathogen is 30 to 95%.

**18.** An epidermal growth factor receptor (EGFR) nucleic acid vaccine, wherein the homology between the epidermal growth factor receptor encoded by the polynucleotide sequence contained in the vaccine and the amino acid sequence in the epidermal growth factor receptor of the organism is 30 to 95%.

**19.** An epidermal growth factor receptor (EGFR) nucleic acid vaccine, wherein the homology between the epidermal growth factor receptor encoded by the polynucleotide sequence contained in the vaccine and the amino acid sequence in the epidermal growth factor receptor of human is 30 to 95%.

**20.** A method for making EGFR vaccine used for treating human tumor comprising the steps of:
1) obtaining a polynucleotide sequence encoding EGFR from xenogeneic organism; and
2) preparing the vaccine by using the polynucleotide sequence obtained in step 1).

**21.** The method according to claim 20, wherein said xenogeneic organism is other organism except for human, such as mice, rat, chicken, mackerel, drosphia, and the like.

**22.** The method according to claim 20, wherein the polynucleotide sequence encoding EGFR from xenogeneic organism is selected from the group consisting of SEQ.ID.No.13, SEQ.ID.No.15, SEQ.ID.No.17, SEQ.ID.No.19, SEQ.ID.No.21, SEQ.ID.No.23, SEQ.ID.No.24, and SEQ.ID.No.26.

**23.** The method according to claim 20, wherein the form of said vaccine is selected from the group consisting of recombined protein vaccine, recombined gene vaccine, recombined virus vaccine, genetically modified vaccine, and stably inverted symbiotic bacteria.

**24.** The method according to claim 20, wherein the human tumor is selected from the group consisting of lung cancer, breast cancer, ovary cancer, colon cancer, prostate cancer, bladder cancer, head and neck squamocarcinoma and glioma, which over-express EGFR molecule.

**25.** A method for making EGFR vaccine used for treating human tumor comprising the steps of:
1) modifying the polynucleotide sequence of human EGFR; and
2) preparing the vaccine by selecting the modified polynucleotide sequence from the polynucleotide sequence obtained in step 1).

**26.** The method according to claim 25, wherein said polynucleotide sequence of human EGFR is selected from the group consisting of SEQ.ID.No.1, SEQ.ID.No.3, SEQ.ID.No.5, SEQ.ID.No.7, SEQ.ID.No.9, SEQ.ID.No.11.

**27.** The method according to claim 25, wherein said modification method is selected from the group consisting of error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, GSSM and any combination thereof.

**28.** The method according to claim 25, wherein the form of said vaccine is selected from the group consisting of recombined protein vaccine, recombined gene vaccine, recombined virus vaccine, genetically modified vaccine, and stably inverted symbiotic bacteria

**29.** The method according to claim 25, wherein the human tumor is selected from the group consisting of lung cancer, breast cancer, ovary cancer, colon cancer, prostate cancer, bladder cancer, head and neck squamocarcinoma and glioma, which over-express EGFR molecule.

**30.** An epidermal growth factor receptor polypeptide vaccine, wherein the homology between the epidermal growth factor receptor polypeptide contained in the vaccine and the amino acid sequence in the epidermal growth factor receptor of human is 30 to 95%.
